# EUROPEAN PATENT SPECIFICATION

(11) **EP 2 971 113 B1**
(45) Date of publication and mention of the grant of the patent: **12.08.2020**
(21) Application number: 14747411.8
(22) Date of filing: 14.03.2014
(51) Int. Cl.: C12Q 1/6886

(54) **METHOD FOR THE PROGNOSIS AND TREATMENT OF CANCER METASTASIS**
VERFAHREN ZUR PROGNOSE UND BEHANDLUNG VON KREBSMETASTASEN
PROCÉDÉ DE PRONOSTIC ET DE TRAITEMENT DE MÉTASTASES CANCÉREUSES

(30) Priority: 15.03.2013 US 201361801718 P
(43) Date of publication of application: 20.01.2016
(73) Proprietor: Fundació Institut de Recerca Biomèdica (IRB Barcelona), 08028 Barcelona (ES); Institució Catalana de Recerca I Estudis Avançats, 08010 Barcelona (ES)
(72) Inventor: GOMIS, Roger, E-08021 Barcelona (ES); PLANET, Evarist, E-08360 Barcelona (ES); PAVLOVIC, Milica, Lajkovac 14224 (RS); ARNAL, Anna, E-08012 Barcelona (ES); TARRAGONA, Maria, E-08006 Barcelona (ES)
(74) Representative: ABG Intellectual Property Law, S.L.
(86) International application number: PCT/IB2014/001253
(87) International publication number: WO 2014/140933

(56) References cited:
- EP-A1- 2 650 682
- EP-A2- 2 626 431
- WO-A2-2012/045905
- WO-A2-2012/064967
- WO-A2-2013/182912
- EMANUELA MATTEUCCI ET AL: "Bone metastatic process of breast cancer involves methylation state affecting E-cadherin expression through TAZ and WWOX nuclear effectors", EUROPEAN JOURNAL OF CANCER, vol. 49, no. 1, 1 January 2013 (2013-01-01), pages 231-244, XP055143941, ISSN: 0959-8049, DOI: 10.1016/j.ejca.2012.05.006
- SUZUKI H ET AL: "THREE DISTINCT COMMONLY DELETED REGIONS OF CHROMOSOME ARM 16Q IN HUMAN PRIMARY AND METASTATIC PROSTATE CANCERS", GENES, CHROMOSOMES & CANCER, XX, XX, vol. 17, no. 4, 1 January 1996 (1996-01-01), pages 225-233, XP009007644, DOI: 10.1002/(SICI)1098-2264(199612)17:4<225::A ID-GCC4>3.3.CO;2-S
- Analyte Specific Reagent: "Vysis LSI IGH/MAF Dual Color Dual Fusion Probe Shopping Cart Product Catalog Abbott Global HOME PRODUCTS TECHNOLOGIES SUPPORT ABOUT US", , 9 January 2005 (2005-01-09), XP055144286, Retrieved from the Internet: URL:http://www.abbottmolecular.com/us/prod ucts/analyte-specific-reagent/fish/vysis-l si-igh-maf-dual-color-dual-fusion-probe.ht ml [retrieved on 2014-10-03]

## Description

### BACKGROUND OF THE INVENTION

### Field of the Invention

The present invention relates to the detection of genetic abnormalities and to the prognosis of bone metastasis in cancer based on same. In one embodiment, the invention involves determining the levels of a gene of interest in a primary tumor sample using a probe. Likewise, the invention also relates to a method for designing a customized therapy in a subject with cancer which comprises determining the level of a gene of interest in a sample using, for example, a probe. In one embodiment, the gene of interest is selected from the group consisting of: MAF, VAT1L, CLEC3A, WWOX, and the gene the sequence of which is provided in the Gene Database with ID 645957. In another embodiment, the cancer is selected from the group consisting of: breast cancer, lung cancer, prostate cancer, thyroid cancer and renal cancer.

### Problem

Metastasis, a complex process caused by elaborate interactions between tumor cells and the surrounding normal tissues in different vital organs, accounts for 90 percent of all cancer deaths in patients with solid tumors. The molecular and cellular mechanisms that lead primary tumors to form metastases must be understood in order to better address this major life-threatening problem. The identification of metastasis genes and mechanisms is essential for understanding the basic biology of this lethal condition and its implications for clinical practice. Previous work provided a sense of the complexity of the metastasis process, but it failed to explain how and why metastasis occurs, what mechanisms make metastasis a tissue-specific process, what events allow dormant metastases to become active and lethal many years after removal of a primary tumor, and what metastasis-mediating genes would eventually constitute worthy diagnostic markers and therapeutic targets.

As published by Suzuki H. et al in "Three distinct commonly deleted regions of chromosome arm 16Q in human primary and metastatic prostate cancers" (Genes, chromosomes and cancer 1996, 17, 225-233), multiple tumor suppressor genes on chromosome arm 16q are involved in the pathogenesis of cancers, particularly in prostate cancer three distinct commonly deleted regions may harbor potential tumor suppressor genes. In another article by Emanuela Matteucci et al., "Bone metastatic process of breast cancer involves methylation state affecting E-cadherin expression through TAZ and WWOX nuclear effectors" (European Journal of Cancer 2013, 49, 231-244) describes the evidence for the WWOX expression in human and experimental bone metastatic processes from breast cancer, and clarifies the epigenetic regulation of WWOX in bone metastasis functions as E-cadherin activator.

WO 2012045905 A2 relates to an in vitro method for the diagnosis of metastasis in a subject with cancer and/or for the prognosis of the tendency to develop metastasis in a subject with cancer. WO2012064967 A2 relates to compositions and methods to inhibit metastases of cancer cells such as of prostate, bones or soft tissue.

The present invention is based on the realization that the identification of markers that predict bone metastasis would provide a preventive therapeutic opportunity by imposing restrictions to the spreading and colonization of bone metastatic tissue by cancer cells and delay or transform a lethal condition. Thus, for example, it has been shown that MAF, a bona fide breast cancer bone metastasis gene, protein and mRNA accumulation acquired by, among other potential mechanisms, 16q22-24 (16q23) amplifications or 16q23 translocations is also responsible for driving the cancer bone metastatic lesions, and, in a preferred embodiment osteolytic cancer bone metastasis.

### SUMMARY OF THE INVENTION

The inventors determined that identifying the balance of signals that affect disseminated cancer cell bone metastasis will provide valuable clues to establish the prognosis and for preventive therapeutic intervention against disease.

Thus, one aspect of the invention is related to an in vitro method for the prognosis of the tendency to develop metastasis and/or recurrence in a subject with cancer, said method comprising;
i) quantifying the copy number or amplification of a gene of interest in a sample of said subject, wherein the gene of interest is selected from the group consisting of VAT1L, CLEC3A or the gene the sequence of which is provided in the Gene Database with ID 6459575, or quantifying the expression level, copy number or amplification of the gene of interest WWOX and
ii) comparing the expression level, copy number or amplification obtained in (i) with with the expression level, copy number or degree of amplification of the gene of interest in a control sample,
wherein if the expression level, copy number or amplification of a gene of interest in said sample is increased with respect to the expression level, copy number or degree of amplification of the gene of interest in the control sample, then said subject has a greater tendency to develop metastasis and/or recurrence.

In another aspect, the present invention describes an in vitro method for designing a customized therapy for a subject with cancer which comprises

i) quantifying the copy number or amplification of a gene of interest in a sample of said subject, wherein the gene of interest is selected from the group consisting of:
VAT1L, CLEC3A or the gene the sequence of which is provided in the Gene Database with ID 6459575 or quantifying the expression level, copy number or amplification of the gene of interest WWOX, and
ii) comparing the expression level, copy number or amplification obtained in (i) with the expression level, copy number or degree of amplification of the gene of interest in a control sample,
wherein if the expression level, copy number or degree of amplification of a gene of interest in the sample is increased with respect to the expression level the gene of interest in the control sample, then said subject is susceptible to receive a therapy intended to prevent, inhibit and/or treat metastasis of the cancer and/or a therapy intended to prevent or inhibit bone degradation.

In another aspect, the invention relates to an in vitro method for the prognosis of the tendency to develop metastasis and/or recurrence in a subject with cancer which comprises determining if a gene of interest is amplified, gained or shows polyploidy in a tumor sample of said subject relative to a reference gene copy number, wherein said amplification, gain or polyploidy of the gene of interest with respect to said reference gene copy number is indicative of an increased risk of developing metastasis and/or recurrence, wherein the gene of interest is selected from the group consisting of: VAT1L, CLEC3A, WWOX, or the gene the sequence of which is provided in the Gene Database with ID 6459575.

Another aspect of the present invention relates to an in vitro method for typing a sample of a subject suffering from cancer, the method comprising:
a) providing a sample from said subject;
b) quantifying the copy number or amplification of a gene of interest in said sample, wherein the gene of interest is selected from the group consisting of: VAT1L, CLEC3A or the gene the sequence of which is provided in the Gene Database with ID 645957 or quantifying the expression level, copy number or amplification of the gene of interest WWOX;
c) typing said sample by comparing the quantified expression level, copy number or amplification of the gene of interest to a predetermined reference level of gene of interest expression, copy number or degree of amplification;
wherein said typing provides prognostic information related to the risk of bone metastasis and/or recurrence in said subject.

In another aspect the invention relates to a method of classifying a subject suffering from cancer into a cohort for conducting a clinical trial comprising: a) determining the copy number or amplification of a gene of interest in a sample of said subject, wherein the gene of interest is selected from the group consisting of: VAT1L, CLEC3A or the gene the sequence of which is provided in the Gene Database with ID 6459575 or quantifying the expression level, copy number or amplification of the gene of interest WWOX; b) comparing the expression level, copy number or amplification of the gene of interest in said sample to a predetermined reference level, copy number or amplification of the gene of interest in said sample, and wherein said expression level, copy number or degree of amplification of the gene of interest in said sample is increased relative to said predetermined reference level, and wherein members of the cohort are classified as having increased risk of bone metastasis.

### BRIEF DESCRIPTION OF THE DRAWINGS

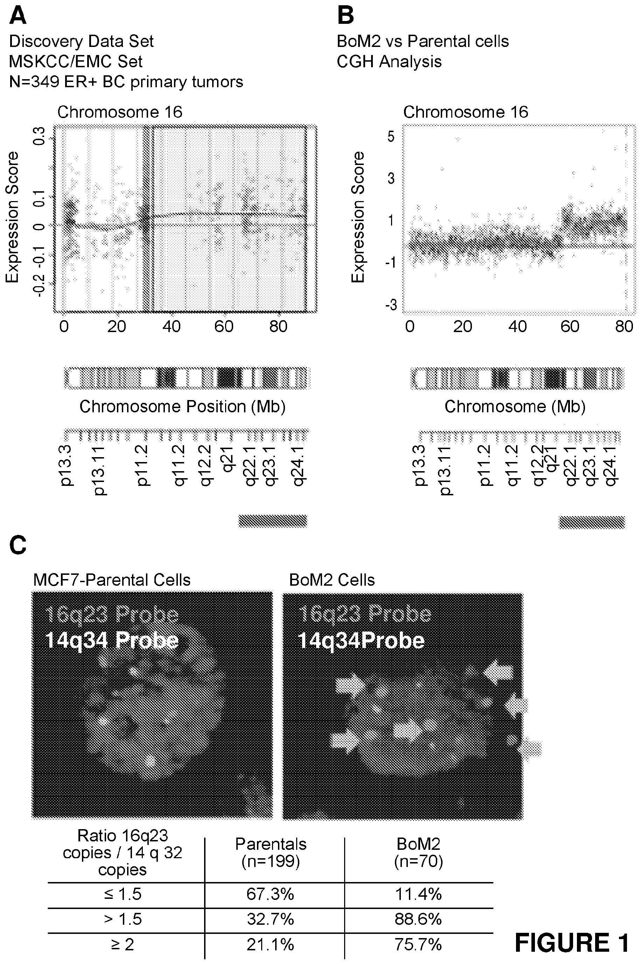

Figure 1. 16q23 overexpression is associated with bone metastasis
a) Analysis of copy number alteration based on gene expression (ACE Algorithm). Shaded area depicts DNA genomic amplification significantly associated with relapse in ER+ breast cancer tumors (union of GSE2603, GSE2034 and GSE12276 data set).
b) For chromosome 16, black dots and grey horizontal lines represent normalized log2 intensity ratios and segments, respectively. BoM2 are compared over MCF7 parental cells. At the bottom, in grey the 16q22-24 DNA, including 16q23 genomic amplification is highlighted.
c) Panel depicting percentage of Parental and BoM2 bone metastatic cells with MAF gene amplification based on ratio between 16q23 gene copies (probe used flanks five genes VAT1L, CLEC3A, WWOX, the gene the sequence of which is provided in the Gene Database with ID 645957 and MAF (ordered from centromer to telomer) and 14q32 gene copies. Representative images of FISH stained Parental and BoM2 cells.

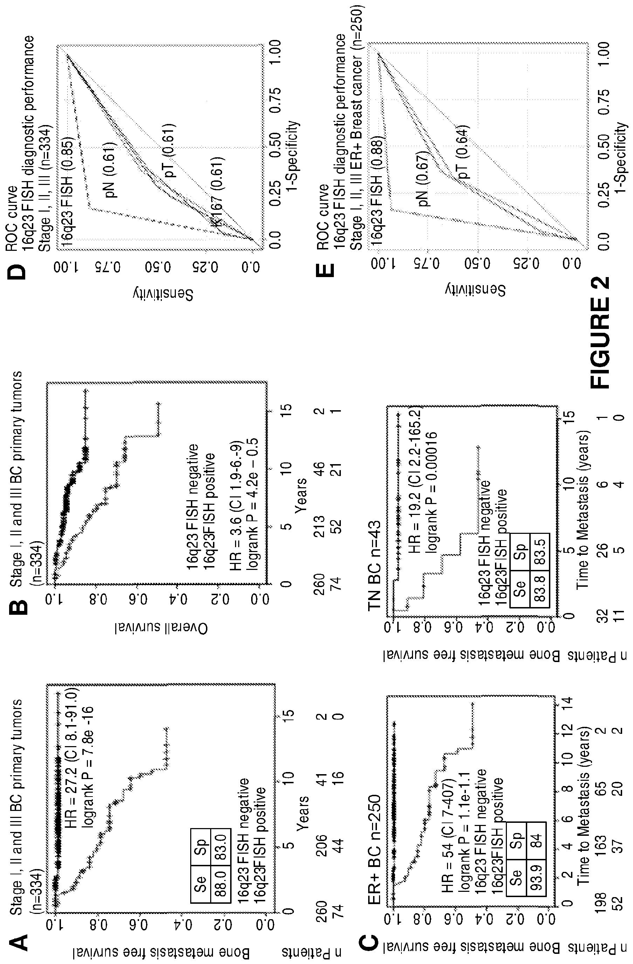

Figure 2. Amplification of 16q23 genomic DNA region, probe used flanks five genes VAT1L, CLEC3A, WWOX, the gene the sequence of which is provided in the Gene Database with ID 645957 and MAF (ordered from centromer to telomer), is associated with breast cancer bone metastasis
a and b) Kaplan-Meier curve of bone (a) metastasis-free or overall (bt) survival in stage I, II, and III BC human primary tumor set (n=334). Patients were stratified according to 16q23 FISH negative and 16q23 FISH positive group based on cut-off of 2.5 16q23 copies per cell as an average, using 3 cores per tumor. Se-sensitivity; Sp-specificity; HR-hazard ratio.
c) Kaplan-Meier curve of bone metastasis free survival for ER-positive (left) or triple negative (right) patients in I, II, and III BC human primary tumor set (n=250 and n=43 respectively). Patients were divided to 16q23 FISH negative and 16q23 FISH positive group based on cut-off of 2.5 for 16q23 copies per cell as an average, using 3 cores per tumor. HR-hazard ratio.
d, e) Receiver Operating Characteristic (ROC) curves for diagnostic performance of 16q23 amplification in overall (d) and ER+ breast cancer (e). In a ROC curve the true positive rate (Sensitivity) is plotted in function of the false positive rate (100-Specificity) for different cut-off points. Each point on the ROC curve represents a sensitivity/specificity pair corresponding to a particular decision threshold.

### DETAILED DESCRIPTION OF THE INVENTION

### Definitions of general terms and expressions

As used herein, "agent for avoiding or preventing bone degradation" refers to any molecule capable of preventing, inhibiting, treating, reducing, or stopping bone degradation either by stimulating the osteoblast proliferation or inhibiting the osteoclast proliferation.

As used herein, the term "amplification of a gene" refers to a process through which various copies of a gene or of a gene fragment are formed in an individual cell or a cell line. The copies of the gene are not necessarily located in the same chromosome. The duplicated region is often called an "amplicon". Normally, the amount of mRNA produced, *i.e.,* the gene expression level also increases in proportion to the copy number of a particular gene.

As used herein, "cancer" refers to any cancer. In some embodiments, the cancer may be breast cancer (including triple negative, basal-like and ER+ breast cancer), prostate cancer, lung cancer, thyroid cancer, or renal cell carcinoma.

As used herein, the term "basal-like" "basal-like subtype," "breast cancer of the basal-like subtype" and the like, as used herein, refers to a particular subtype of breast cancer characterized by the two negative receptors ER and HER2 and at least one positive receptor of the group consisting of CK5/6, CK14, CK17 and EGFR. Thus, all sentences in the present application which cite and refer to triple negative breast cancer (ER, HER-2, PgR) can also be cited and refer also to basal-like breast cancer wherein ER and HER2 are negative and wherein at least one of CK5/6, CK14, CK17 and EGFR is positive. Alternatively, "basal-like" also refers to breast cancer characterized by a gene expression profile based on the up-regulation and/or down-regulation of the following ten genes: (1) Forkhead box CI (FOXC 1); (2) Melanoma inhibitory activity (MIA); (3) NDC80 homolog, kinetochore complex component (KNTC2); (4) Centrosomal protein 55kDa (CEP55); (5) Anillin, actin binding protein (ANLN); (6) Maternal embryonic leucine zipper kinase (MELK); (7) G protein-coupled receptor 160 (GPR160); (8) Transmembrane protein 45B (TMEM45B); (9) Estrogen receptor 1 (ESR1); (10) Forkhead box Al (FOXA1). Because the gene expression profile used to classify breast cancer tumors as basal-like subtype does not include the estrogen receptor, the progesterone receptor or Her2, both triple negative and non-triple negative breast cancers may be classified as basal-like subtype.

As used herein, "Triple-negative breast cancer" refers to a breast cancer which is characterized by a lack of detectable expression of both ER and PR (preferably when the measures of expression of ER and PR are carried out by the method disclosed by M. Elizabeth H et al., Journal of Clinical Oncology, 28(16): 2784-2795, 2010) and the tumor cells are not amplified for epidermal growth factor receptor type 2 (HER2 or ErbB2), a receptor normally located on the cell surface. Tumor cells are considered negative for expression of ER and PR if less than 5 percent of the tumor cell nuclei are stained for ER and PR expression using standard immunohistochemical techniques. As used herein, tumor cells are considered negative for HER2 overexpression if they yield a test result score of 0 or 1+, or 2+ when tested with a HercepTest™ Kit (Code K5204, Dako North America, Inc., Carpinteria, CA), a semi-quantitative immunohistochemical assay using a polyclonal anti-HER2 primary antibody or if they are HER2 FISH negative.

"Lung cancer" refers to any cancer that originates in the lungs. Lung cancer consist of four major types of lung cancer and multiple minor or rare forms. For clinico-pathological reasons they are often divided into the broad categories of small-cell lung cancer (SCLC), also called oat cell cancer, and non-small-cell lung cancer (NSCLC). NSCLC is further divided into three major types, squamous cell carcinoma (SCC), adenocarcinoma and large cell carcinomas.

"Prostate cancer" refers to any cancer that originates in the prostate. Prostate cancer is classified as an adenocarcinoma, or glandular cancer, that begins when normal semen-secreting prostate gland cells mutate into cancer cells. The region of prostate gland where the adenocarcinoma is most common is the peripheral zone. Initially, small clumps of cancer cells remain confined to otherwise normal prostate glands, a condition known as carcinoma in situ or prostatic intraepithelial neoplasia (PIN). Although there is no proof that PIN is a cancer precursor, it is closely associated with cancer.

"Thyroid cancer" includes cancers derived from both follicular thyroid cells and parafollicular C cells. Follicular thyroid cell-derived tumours include papillary cancer (PTC), follicular cancer (FTC), poorly differentiated cancer (PDTC) and anaplastic cancer (ATC). PTC and FTC are collectively classified as differentiated cancer (DTC). Parafollicular C cell-derived medullary cancer (MTC) is derived from parafollicular C cells. Other types of cancer include thyroid lymphoma, squamous cell thyroid carcinoma and sarcoma of the thyroid.

As used herein, "gene of interest" refers to any gene in the 16q22-24 locus. The gene of interest may be MAF. The gene of interest may be VAT1L (Gene ID: 57687 located at Chromosome: 16; NC_000016.9 (77822483..78014001)). The gene of interest may be CLEC3A (Gene ID: 10143, located at Chromosome: 16; NC_000016.9 (78056443..78066003)). The gene of interest may be WWOX (Gene ID: 51741, Chromosome: 16; NC_000016.9 (78133327..79246564). The gene of interest may be the gene the sequence of which is provided in the Gene Database with ID 645957 (located at Chromosome: 16; NC_000016.9 (78859149..78859982).

As used herein, "c-MAF gene" or "MAF" (v-maf musculoaponeurotic fibrosarcoma oncogene homologue (avian) also known as MAF or MGC71685) is a transcription factor containing a leucine zipper which acts like a homodimer or a heterodimer. Depending on the DNA binding site, the encoded protein can be a transcriptional activator or repressor. The DNA sequence encoding c-MAF is described in the NCBI database under accession number NG_016440 (SEQ ID NO: 1 (genomic)). The coding sequence of c-MAF is set forth in SEQ ID NO: 13. The methods of the present invention may utilize either the coding sequence or the genomic DNA sequence. Two messenger RNA are transcribed from said DNA sequence, each of which will give rise to one of the two c-MAF protein isoforms, the α isoform and the β isoform. The complementary DNA sequences for each of said isoforms are described, respectively, in the NCBI database under accession numbers NM_005360.4 (SEQ ID NO: 2) and NM_001031804.2 (SEQ ID NO: 3).

As used herein, a "c-MAF inhibitory agent" refers to any molecule capable of completely or partially inhibiting the c-MAF gene expression, both by preventing the expression product of said gene from being produced (interrupting the c-MAF gene transcription and/or blocking the translation of the mRNA coming from the c-MAF gene expression) and by directly inhibiting the c-MAF protein activity. C-MAF gene expression inhibitors can be identified using methods based on the capacity of the so-called inhibitor to block the capacity of c-MAF to promote the *in vitro* cell proliferation, such as shown in the international patent application WO2005/046731, based on the capacity of the so-called inhibitor to block the transcription capacity of a reporter gene under the control of the cyclin D2 promoter or of a promoter containing the c-MAF response region (MARE or c-MAF responsive element) in cells which express c-MAF such as described in WO2008098351 or based on the capacity of the so-called inhibitor to block the expression of a reporter gene under the control of the IL-4 promoter in response to the stimulation with PMA/ionomycin in cells which express NFATc2 and c-MAF such as described in US2009048117A.

As used herein, Mammalian target of rapamycin (mTOR) or "mTor" refers to those proteins that correspond to EC 2.7.11.1. mTor enzymes are serine/threonine protein kinases and regulate cell proliferation, cell motility, cell growth, cell survival, and transcription.

As used herein, an "mTor inhibitor" refers to any molecule capable of completely or partially inhibiting the mTor gene expression, both by preventing the expression product of said gene from being produced (interrupting the mTor gene transcription and/or blocking the translation of the mRNA coming from the mTor gene expression) and by directly inhibiting the mTor protein activity, including inhibitors that have a dual or more targets and among them mTor protein activity.

As used herein, "Src" refers to those proteins that correspond to EC 2.7.10.2. Src is a non-receptor tyrosine kinase and a proto-oncogene. Src may play a role in cell growth and embryonic development.

As used herein, a "Src inhibitor" refers to any molecule capable of completely or partially inhibiting the Src gene expression, both by preventing the expression product of said gene from being produced (interrupting the Src gene transcription and/or blocking the translation of the mRNA coming from the Src gene expression) and by directly inhibiting the Src protein activity.

As used herein, "Prostaglandin-endoperoxide synthase 2", "cyclooxygenase-2" or "COX-2" refers to those proteins that correspond to EC 1.14.99.1. COX-2 is responsible for converting arachidonic acid to prostaglandin endoperoxide H2.

As used herein, a "COX-2 inhibitor" refers to any molecule capable of completely or partially inhibiting the COX-2 gene expression, both by preventing the expression product of said gene from being produced (interrupting the COX-2 gene transcription and/or blocking the translation of the mRNA coming from the COX-2 gene expression) and by directly inhibiting the COX-2 protein activity.

As used herein "outcome" or "clinical outcome" refers to the resulting course of disease and/or disease progression and can be characterized for example by recurrence, period of time until recurrence, metastasis, period of time until metastasis, number of metastases, number of sites of metastasis and/or death due to disease. For example a good clinical outcome includes cure, prevention of recurrence, prevention of metastasis and/or survival within a fixed period of time (without recurrence), and a poor clinical outcome includes disease progression, metastasis and/or death within a fixed period of time.

As used herein, "ER+ breast cancer" is understood as breast cancer the tumor cells of which express the estrogen receptor (ER). This makes said tumors sensitive to estrogen, meaning that the estrogen makes the cancerous breast tumor grow. In contrast, "ER- breast cancer" is understood as breast cancer the tumor cells of which do not express the estrogen receptor (ER).

As used herein, the term "expression level" of a gene as used herein refers to the measurable quantity of gene product produced by the gene in a sample of the subject, wherein the gene product can be a transcriptional product or a translational product. Accordingly, the expression level can pertain to a nucleic acid gene product such as mRNA or cDNA or a polypeptide gene product. The expression level is derived from a subject's sample and/or a reference sample or samples, and can for example be detected de novo or correspond to a previous determination. The expression level can be determined or measured, for example, using microarray methods, PCR methods (such as qPCR), and/or antibody based methods, as is known to a person of skill in the art.

As used herein, the term "gene copy number" refers to the copy number of a nucleic acid molecule in a cell. The gene copy number includes the gene copy number in the genomic (chromosomal) DNA of a cell. In a normal cell (non-tumoral cell), the gene copy number is normally two copies (one copy in each member of the chromosome pair). The gene copy number sometimes includes half of the gene copy number taken from samples of a cell population.

"Increased expression level" is understood as the expression level when it refers to the levels of the c-MAF gene greater than those in a reference sample or control sample. Particularly, a sample can be considered to have high c-MAF expression level when the expression level in the sample isolated from the patient is at least about 1.1 times, 1.5 times, 5 times, 10 times, 20 times, 30 times, 40 times, 50 times, 60 times, 70 times, 80 times, 90 times, 100 times or even more with respect to the reference or control.

"Probe", as used herein, refers to an oligonucleotide sequence that is complementary to a specific nucleic acid sequence of interest. In some embodiments, the probes may be specific to regions of chromosomes which are known to undergo translocations. In some embodiments, the probes have a specific label or tag. In some embodiments, the tag is a fluorophore. In some embodiments, the probe is a DNA *in situ* hybridization probe whose labeling is based on the stable coordinative binding of platinum to nucleic acids and proteins. In some embodiments, the probe is described in U.S. Patent Appl. 12/067532 and U.S. Patent Appl. 12/181,399, or as described in Swennenhuis et al. "Construction of repeat-free fluorescence in situ hybridization probes" Nucleic Acids Research 40(3):e20 (2012).

"Tag" or "label", as used herein, refers to any physical molecule which is directly or indirectly associated with a probe, allowing the probe or the location of the probed to be visualized, marked, or otherwise captured.

"Translocation", as used herein, refers to the exchange of chromosomal material in unequal or equal amounts between chromosomes. In some cases, the translocation is on the same chromosome. In some cases, the translocation is between different chromosomes. Translocations occur at a high frequency in many types of cancer, including breast cancer and leukemia. Translocations can be either primary reciprocal translocations or the more complex secondary translocations. There are several primary translocations that involve the immunoglobulin heavy chain (IgH) locus that are believed to constitute the initiating event in many cancers. (Eychène, A., Rocques, N., and Puoponnot, C., A new MAFia in cancer. 2008. Nature Reviews: Cancer. 8: 683-693.)

"Polyploid" or "polyploidy", as used herein, indicates that the cell contains more than two copies of a gene of interest. In some instances, the gene of interest is MAF. In some embodiments, polyploidy is associated with an accumulation of expression of the gene of interest. In some embodiments, polyploidy is associated with genomic instability. In some embodiments, the genomic instability may lead to chromosome translocations.

"Whole genome sequencing", as used herein, is a process by which the entire genome of an organism is sequenced at a single time. *See, e.g.,* Ng., P.C. and Kirkness, E.F., Whole Genome Sequencing. 2010. Methods in Molecular Biology. 628: 215-226.

"Exome sequencing" or "exosome sequencing", as used herein, is a process by which the entire coding region of the DNA of an organism is sequenced. In exome sequencing, the mRNA is sequenced. The untranslated regions of the genome are not included in exome sequencing. *See, e.g.,* Choi, M. et al., Genetic diagnosis by whole exome capture and massively parallel DNA sequencing. 2009. PNAS. 106(45): 19096-19101.

"Metastasis", as used herein, is understood as the propagation of a cancer from the organ where it started to a different organ. It generally occurs through the blood or lymphatic system. When the cancer cells spread and form a new tumor, the latter is called a secondary or metastatic tumor. The cancer cells forming the secondary tumor are like those of the original tumor. If a breast cancer, for example, spreads (metastasizes) to the lung, the secondary tumor is formed of malignant breast cancer cells. The disease in the lung is metastatic breast cancer and not lung cancer. In a particular embodiment of the method of the invention, the metastasis has spread (metastasized) to the bone.

"Predicting", as used herein, refers to the determination of the likelihood that the subject suffering from cancer will develop metastasis to a distant organ. As used herein, "good prognosis" indicates that the subject is expected (e.g. predicted) to survive and/or have no, or is at low risk of having, recurrence or distant metastases within a set time period. The term "low" is a relative term and, in the context of this application, refers to the risk of the "low" expression group with respect to a clinical outcome (recurrence, distant metastases, etc.). A "low" risk can be considered as a risk lower than the average risk for a heterogeneous cancer patient population. In the study of Paik et al. (2004), an overall "low" risk of recurrence was considered to be lower than 15 percent. The risk will also vary in function of the time period. The time period can be, for example, five years, ten years, fifteen years or even twenty years after initial diagnosis of cancer or after the prognosis was made.

As used herein, "poor prognosis" indicates that the subject is expected, e.g. predicted to not survive and/or to have, or is at high risk of having, recurrence or distant metastases within a set time period. The term "high" is a relative term and, in the context of this application, refers to the risk of the "high" expression group with respect to a clinical outcome (recurrence, distant metastases, etc.). A "high" risk can be considered as a risk higher than the average risk for a heterogeneous cancer patient population. In the study of Paik et al. (2004), an overall "high" risk of recurrence was considered to be higher than 15 percent. The risk will also vary in function of the time period. The time period can be, for example, five years, ten years, fifteen years or even twenty years of initial diagnosis of cancer or after the prognosis was made.

"Reference value", as used herein, refers to a laboratory value used as a reference for values/data obtained by laboratory examinations of patients or samples collected from patients. The reference value or reference level can be an absolute value; a relative value; a value that has an upper and/or lower limit; a range of values; an average value; a median value, a mean value, or a value as compared to a particular control or baseline value. A reference value can be based on an individual sample value, such as for example, a value obtained from a sample from the subject being tested, but at an earlier point in time. The reference value can be based on a large number of samples, such as from a population of subjects of the chronological age matched group, or based on a pool of samples including or excluding the sample to be tested.

As used herein, "Subject" or "patient" refers to all animals classified as mammals and includes but is not limited to domestic and farm animals, primates and humans, for example, human beings, non-human primates, cows, horses, pigs, sheep, goats, dogs, cats, or rodents. Preferably, the subject is a human man or woman of any age or race.

The term "treatment", as used herein, refers to any type of therapy, which aims at terminating, preventing, ameliorating or reducing the susceptibility to a clinical condition as described herein. In a preferred embodiment, the term treatment relates to prophylactic treatment (i.e. a therapy to reduce the susceptibility to a clinical condition), of a disorder or a condition as defined herein. Thus, "treatment," "treating," and their equivalent terms refer to obtaining a desired pharmacologic or physiologic effect, covering any treatment of a pathological condition or disorder in a mammal, including a human. The effect may be prophylactic in terms of completely or partially preventing a disorder or symptom thereof and/or may be therapeutic in terms of a partial or complete cure for a disorder and/or adverse effect attributable to the disorder. That is, "treatment" includes (1) preventing the disorder from occurring or recurring in a subject, (2) inhibiting the disorder, such as arresting its development, (3) stopping or terminating the disorder or at least symptoms associated therewith, so that the host no longer suffers from the disorder or its symptoms, such as causing regression of the disorder or its symptoms, for example, by restoring or repairing a lost, missing or defective function, or stimulating an inefficient process, or (4) relieving, alleviating, or ameliorating the disorder, or symptoms associated therewith, where ameliorating is used in a broad sense to refer to at least a reduction in the magnitude of a parameter, such as inflammation, pain, or immune deficiency.

As used herein, "sample" or "biological sample" means biological material isolated from a subject. The biological sample may contain any biological material suitable for determining the expression level of the c-MAF gene. The sample can be isolated from any suitable biological tissue or fluid such as, for example, tumor tissue, blood, blood plasma, serum, urine or cerebral spinal fluid (CSF).

"Tumor tissue sample" is understood as the tissue sample originating from the primary cancer tumor. Said sample can be obtained by conventional methods, for example biopsy, using methods well known by the persons skilled in related medical techniques.

"Osteolytic bone metastasis" refers to a type of metastasis in which bone resorption (progressive loss of the bone density) is produced in the proximity of the metastasis resulting from the stimulation of the osteoclast activity by the tumor cells and is characterized by severe pain, pathological fractures, hypercalcaemia, spinal cord compression and other syndromes resulting from nerve compression.

In a first aspect, the invention relates to an *in vitro* method (hereinafter first method of the invention) for the prognosis of the tendency to develop metastasis and/or recurrence in a subject suffering of cancer which comprises:
i) quantifying the copy number or amplification of a gene of interest selected from VAT1L, CLEC3A or the gene the sequence of which is provided in the Gene Database with ID 645957, or quantifying the expression level, copy number or amplification of the gene of interest WWOX in a sample of said subject and
ii) comparing the copy number, amplification or expression level obtained in step i) with a reference value,
wherein an increased copy number, amplification or expression level of said gene of interest with respect to said reference value is indicative of increased risk of developing metastasis and/or recurrence.

The method of the invention comprises in a first step determining the copy number, amplification or expression level of the gene of interest in a sample from a subject. In a preferred embodiment, the sample is a tumor tissue sample.

The methods for obtaining a biopsy sample include splitting a tumor into large pieces, or microdissection, or other cell separating methods known in the art. The tumor cells can additionally be obtained by means of cytology through aspiration with a small gauge needle. To simplify sample preservation and handling, samples can be fixed in formalin and soaked in paraffin or first frozen and then soaked in a tissue freezing medium such as OCT compound by means of immersion in a highly cryogenic medium which allows rapid freezing.

In a preferred embodiment, the first method of the invention comprises quantifying only the gene of interest expression level as a single marker, *i.e.,* the method does not involve determining the expression level of any additional marker.

As understood by the person skilled in the art, the gene expression level can be quantified by measuring the messenger RNA levels of said gene or of the protein encoded by said gene, as well as the number of genomic region copies or translocations containing said gene.

For this purpose, the biological sample can be treated to physically or mechanically break up the tissue or cell structure, releasing the intracellular components into an aqueous or organic solution for preparing nucleic acids. The nucleic acids are extracted by means of commercially available methods known by the person skilled in the art (Sambrook, J., et al., "Molecular cloning: a Laboratory Manual", 3rd ed., Cold Spring Harbor Laboratory Press, N.Y., Vol. 1-3.)

Thus, the gene of interest expression level can be quantified from the RNA resulting from the transcription of said gene (messenger RNA or mRNA) or, alternatively, from the complementary DNA (cDNA) of said gene. Therefore, in a particular disclosure, the quantification of the gene of interest expression level comprises the quantification of the messenger RNA of the c-MAF gene or a fragment of said mRNA, complementary DNA of the c-MAF gene or a fragment of said cDNA or the mixtures thereof.

Virtually any conventional method can be used within the scope of the disclosure for detecting and quantifying the mRNA levels encoded by MAF, VAT1L CLEC3, WWOX, or the gene the sequence of which is provided in the Gene Database with ID 645957 or of the corresponding cDNA thereof. By way of non-limiting illustration, the mRNA levels encoded by said gene can be quantified using conventional methods, for example, methods comprising mRNA amplification and the quantification of said mRNA amplification product, such as electrophoresis and staining, or alternatively, by Southern blot and using suitable probes, Northern blot and using specific probes of the mRNA of the gene of interest or of the corresponding cDNA thereof, mapping with S1 nuclease, RT-PCR, hybridization, microarrays, etc., preferably by means of real time quantitative PCR using a suitable marker. Likewise, the cDNA levels corresponding to said mRNA encoded by the gene of interest can also be quantified by means of using conventional techniques; in this case, the method of the invention includes a step for synthesizing the corresponding cDNA by means of reverse transcription (RT) of the corresponding mRNA followed by the amplification and quantification of said cDNA amplification product. Conventional methods for quantifying expression level can be found, for example, in Sambrook *et al.,* 2001 (cited *ad supra*). These methods are known in the art and a person skilled in the art would be familiar with the normalizations necessary for each technique. For example, the expression measurements generated using multiplex PCR should be normalized by comparing the expression of the genes being measured to so called "housekeeping" genes, the expression of which should be constant over all samples, thus providing a baseline expression to compare against or other control genes whose expression are known to be modulated with cancer.

In a particular embodiment, the gene of interest expression level is quantified by means of quantitative polymerase chain reaction (PCR) or a DNA/RNA array or nucleotide hybridization technique.

In an additional disclosure, the gene of interest expression level can also be quantified by means of quantifying the expression level of the protein encoded by said gene, *e.g.* the c-MAF protein (c-MAF) [NCBI, accession number 075444], or any functionally equivalent variant of the c-MAF protein, the VAT1L protein, the CLEC3A protein, the WWOX protein, the gene the sequence of which is provided in the Gene Database with ID 645957 protein. There are two c-MAF protein isoforms, the α isoform (NCBI, NP_005351.2) made up of 403 amino acids (SEQ ID NO: 4) and the β isoform (NCBI, NP_001026974.1) made up of 373 amino acids (SEQ ID NO: 5). The c-MAF gene expression level can be quantified by means of quantifying the expression level of any of the c-MAF protein isoforms. Thus, in a particular embodiment, the quantification of the level of the protein encoded by the c-MAF gene comprises the quantification of the c-MAF protein.

In the context of the present invention, "functionally equivalent variant of the gene of interest", *e.g*., c-MAF, VAT1L, CLEC3A, WWOX, and the gene the sequence of which is provided in the Gene Database with ID 645957, is understood as (i) variants of the gene of interest protein in which one or more of the amino acid residues are substituted by a conserved or non-conserved amino acid residue (preferably a conserved amino acid residue), wherein such substituted amino acid residue may or may not be one encoded by the genetic code, or (ii) variants comprising an insertion or a deletion of one or more amino acids and having the same function as the gene of interest protein, *i.e.,* to act as a DNA binding transcription factor. Variants of the gene of interest protein can be identified using methods based on the capacity of gene of interest for promoting *in vitro* cell proliferation as shown in international patent application WO2005/046731, based on the capacity of the so-called inhibitor for blocking the transcription capacity of a reporter gene under the control of cyclin D2 promoter or of a promoter containing the c-MAF responsive region (MARE or c-MAF responsive element) in cells expressing c-MAF as described in WO2008098351, or based on the capacity of the so-called inhibitor for blocking reporter gene expression under the control of the IL-4 promoter in response to the stimulation with PMA/ionomycin in cells expressing NFATc2 and c-MAF as described in US2009048117A.

The variants according to the invention preferably have sequence similarity with the amino acid sequence of any of the genes of interest of at least about 50%, at least about 60%, at least about 70%, at least about 80%, at least about 90%, at least about 91%, at least about 92%, at least about 93%, at least about 94%, at least about 95%, at least about 96%, at least about 97%, at least about 98% or at least about 99%. The degree of similarity between the variants and the specific gene of interest protein sequences defined previously is determined using algorithms and computer processes which are widely known by the persons skilled in the art. The similarity between two amino acid sequences is preferably determined using the BLASTP algorithm [BLAST Manual, Altschul, S., *et al.,* NCBI NLM NIH Bethesda, Md. 20894, Altschul, S., et al., J. Mol. Biol. 215: 403-410 (1990)].

The protein expression level of the gene of interest can be quantified by any conventional method which allows detecting and quantifying said protein in a sample from a subject. By way of non-limiting illustration, said protein levels can be quantified, for example, by using antibodies with binding capacity for the gene of interest (or a fragment thereof containing an antigenic determinant) and the subsequent quantification of the complexes formed. The antibodies used in these assays may or may not be labeled. Illustrative examples of markers that can be used include radioactive isotopes, enzymes, fluorophores, chemiluminescence reagents, enzyme substrates or cofactors, enzyme inhibitors, particles, dyes, etc. There is a wide range of known assays that can be used in the present invention which use unlabeled antibodies (primary antibody) and labeled antibodies (secondary antibody); these techniques include Western-blot or Western transfer, ELISA (enzyme-linked immunosorbent assay), RIA (radioimmunoassay), competitive EIA (competitive enzyme immunoassay), DAS-ELISA (double antibody sandwich ELISA), immunocytochemical and immunohistochemical techniques, techniques based on the use of protein microarrays or biochips including specific antibodies or assays based on colloidal precipitation in formats such as dipsticks. Other ways for detecting and quantifying said gene of interest protein include affinity chromatography techniques, ligand binding assays, etc. When an immunological method is used, any antibody or reagent that is known to bind to the gene of interest protein with a high affinity can be used for detecting the amount thereof. Nevertheless, the use of an antibody, for example, polyclonal sera, supernatants of hybridomas or monoclonal antibodies, antibody fragments, Fv, Fab, Fab' and F(ab')2, scFv, humanized diabodies, triabodies, tetrabodies, nanobodies, alphabodies, stapled peptides, cyclopeptides and antibodies is preferred. For example, there are commercial anti-c-MAF protein antibodies on the market which can be used in the context of the present invention, such as for example antibodies ab427, ab55502, ab55502, ab72584, ab76817, ab77071 (Abcam plc, 330 Science Park, Cambridge CB4 OFL, United Kingdom), the 075444 monoclonal antibody (Mouse Anti-Human MAF Azide free Monoclonal antibody, Unconjugated, Clone 6b8) of AbD Serotec, etc. There are many commercial companies offering anti-c-MAF antibodies, such as Abnova Corporation, Bethyl Laboratories, Santa Cruz Biotechnology, Bioworld Technology, GeneTex, etc.

In a particular embodiment, the protein levels of the gene of interest are quantified by means of western blot, immunohistochemistry, ELISA or a protein array.

In another particular embodiment, the protein levels of the gene of interest are quantified from exosomes or circulating DNA. Exosomes are 40 - 100 nm membrane vesicles secreted by most cell types in *vivo* and *in vitro.* Exosomes form in a particular population of endosomes, called multivesicular bodies (MVBs) by inward budding into the lumen of the compartment. Upon fusion of MVBs with the plasma membrane, these internal vesicles are secreted. Exosomes can be isolated from diverse cell lines or body fluids by several methods well known in the art (Théry C. et al., Curr Protoc Cell Biol. 2006 Apr;Chapter 3:Unit 3.22). Several commercial kits are available for the isolation of exosomes such as ExoQuick™ or ExoTest™.

The first method of the invention comprises in a second step comparing expression level of a gene of interest obtained in the sample (*e.g*., tumor sample) from the subject with a reference value.

Once the expression level of the gene of interest in a sample from a subject with cancer, have been measured and compared with the reference value, if the expression level of said gene is increased with respect to said reference value, then it can be concluded that said subject has a greater tendency to develop bone metastasis.

The determination of the expression level of the gene of interest must be correlated with the reference value.

In an embodiment, reference value(s) as intended herein may convey absolute quantities of the gene of interest. In another embodiment, the quantity of any one or more biomarkers in a sample from a tested subject may be determined directly relative to the reference value (*e.g.,* in terms of increase or decrease, or fold-increase or fold-decrease). Advantageously, this may allow to compare the quantity of any one or more biomarkers in the sample from the subject with the reference value (in other words to measure the relative quantity of any one or more biomarkers in the sample from the subject vis-a-vis the reference value) without the need to first determine the respective absolute quantities of said one or more biomarkers.

In a preferred embodiment, the reference value is the expression level of the gene of interest in a control sample or reference sample. Depending on the type of tumor to be analyzed, the exact nature of the control or reference sample may vary. Thus, in the event that a prognosis is to be evaluated, then the reference sample is a sample from a subject with cancer, that has not metastasized or that corresponds to the median value of the expression level of the gene of interest measured in a tumor tissue collection in biopsy samples from subjects with cancer, which have not metastasized.

Said reference sample is typically obtained by combining equal amounts of samples from a subject population. Generally, the typical reference samples will be obtained from subjects who are clinically well documented and in whom the absence of metastasis is well characterized. In such samples, the normal concentrations (reference concentration) of the biomarker (gene of interest) can be determined, for example by providing the mean concentration over the reference population. Various considerations are taken into account when determining the reference concentration of the marker. Among such considerations are the age, weight, sex, general physical condition of the patient and the like. For example, equal amounts of a group of at least about 2, at least about 10, at least about 100 to preferably more than about 1000 subjects, preferably classified according to the foregoing considerations, for example according to various age categories, are taken as the reference group. The sample collection from which the reference level is derived will preferably be formed by subjects suffering from the same type of cancer as the patient object of the study.

In a particular embodiment the reference values for "increased" or "reduced" expression of the gene of interest expression are determined by calculating the percentiles by conventional means which involves performing assays in one or several samples isolated from subjects whose disease is well documented by any of the methods mentioned above the gene of interest expression level. The "reduced" level of the gene of interest can then preferably be assigned to samples wherein the gene of interest expression level is equal to or lower than 50^{th} percentile in the normal population including, for example, expression level equal to or lower than the 60^{th} percentile in the normal population, equal to or lower than the 70^{th} percentile in the normal population, equal to or lower than the 80^{th} percentile in the normal population, equal to or lower than the 90^{th} percentile in the normal population, and equal to or lower than the 95^{th} percentile in the normal population. The "increased" expression level of the gene of interest can then preferably be assigned to samples wherein the c-MAF gene expression level is equal to or greater than the 50^{th} percentile in the normal population including, for example, expression level equal to or greater than the 60^{th} percentile in the normal population, equal to or greater than the 70^{th} percentile in the normal population, equal to or greater than the 80^{th} percentile in the normal population, equal to or greater than the 90^{th} percentile in the normal population, and equal to or greater than the 95^{th} percentile in the normal population.

The person skilled in the art will understand that the prediction of the tendency for a primary cancer tumor to metastasize is not needed to be correct for all the subjects to be identified (*i.e.,* for 100% of the subjects). Nevertheless, the term requires enabling the identification of a statistically significant part of the subjects (for example, a cohort in a cohort study). Whether a part is statistically significant can be determined in a simple manner by the person skilled in the art using various well known statistical evaluation tools, for example, the determination of confidence intervals, determination of p values, Student's T test, Mann-Whitney test, etc. Details are provided in Dowdy and Wearden, Statistics for Research, John Wiley and Sons, New York 1983. The preferred confidence intervals are at least 90%, at least 95%, at least 97%, at least 98% or at least 99%. The p values are preferably 0.1, 0.05, 0.01, 0.005 or 0.0001. More preferably, at least 60%, at least 70%, at least 80% or at least 90% of the subjects of a population can be suitably identified by the method of the present invention.

In yet another embodiment, the metastasis to bone is an osteolytic bone metastasis.

In yet another embodiment, an expression level of the gene of interest which is above the average indicates increased risk of bone metastasis, being said risk is proportional to the levels of expression of the gene of interest. Thus, the risk of bone metastasis in a subject suffering cancer is dose-dependent.

### Method for predicting the clinical outcome of a patient suffering bone metastasis from cancer, based on the expression level of a gene of interest

In another aspect, the disclosure relates to an *in vitro* method for using a probe to predict the clinical outcome of a patient suffering bone metastatic cancer which comprises:
i) using a probe to quantify the expression level of a gene of interest in a sample of said subject and
ii) comparing the expression level obtained in step i) with a reference value,
wherein increased expression level of said gene with respect to said reference value is indicative of a poor clinical outcome.

The disclosure comprises in a first step, quantifying expression level of a gene of interest in a sample of a subject suffering cancer. In a preferred embodiment, the sample is a tumor tissue sample.

In a preferred embodiment, the disclosure comprises quantifying only the expression level of the gene of interest as a single marker, *i.e.,* the method does not involve determining the expression level of any additional marker.

In a second step, the expression level of the gene of interest obtained in the tumor sample of the subject is compared with a reference value. In a preferred embodiment, the reference value is the expression level of said gene in a control sample. The determination of the expression level of the gene of interest must be correlated to values of a control sample or reference sample. Depending on the type of tumor to be analyzed, the exact nature of the control sample may vary. Thus, in the case involving the second method of the invention, then the reference sample is a sample of subject with cancer who has not suffered bone metastasis or that corresponds to the median value of the expression level of the gene of interest measured in a tumor tissue collection in biopsy samples of subjects with cancer who have not suffered metastasis.

Once the expression level of the gene of interest in the sample is measured and compared with the control sample, if the expression level of said gene is increased with respect to its expression level in the control sample, then it is indicative of a poor clinical outcome.

In a specific embodiment, the bone metastasis is osteolytic metastasis.

In another specific embodiment, the quantification of the expression level of the gene of interest comprises quantifying the messenger RNA (mRNA) of said gene, or a fragment of said mRNA, the complementary DNA (cDNA) of said gene, or a fragment of said cDNA. In a more preferred embodiment, the expression level is quantified by means of a quantitative polymerase chain reaction (PCR) or a DNA or RNA array.

In another embodiment, the quantification of the expression level of the gene of interest comprises quantifying the level of protein encoded by said gene or of a variant thereof. In a yet more preferred embodiment, the protein level is determined by means of Western blot, immunohistochemistry, ELISA or a protein array.

In another embodiment, the reference sample is a tumor tissue sample of cancer, from a subject who has not suffered metastasis.

Any parameter which is widely accepted for determining clinical outcome of a patient can be used in the present invention including, without limitation:
- disease-free progression which, as used herein, describes the proportion of subjects in complete remission who have had no recurrence of disease during the time period under study.
- disease-free survival (DFS), as used herewith, is understood as the length of time after treatment for a disease during which a subject survives with no sign of the disease.
- objective response which, as used in the present invention, describes the proportion of treated subjects in whom a complete or partial response is observed.
- tumour control which, as used in the present invention, relates to the proportion of treated subjects in whom complete response, partial response, minor response or stable disease ≥ 6 months is observed.
- progression free survival which, as used herein, is defined as the time from start of treatment to the first measurement of cancer growth.
- Time to progression (TTP), as used herein, relates to the time after a disease is treated until the disease starts to get worse. The term "progression" has been previously defined.
- six-month progression free survival or "PFS6" rate which, as used herein, relates to the percentage of subjects who are free of progression in the first six months after the initiation of the therapy and
- median survival which, as used herein, relates to the time at which half of the subjects enrolled in the study are still alive.

The terms "poor" or "good", as used herein to refer to a clinical outcome, mean that the subject will show a favourable or unfavourable outcome. As will be understood by those skilled in the art, such the assessment of the probability, although preferred to be, may not be correct for 100% of the subjects to be diagnosed. The term, however, requires that a statistically significant portion of subjects can be identified as having a predisposition for a given outcome. Whether a portion is statistically significant can be determined readily by the person skilled in the art using various well known statistic evaluation tools, *e.g.*, determination of confidence intervals, p-value determination, Student's t-test, Mann-Whitney test, etc. Details are found in Dowdy and Wearden, Statistics for Research, John Wiley & Sons, New York 1983. Preferred confidence intervals are at least about 50%, at least about 60%, at least about 70%, at least about 80%, at least about 90% at least about 95%. The p-values are, preferably, 0.05, 0.01, 0.005, or 0.0001 or less. More preferably, at least about 60 percent, at least about 70 percent, at least about 80 percent or at least about 90 percent of the subjects of a population can be properly identified by the method of the present invention.

### Method for designing customized therapy - in patients with cancer

As is known in the state of the art, the treatment to be administered to a subject suffering from cancer depends on whether the latter is a malignant tumor, *i.e.,* whether it has high probabilities of undergoing metastasis, or whether the latter is a benign tumor. In the first assumption, the treatment of choice is a systemic treatment such as chemotherapy and in the second assumption, the treatment of choice is a localized treatment such as radiotherapy.

Therefore, as described in the present invention, given that overexpression of the gene of interest in cancer cells is related to the presence of bone metastasis, the expression level of the gene of interest is useful for making decisions in terms of the most suitable therapy for the subject suffering said cancer.

Thus, in another aspect the invention relates to an *in vitro* method (hereinafter second method of the invention) for designing a customized therapy for a subject suffering cancer, which comprises
i) quantifying the copy number or amplification of a gene of interest in a sample of said subject, wherein the gene of interest is selected from the group consisting of: VAT1L, CLEC3A, or the gene the sequence of which is provided in the Gene Database with ID 645957 or quantifying the expression level, copy number or amplification of the gene of interest WWOX in a sample of said subject and
ii) comparing the expression level, copy number or amplification obtained in i) with the expression level, copy number or amplification of the gene of interest of a reference value from a control sample,
wherein if the expression level, copy number or degree of amplification of a gene of interest is increased with respect to said reference value from a control sample, then said subject is susceptible to receive a therapy aiming to prevent and/or treat the bone metastasis. If the copy number, degree of amplification or expression level is not increased with respect to said reference value, then said subject is not susceptible to receive a therapy aiming to prevent and/or treat the bone metastasis.

In a particular embodiment, the bone metastasis is osteolytic metastasis.

The second method of the invention comprises in a first step quantifying the expression level of a gene of interest in a sample in a subject suffering from cancer. In a preferred embodiment, the sample is a tumor tissue sample.

In another particular embodiment, the second method of the invention comprises quantifying only the copy number, amplification or expression level gene of interest as a single marker, *i.e.,* the method does not involve determining the copy number, amplification or expression level of any additional marker.

In the case of the second method of the invention the sample can be a primary tumor tissue sample of the subject.

In a second step, the copy number, amplification or expression level of a gene of interest obtained in the tumor sample of the subject is compared with a reference value. In a preferred embodiment, the reference value is the copy number, amplification or expression level of said gene in a control sample. The determination of the expression level of the gene of interest must be related to values of a control sample or reference sample. Depending on the type of tumor to be analyzed, the exact nature of the control sample may vary. Thus preferably the reference sample is a sample of a subject with cancer, that has not metastasized or that corresponds to the median value of the expression level of the gene of interest measured in a tumor tissue collection in biopsy samples of subjects with cancer, which has not metastasized.

Once the copy number, amplification or expression level of the gene of interest in the sample has been measured and compared with the reference value, if the copy number, amplification or expression level of said gene is increased with respect to the reference value, then it can be concluded that said subject is susceptible to receiving therapy aiming to prevent (if the subject has yet to undergo metastasis) and/or treat metastasis (if the subject has already experienced metastasis).

When the cancer has metastasized, systemic treatments including but not limited to chemotherapy, hormone treatment, immunotherapy, or a combination thereof can be used. Additionally, radiotherapy and/or surgery can be used. The choice of treatment generally depends on the type of primary cancer, the size, the location of the metastasis, the age, the general health of the patient and the types of treatments used previously.

The systemic treatments are those that reach the entire body, such as:
- Chemotherapy is the use of medicaments to destroy cancer cells. The medicaments are generally administered through oral or intravenous route. Sometimes, chemotherapy is used together with radiation treatment. Suitable chemotherapeutic treatments for cancer include, without limitation, anthracyclines (doxorubicin, epirubicin, pegylated liposomal doxorubicin), Taxanes (paclitaxel, docetaxel, albumin nano-particle bound paclitaxel), 5-fluorouracil (continuous infusion 5-FU, capecitabine), Vinca alkaloids (vinorelbine, vinblastine), Gemcitabine, Platinum salts (cisplatin, carboplatin), cyclophosphamide, Etoposide and combinations of one or more of the above such as Cyclophosphamide/anthracycline +/- 5-fluorouracil regimens (such as doxorubicin/ cyclophosphamide (AC), epirubicin/cyclophosphamide, (EC) cyclophosphamide/epirubicin/5-fluorouracil (CEF), cyclophosphamide/doxorubicin/5-fluorouracil (CAF), 5-fluorouracil /epirubicin/cyclophosphamide (FEC)), cyclophosphamide/metothrexate/5-fluorouracil (CMF), anthracyclines/taxanes (such as doxorubicin/paclitaxel or doxorubicin/docetaxel), Docetaxel/capecitabine, Gemcitabine/paclitaxel, Taxane/platinum regimens (such as paclitaxel/carboplatin or docetaxel/ carboplatin).
- Immunotherapy is a treatment that aids the immune system itself of the patient to combat cancer. There are several types of immunotherapy which are used to treat metastasis in patients. These include but are not limited to cytokines, monoclonal antibodies and antitumor vaccines.

In another aspect, cancer may require surgery. Common surgeries include thyroidectomy and lobectomy.

In another aspect disclosed herein, radioactive iodine-131 is used in patients with papillary or follicular cancer for ablation of residual thyroid tissue after surgery and for the treatment of cancer. Patients with medullary, anaplastic, and most Hurthle cell cancers do not benefit from this therapy.

In another aspect, external irradiation may be used when the cancer is unresectable, when it recurs after resection, or to relieve pain from bone metastasis.

In another aspect disclosed herein, the treatment is Alpharadin (radium-223 dichloride). Alpharadin uses alpha radiation from radium-223 decay to kill cancer cells. Radium-223 naturally self-targets to bone metastases by virtue of its properties as a calcium-mimic. Alpha radiation has a very short range of 2-10 cells (when compared to current radiation therapy which is based on beta or gamma radiation), and therefore causes less damage to surrounding healthy tissues (particularly bone marrow). With similar properties to calcium, radium-223 is drawn to places where calcium is used to build bone in the body, including the site of faster, abnormal bone growth - such as that seen in the skeletal metastases of men with advanced, castration-resistant prostate cancer. Radium-223, after injection, is carried in the bloodstream to sites of abnormal bone growth. The place where a cancer starts in the body is known as the primary tumor. Some of these cells may break away and be carried in the bloodstream to another part of the body. The cancer cells may then settle in that part of the body and form a new tumor. If this happens it is called a secondary cancer or a metastasis. Most patients with late stage prostate cancer suffer the maximum burden of disease in their bones. The aim with radium-223 is to selectively target this secondary cancer. Any radium-223 not taken-up in the bones is quickly routed to the gut and excreted.

In another aspect disclosed herein, the treatment is vandetanib. Vandetanib is a small-molecule inhibitor of vascular endothelial growth factor receptor (VEGFR), epidermal growth factor receptor (EGFR), and RET tyrosine kinases that has demonstrated clinical benefits in patients with medullary cancer (MTC).

In another aspect, the treatment is sorafenib or sunitinib. Sorafenib and sunitinib are approved for other indications show promise for cancer and are being used for some patients who do not qualify for clinical trials.

In another aspect, the treatment is an mTor inhibitor. In some aspects, the mTor inhibitor is a dual mTor/PI3kinase inhibitor. In some aspects, the mTor inhibitor is used to prevent or inhibit metastasis. In some aspects the mTor inhibitor is selected from the group consisting of: ABI009 (sirolimus), rapamycin (sirolimus), Abraxane (paclitaxel), Absorb (everolimus), Afinitor (everolimus), Afinitor with Gleevec, AS703026 (pimasertib), Axxess (umirolimus), AZD2014, BEZ235, Biofreedom (umirolimus), BioMatrix (umirolimus), BioMatrix flex (umirolimus), CC115, CC223, Combo Bio-engineered Sirolimus Eluting Stent ORBUSNEICH (sirolimus), Curaxin CBLC102 (mepacrine), DE109 (sirolimus), DS3078, Endeavor DES (zotarolimus), Endeavor Resolute (zotarolimus), Femara (letrozole), Hocena (antroquinonol), INK128, Inspiron (sirolimus), IPI504 (retaspimycin hydrochloride), KRN951 (tivozanib), ME344, MGA031 (teplizumab), MiStent SES (sirolimus), MKC1, Nobori (umirolimus), OSI027, OVI123 (cordycepin), Palomid 529, PF04691502, Promus Element (everolimus), PWT33597, Rapamune (sirolimus), Resolute DES (zotarolimus), RG7422, SAR245409, SF1126, SGN75 (vorsetuzumab mafodotin), Synergy (everolimus), Taltorvic (ridaforolimus), Tarceva (erlotinib), Torisel (temsirolimus), Xience Prime (everolimus), Xience V (everolimus), Zomaxx (zotarolimus), Zortress (everolimus), Zotarolimus Eluting Peripheral Stent MEDTRONIC (zotarolimus), AP23841, AP24170, ARmTOR26, BN107, BN108, Canstatin GENZYME (canstatin), CU906, EC0371, EC0565, KI1004, LOR220, NV128, Rapamycin ONCOIMMUNE (sirolimus), SB2602, Sirolimus PNP SAMYANG BIOPHARMACEUTICALS (sirolimus), TOP216, VLI27, VS5584, WYE125132, XL388, Advacan (everolimus), AZD8055, Cypher Select Plus Sirolimus eluting Coronary Stent (sirolimus), Cypher Sirolimus eluting coronary stent (sirolimus), Drug Coated Balloon (sirolimus), E-Magic Plus (sirolimus), Emtor (sirolimus), Esprit (everolimus), Evertor (everolimus), HBF0079, LCP-Siro (sirolimus), Limus CLARIS (sirolimus), mTOR Inhibitor CELLZOME, Nevo Sirolimus eluting Coronary Stent (sirolimus), nPT-mTOR, Rapacan (sirolimus), Renacept (sirolimus), ReZolve (sirolimus), Rocas (sirolimus), SF1126, Sirolim (sirolimus), Sirolimus NORTH CHINA (sirolimus), Sirolimus RANBAXY (sirolimus), Sirolimus WATSON (sirolimus) Siropan (sirolimus), Sirova (sirolimus), Supralimus (sirolimus), Supralimus-Core (sirolimus), Tacrolimus WATSON (tacrolimus), TAFA93, Temsirolimus ACCORD (temsirolimus), Temsirolimus SANDOZ (temsirolimus), TOP216, Xience Prime (everolimus), Xience V (everolimus). In a specific aspect the mTor inhibitor is Afinitor (everolimus) (http://www.afinitor.com/indexjsp?usertrack.filter_applied=true&Novald=40294620643 38207963; last accessed 11/28/2012). In another aspect, everolimus is combined with an aromatase inhibitor. (*See. e.g.,* Baselga, J., et al., Everolimus in Postmenopausal Hormone-Receptor Positive Advanced Breast Cancer. 2012. N. Engl. J. Med. 366(6): 520-529). In another aspect, mTor inhibitors can be identified through methods known in the art. (*See, e.g.,* Zhou, H. et al. Updates of mTor inhibitors. 2010. Anticancer Agents Med. Chem. 10(7): 571-81). In some aspects, the mTor inhibitor is used to treat or prevent or inhibit metastasis in a patient that is positive for a hormone receptor. (*See. e.g.,* Baselga, J., el al., Everolimus in Postmenopausal Hormone-Receptor Positive Advanced Breast Cancer. 2012. N. Engl. J. Med. 366(6): 520-529). In some aspects, the mTor inhibitor is used to treat or prevent or inhibit metastasis in a patient with advanced cancer. In some aspects, the mTor inhibitor is used in combination with a second treatment. In some aspects, the second treatment is any treatment described herein.

In another aspect, the treatment is a Src kinase inhibitor. In some aspects, the Src inhibitor is used to prevent or inhibit metastasis. In some aspects, the Src kinase inhibitor is selected from the group: AZD0530 (saracatinib), Bosulif (bosutinib), ENMD981693, KD020, KX01, Sprycel (dasatinib), Yervoy (ipilimumab), AP23464, AP23485, AP23588, AZD0424, c-Src Kinase Inhibitor KISSEI, CU201, KX2361, SKS927, SRN004, SUNK706, TG100435, TG100948, AP23451, Dasatinib HETERO (dasatinib), Dasatinib VALEANT (dasatinib), Fontrax (dasatinib), Src Kinase Inhibitor KINEX, VX680,(tozasertib lactate), XL228, and SUNK706. In some embodiments, the Src kinase inhibitor is dasatinib. In another aspect, Src kinase inhibitors can be identified through methods known in the art (*See, e.g.,* Sen, B. and Johnson, F.M. Regulation of Src Family Kinases in Human Cancers. 2011. J. Signal Transduction. 2011: 14 pages). In some aspects, the Src kinase inhibitor is used to treat or prevent or inhibit metastasis in a patient that is positive for the SRC-responsive signature (SRS). In some aspects, the patient is SRS+. (*See. e.g.,* Zhang, CH.-F, et al. Latent Bone Metastasis in Breast Cancer Tied to Src-Dependent survival signals. 2009. Cancer Cell. 16: 67-78). In some aspects, the Src kinase inhibitor is used to treat or prevent or inhibit metastasis in a patient with advanced cancer. In some aspects, the Src kinase inhibitor is used in combination with a second treatment. In some aspects, the second treatment is any treatment described herein.

In another aspect, the treatment is a COX-2 inhibitor. In some aspects, the COX-2 inhibitor is used to prevent or inhibit metastasis. In some aspects, the COX-2 inhibitor is selected from the group: ABT963, Acetaminophen ER JOHNSON (acetaminophen), Acular X (ketorolac tromethamine), BAY1019036 (aspirin), BAY987111 (diphenhydramine, naproxen sodium), BAY11902 (piroxicam), BCIBUCH001 (ibuprofen), Capoxigem (apricoxib), CS502, CS670 (pelubiprofen), Diclofenac HPBCD (diclofenac), Diractin (ketoprofen), GW406381, HCT1026 (nitroflurbiprofen), Hyanalgese-D (diclofenac), HydrocoDex (acetaminophen, dextromethorphan, hydrocodone), Ibuprofen Sodium PFIZER (ibuprofen sodium), Ibuprofen with Acetaminophen PFIZER (acetaminophen, ibuprofen), Impracor (ketoprofen), IP880 (diclofenac), IP940 (indomethacin), ISV205 (diclofenac sodium), JNS013 (acetaminophen, tramadol hydrochloride), Ketoprofen TDS (ketoprofen), LTNS001 (naproxen etemesil), Mesalamine SALIX (mesalamine), Mesalamine SOFAR (mesalamine), Mesalazine (mesalamine), ML3000 (licofelone), MRX7EAT (etodolac), Naproxen IROKO (naproxen), NCX4016 (nitroaspirin), NCX701 (nitroacetaminophen), Nuprin SCOLR (ibuprofen), OMS103HP (amitriptyline hydrochloride, ketoprofen, oxymetazoline hydrochloride), Oralease (diclofenac), OxycoDex (dextromethorphan, oxycodone), P54, PercoDex (acetaminophen, dextromethorphan, oxycodone), PL3100 (naproxen, phosphatidyl choline), PSD508, R-Ketoprofen (ketoprofen), Remura (bromfenac sodium), ROX828 (ketorolac tromethamine), RP19583 (ketoprofen lysine), RQ00317076, SDX101 (R-etodolac), TDS943 (diclofenac sodium), TDT070 (ketoprofen), TPR100, TQ1011 (ketoprofen), TT063 (S-flurbiprofen), UR8880 (cimicoxib), V0498TA01A (ibuprofen), VT122 (etodolac, propranolol), XP20B (acetaminophen, dextropropoxyphene), XP21B (diclofenac potassium), XP21L (diclofenac potassium), Zoenasa (acetylcysteine, mesalamine), Acephen, Actifed Plus, Actifed-P, Acular, Acular LS, Acular PF, Acular X, Acuvail, Advil, Advil Allergy Sinus ,Advil Cold and Sinus ,Advil Congestion Relief ,Advil PM, Advil PM Capsule, Air Salonpas, Airtal, Alcohol-Free NyQuil Cold & Flu Relief, Aleve ,Aleve ABDI IBRAHIM ,Aleve-D, Alka-Seltzer ,Alka-Seltzer BAYER, Alka-Seltzer Extra Strength, Alka-Seltzer Lemon-Lime, Alka-Seltzer Original, Alka-Seltzer Plus, Alka-Seltzer plus Cold and Cough, Alka-Seltzer plus Cold and Cough Formula, Alka-Seltzer Plus Day and Night Cold Formula, Alka-Seltzer Plus Day Non-Drowsy Cold Formula, Alka-Seltzer Plus Flu Formula, Alka-Seltzer Plus Night Cold Formula, Alka-Seltzer Plus Sinus Formula, Alka-Seltzer Plus Sparkling Original Cold Formula, Alka-Seltzer PM, Alka-Seltzer Wake-Up Call, Anacin, Anaprox, Anaprox MINERVA, Ansaid, Apitoxin, Apranax, Apranax abdi, Arcoxia, Arthritis Formula Bengay, Arthrotec, Asacol, Asacol HD, Asacol MEDUNA ARZNEIMITTEL, Asacol ORIFARM, Aspirin BAYER, Aspirin Complex, Aspirin Migran, AZD3582, Azulfidine, Baralgan M, BAY1019036, BAY987111, BAY11902, BCIBUCH001, Benadryl Allergy, Benadryl Day and Night, Benylin 4 Flu, Benylin Cold and Flu, Benylin Cold and Flu Day and Night, Benylin Cold and Sinus Day and Night, Benylin Cold and Sinus Plus, Benylin Day and Night Cold and Flu Relief, Benylinl All-In-One, Brexin, Brexin ANGELINI, Bromday, Bufferin, Buscopan Plus, Caldolor, Calmatel, Cambia, Canasa, Capoxigem, Cataflam, Celebrex, Celebrex ORIFARM, Children's Advil Allergy Sinus, Children's Tylenol, Children's Tylenol Cough and Runny Nose, Children's Tylenol plus cold, Children's Tylenol plus Cold and Cough, Children's Tylenol plus cold and stuffy nose, Children's Tylenol plus Flu, Children's Tylenol plus cold & allergy, Children's Tylenol plus Cough & Runny Nose, Children's Tylenol plus Cough & Sore Throat, Children's Tylenol plus multi symptom cold, Clinoril, Codral Cold and Flu, Codral Day and Night Day Tablets, Codral Day and Night Night Tablets, Codral Nightime, Colazal, Combunox, Contac Cold plus Flu, Contac Cold plus Flu Non-Drowsy, Coricidin D, Coricidin HBP Cold and Flu, Coricidin HBP Day and Night Multi-Symptom Cold, Coricidin HBP Maximum Strength Flu, Coricidin HBP Nighttime Multi-Symptom Cold, Coricidin II Extra Strength Cold and Flu, CS502, CS670, Daypro, Daypro Alta, DDS06C, Demazin Cold and Flu, Demazin Cough, Cold and Flu, Demazin day/night Cold and Flu, Demazin PE Cold and Flu, Demazin PE day/night Cold and Flu, Diclofenac HPBCD, Dimetapp Day Relief, Dimetapp Multi-Symptom Cold and Flu, Dimetapp Night Relief, Dimetapp Pain and Fever Relief, Dimetapp PE Sinus Pain, Dimetapp PE Sinus Pain plus Allergy, Dipentum, Diractin, Disprin Cold 'n' Fever, Disprin Extra, Disprin Forte. Disprin Plus, Dristan Cold, Dristan Junior, Drixoral Plus, Duexis, Dynastat, Efferalgan, Efferalgan Plus Vitamin C, Efferalgan Vitamin C, Elixsure IB, Excedrin Back and Body, Excedrin Migraine, Excedrin PM, Excedrin Sinus Headache, Excedrin Tension Headache, Falcol, Fansamac, Feldene, FeverAll, Fiorinal, Fiorinal with Codeine, Flanax, Flector Patch, Flucam, Fortagesic, Gerbin, Giazo, Gladio, Goody's Back and Body Pain, Goody's Cool Orange, Goody's Extra Strength, Goody's PM, Greaseless Bengay, GW406381, HCT1026, He Xing Yi, Hyanalgese-D, HydrocoDex, Ibuprofen Sodium PFIZER, Ibuprofen with, Acetaminophen PFIZER, Icy Hot SANOFI AVENTIS, Impracor, Indocin, Indomethacin APP PHARMA, Indomethacin MYLAN, Infants' Tylenol, IP880, IP940, Iremod, ISV205, JNS013, Jr. Tylenol, Junifen, Junior Strength Advil, Junior Strength Motrin, Ketoprofen TDS, Lemsip Max, Lemsip Max All in One, Lemsip Max All Night, Lemsip Max Cold and Flu, Lialda, Listerine Mouth Wash, Lloyds Cream, Lodine, Lorfit P, Loxonin, LTNS001, Mersyndol, Mesalamine SALIX, Mesalamine SOFAR, Mesalazine, Mesasal GLAXO, Mesasal SANOFI, Mesulid, Metsal Heat Rub, Midol Complete, Midol Extended Relief, Midol Liquid Gels, Midol PM, Midol Teen Formula, Migranin COATED TABLETS, ML3000, Mobic, Mohrus, Motrin, Motrin Cold and Sinus Pain, Motrin PM, Movalis ASPEN, MRX7EAT, Nalfon, Nalfon PEDINOL, Naprelan, Naprosyn, Naprosyn RPG LIFE SCIENCE, Naproxen IROKO, NCX4016, NCX701, NeoProfen LUNDBECK, Nevanac, Nexcede, Niflan, Norgesic MEDICIS, Novalgin, Nuprin SCOLR, Nurofen, Nurofen Cold and Flu, Nurofen Max Strength Migraine, Nurofen Plus, Nuromol, NyQuil with Vitamin C, Ocufen, OMS103HP, Oralease, Orudis ABBOTT JAPAN, Oruvail, Osteluc, OxycoDex, P54, Panadol, Panadol Actifast, Paradine, Paramax, Parfenac, Pedea, Pennsaid, Pentasa, Pentasa ORIFARM, Peon, Percodan, Percodan-Demi, PercoDex, Percogesic, Perfalgan, PL2200, PL3100, Ponstel, Prexige, Prolensa, PSD508, R-Ketoprofen, Rantudil, Relafen, Remura, Robaxisal, Rotec, Rowasa, ROX828, RP19583, RQ00317076, Rubor, Salofalk, Salonpas, Saridon, SDX101, Seltouch, sfRowasa, Shinbaro, Sinumax, Sinutab, Sinutab, sinus, Spalt, Sprix, Strefen, Sudafed Cold and Cough, Sudafed Head Cold and Sinus, Sudafed PE Cold plus Cough, Sudafed PE Pressure plus Pain, Sudafed PE, Severe Cold, Sudafed PE Sinus Day plus Night Relief Day Tablets, Sudafed PE Sinus Day plus Night Relief Night Tablets, Sudafed PE Sinus plus Anti-inflammatory Pain Relief, Sudafed Sinus Advance, Surgam, Synalgos-DC, Synflex, Tavist allergy/sinus/headache, TDS943, TDT070, Theraflu Cold and Sore Throat, Theraflu Daytime Severe Cold and Cough, Theraflu Daytime Warming Relief,Theraflu Warming Relief Caplets Daytime Multi-Symptom Cold, Theraflu Warming Relief Cold and Chest Congestion, Thomapyrin, Thomapyrin C, Thomapyrin Effervescent, Thomapyrin Medium, Tilcotil, Tispol, Tolectin, Toradol, TPR100, TQ1011, Trauma-Salbe, Trauma-Salbe Kwizda, Treo, Treximet, Trovex, TT063, Tylenol, Tylenol Allergy Multi-Symptom, Tylenol Back Pain, Tylenol Cold & Cough Daytime, Tylenol Cold & Cough Nighttime, Tylenol Cold and Sinus Daytime, Tylenol Cold and Sinus Nighttime, Tylenol Cold Head Congestion Severe, Tylenol Cold Multi Symptom Daytime, Tylenol Cold Multi Symptom Nighttime Liquid, Tylenol Cold Multi Symptom Severe, Tylenol Cold Non-Drowsiness Formula, Tylenol Cold Severe Congestion Daytime, Tylenol Complete Cold, Cough and Flu Night time, Tylenol Flu Nighttime, Tylenol Menstrual, Tylenol PM, Tylenol Sinus Congestion & Pain Daytime, Tylenol Sinus Congestion & Pain Nighttime, Tylenol Sinus Congestion & Pain Severe, Tylenol Sinus Severe Congestion Daytime, Tylenol Ultra Relief, Tylenol with Caffeine and Codeine phosphate, Tylenol with Codeine phosphate, Ultra Strength Bengay Cream, Ultracet, UR8880, V0498TA01A, Vicks NyQuil Cold and Flu Relief, Vicoprofen, Vimovo, Voltaren Emulgel, Voltaren GEL, Voltaren NOVARTIS CONSUMER HEALTH GMBH, Voltaren XR, VT122, Xefo, Xefo Rapid, Xefocam, Xibrom, XL3, Xodol, XP20B, XP21B, XP21L, Zipsor, and Zoenasa. In another aspect, COX-2 inhibitors can be identified through methods known in the art (*See, e.g.,* Dannhardt, G. and Kiefer, W. Cyclooxygenase inhibitors- current status and future prospects. 2001. Eur. J. Med. Chem. 36: 109-126). In some aspects, the COX-2 inhibitor is used to treat or prevent or inhibit metastasis in a patient with advanced cancer. In some aspects, the COX-2 inhibitor is used in combination with a second treatment. In some aspects, the second treatment is any treatment described herein. In some aspects, the COX-2 inhibitor is used in combination with a second treatment selected from the group consisting of: Denosumab, Zometa (http://www.us.zometa.com/index.jsp?usertrack.filter_applied=true&NovaId=293537693 4467633633; last accessed 12/2/2012), Carbozantinib or Cabozantinib, Antibody or peptide blocking PTHLH (parathyroid hormone like hormone) or PTHrP (parathyroid hormone related protein) and Everolimus.

In another aspect, the treatment to prevent or inhibit bone metastasis is selected from
- Parathyroid hormone (PTH) and Parathyroid like hormone (PTHLH) inhibitors (including blocking antibodies) or recombinant forms thereof (teriparatide corresponding to the amino acids 7-34 of PTH). This hormone acts by stimulating the osteoclasts and increasing their activity.
- Strontium ranelate: is an alternative oral treatment, and forms part of the group of drugs called "dual action bone agents" (DABAs) because they stimulate the osteoblast proliferation and inhibit the osteoclast proliferation.
- "Estrogen receptor modulators" (SERM) refers to compounds which interfere or inhibit the binding of estrogens to the receptor, regardless of the mechanism. Examples of estrogen receptor modulators include, among others, estrogens progestagen, estradiol, droloxifene, raloxifene, lasofoxifene, TSE-424, tamoxifen, idoxifene, L Y353381, LY117081, toremifene, fluvestrant, 4-[7-(2,2-dimethyl-1-oxopropoxy-4-methyl-2-[4-[2-(1-piperidinyl)ethoxy]phenyl]-2H-1-benzopyran-3-yl]-phenyl-2,2-dimethylpropanoate 4,4'dihydroxybenzophenone-2,4-dinitrophenyl-hydrazone and SH646.
- Calcitonin: directly inhibits the osteoclast activity through the calcitonin receptor. The calcitonin receptors have been identified on the surface of the osteoclasts.
- Bisphosphonates: are a group of medicinal products used for the prevention and the treatment of diseases with bone resorption and reabsorption such as osteoporosis and cancer with bone metastasis, the latter being with or without hypercalcaemia, associated to breast cancer and prostate cancer. Examples of bisphosphonates which can be used in the therapy designed by means of the fifth method of the invention include, although not limited to, nitrogenous bisphosphonates (such as pamidronate, neridronate, olpadronate, alendronate, ibandronate, risedronate, incadronate, zoledronate or zoledronic acid, etc.) and non-nitrogenous bisphosphonates (such as etidronate, clodronate, tiludronate, etc.).
- "Cathepsin K inhibitors" refers to compounds which interfere in the cathepsin K cysteine protease activity. Non-limiting examples of cathepsin K inhibitors include 4-amino-pyrimidine-2-carbonitrile derivatives (described in the International patent application WO 03/020278 under the name of Novartis Pharma GMBH), pyrrolo-pyrimidines described in the publication WO 03/020721 (Novartis Pharma GMBH) and the publication WO 04/000843 (ASTRAZENECA AB) as well as the inhibitors described in the publications PCT WO 00/55126 of Axys Pharmaceuticals, WO 01/49288 of Merck Frosst Canada & Co. and Axys Pharmaceuticals.
- "DKK-1(Dickkopf-1) inhibitor" as used herein refers to any compound which is capable of reducing DKK-1 activity. DKK-1 is a soluble Wnt pathway antagonist expressed predominantly in adult bone and upregulated in myeloma patients with osteolytic lesions. Agents targeting DKK-1 may play a role in preventing osteolytic bone disease in multiple myeloma patients. BHQ880 from Novartis is a first-in-class, fully human, anti-DKK-1 neutralizing antibody. Preclinical studies support the hypothesis that BHQ880 promotes bone formation and thereby inhibits tumor-induced osteolytic disease (Ettenberg S. et al., American Association for Cancer Research Annual Meeting. April 12-16, 2008; San Diego, Calif. Abstract).
- "Dual MET and VEGFR2 inhibitor" as used herein refers to any compound which is a potent dual inhibitor of the MET and VEGF pathways designed to block MET driven tumor escape. MET is expressed not only in tumor cells and endothelial cells, but also in osteoblasts (bone-forming cells) and osteoclasts (bone-removing cells). HGF binds to MET on all of these cell types, giving the MET pathway an important role in multiple autocrine and paracrine loops. Activation of MET in tumor cells appears to be important in the establishment of metastatic bone lesions. At the same time, activation of the MET pathway in osteoblasts and osteoclasts may lead to pathological features of bone metastases, including abnormal bone growth (*i.e.,* blastic lesions) or destruction (*i.e.,* lytic lesion). Thus, targeting the MET pathway may be a viable strategy in preventing the establishment and progression of metastatic bone lesions. Cabozantinib (Exelixis, Inc), formerly known as XL184 (CAS 849217-68-1), is a potent dual inhibitor of the MET and VEGF pathways designed to block MET driven tumor escape. In multiple preclinical studies cabozantinib has been shown to kill tumor cells, reduce metastases, and inhibit angiogenesis (the formation of new blood vessels necessary to support tumor growth). Another suitable dual inhibitors are E7050 (N-[2-Fluoro-4-({2-[4-(4-methylpiperazin-1-yl)piperidin-1-yl] carbonylaminopyridin-4-yl}oxy) phenyl]-N'-(4-fluorophenyl) cyclopropane-1,1-dicarboxamide (2R,3R)-tartrate) (CAS 928037-13-2) or Foretinib (also known as GSK1363089, XL880, CAS 849217-64-7).
- "RANKL inhibitors" as used herein refer to any compound which is capable of reducing the RANK activity. RANKL is found on the surface of the osteoblast membrane of the stroma and T-lymphocyte cells, and these T-lymphocyte cells are the only ones which have demonstrated the capacity for secreting it. Its main function is the activation of the osteoclasts, cells involved in the bone resorption. The RANKL inhibitors can act by blocking the binding of RANKL to its receptor (RANK), blocking the RANK-mediated signaling or reducing the expression of RANKL by blocking the transcription or the translation of RANKL. RANKL antagonists or inhibitors suitable for use in the present invention include, without limitation:
   o a suitable RANK protein which is capable of binding RANKL and which comprises the entire or a fragment of the extracellular domain of a RANK protein. The soluble RANK may comprise the signal peptide and the extracellular domain of the murine or human RANK polypeptides, or alternatively, the mature form of the protein with the signal peptide removed can be used.
   ∘ Osteoprotegerin or a variant thereof with RANKL-binding capacity.
   ∘ RANKL-specific antisense molecules
   ∘ Ribozymes capable of processing the transcribed products of RANKL
   ∘ Specific anti-RANKL antibodies. "Anti-RANKL antibody or antibody directed against RANKL" is understood herein as all that antibody which is capable of binding specifically to the ligand of the activating receptor for the nuclear factor κB (RANKL) inhibiting one or more RANKL functions. The antibodies can be prepared using any of the methods which are known by the person skilled in the art. Thus, the polyclonal antibodies are prepared by means of immunizing an animal with the protein to be inhibited. The monoclonal antibodies are prepared using the method described by Kohler, Milstein et al. (Nature, 1975, 256: 495). Antibodies suitable in the context of the present invention include intact antibodies which comprise a variable antigen binding region and a constant region, fragments "Fab", "F(ab')2" and "Fab'", Fv, scFv, diabodies and bispecific antibodies.
   ∘ Specific anti-RANKL nanobodies. Nanobodies are antibody-derived therapeutic proteins that contain the unique structural and functional properties of naturally-occurring heavy-chain antibodies. The Nanobody technology was originally developed following the discovery that camelidae (camels and llamas) possess fully functional antibodies that lack light chains. The general structure of nanobodies is

      FR1-CDR1-FR2-CDR2-FR3-CDR3-FR4
wherein FR1 to FR4 are the framework regions 1 to 4 CDR1 to CDR3 are the complementarity determining regions 1 to 3. These heavy-chain antibodies contain a single variable domain (VHH) and two constant domains (CH2 and CH3). Importantly, the cloned and isolated VHH domain is a perfectly stable polypeptide harbouring the full antigen-binding capacity of the original heavy-chain antibody. These newly discovered VHH domains with their unique structural and functional properties form the basis of a new generation of therapeutic antibodies which Ablynx has named Nanobodies.

In one embodiment, the RANKL inhibitor is selected from the group consisting of a RANKL specific antibody, a RANKL specific nanobody and osteoprotegerin. In a specific embodiment, the anti-RANKL antibody is a monoclonal antibody. In a yet more specific embodiment, the anti-RANKL antibody is Denosumab (Pageau, Steven C. (2009). mAbs 1 (3): 210-215, CAS number 615258-40-7). Denosumab is a fully human monoclonal antibody which binds to RANKL and prevents its activation (it does not bind to the RANK receptor). Various aspects of Denosumab are covered by U.S. Pat. Nos. 6,740,522; 7,411,050; 7,097,834; 7,364,736. In another embodiment, the RANKL inhibitor an antibody, antibody fragment, or fusion construct that binds the same epitope as Denosumab.

In a preferred embodiment, the anti-RANKL nanobody is any of the nanobodies as described in WO2008142164. In a still more preferred embodiment, the anti-RANKL antibody is the ALX-0141 (Ablynx). ALX-0141 has been designed to inhibit bone loss associated with post-menopausal osteoporosis, rheumatoid arthritis, cancer and certain medications, and to restore the balance of healthy bone metabolism.

In a preferred embodiment, the agent preventing the bone degradation is selected from the group consisting of a bisphosphonate, a RANKL inhibitor, PTH and PTHLH inhibitor or a PRG analog, strontium ranelate, a DKK-1 inhibitor, a dual MET and VEGFR2 inhibitor, an estrogen receptor modulator, calcitonin, and a cathepsin K inhibitor. In a more preferred embodiment the agent preventing the bone degradation is a bisphosphonate. In a yet more preferred embodiment, the bisphosphonate is the zoledronic acid.

In one embodiment, a CCR5 antagonist is administered to prevent or inhibit metastasis of the primary cancer tumor to bone. In one embodiment, the CCR5 antagonist is a large molecule. In another embodiment, the CCR5 antagonist is a small molecule. In some embodiments, the CCR5 antagonist is Maraviroc (Velasco-Veláquez, M. et al. 2012. CCR5 Antagonist Blocks Metastasis of Basal Breast Cancer Cells. Cancer Research. 72:3839-3850.). In some embodiments, the CCR5 antagonist is Vicriviroc. Velasco-Veláquez, M. et al. 2012. CCR5 Antagonist Blocks Metastasis of Basal Breast Cancer Cells. Cancer Research. 72:3839-3850.). In some aspects, the CCR5 antagonist is Aplaviroc (Demarest J.F. et al. 2005. Update on Aplaviroc: An HIV Entry Inhibitor Targeting CCR5. Retrovirology 2(Suppl. 1): S13). In some aspects, the CCR5 antagonist is a spiropiperidine CCR5 antagonist. (Rotstein D.M. et al. 2009. Spiropiperidine CCR5 antagonists. Bioorganic & Medicinal Chemistry Letters. 19 (18): 5401-5406. In some embodiments, the CCR5 antagonist is INCB009471 (Kuritzkes, D.R. 2009. HIV-1 entry inhibitors: an overview. Curr. Opin. HIV AIDS. 4(2): 82-7).

In a preferred embodiment the dual MET and VEGFR2 inhibitor is selected from the group consisting of Cabozantinib, Foretinib and E7050.

In a preferred embodiment the Radium-223 salt is selected from the group consisting of Radium-223 dicloride

Alternatively a combined treatment can be carried out in which more than one agent from those mentioned above are combined to treat and/or prevent the metastasis or said agents can be combined with other supplements, such as calcium or vitamin D or with a hormone treatment.

### Method for predicting early bone metastasis in cancer patients.

In another aspect, the invention relates to an *in vitro* method for the prognosis of the tendency to develop metastasis and/or recurrence in a subject suffering cancer, which comprises determining if a gene of interest in a sample of said subject is amplified, gained or shows polyploidy in a tumor sample of said subject relative to a reference gene copy number, wherein an expression level of said gene above the average value plus one standard deviation is indicative of an increased risk of developing metastasis and/or recurrence.

In a preferred embodiment, the bone metastasis is very early bone metastasis.

In a preferred embodiment, the bone metastasis is osteolytic metastasis.

"Early bone metastasis" as used herein, relates to a bone metastasis that appears before 5 years post-surgery in a patient with cancer.

"Very early bone metastasis" as used herein, relates to a bone metastasis that appears before 3 years post-surgery in a patient with cancer.

The third method of the invention comprises in a first step, quantifying the expression level of a gene of interest in a sample of a subject suffering cancer. In a preferred embodiment, the sample is a tumor tissue sample.

In a preferred embodiment, the third method of the invention comprises quantifying only the expression level of a gene of interest as a single marker, *i.e.,* no other markers are quantified. The method does not involve determining the expression level of any additional marker. The expression level of a gene of interest can be quantified as previously disclosed for the first method of the invention.

In a second step, an expression level of said gene above the average value plus one standard deviation is indicative of an increased risk of early bone metastasis.

"Average level" as used herein relates to a single value of expression level (as a mean, mode, or median) that summarizes or represents the general significance of a set of unequal values. In a preferred embodiment the average level corresponds to the average of expression levels obtained from a representative cohort of cancer tumors. The patient cohort is defined by age that is representative of the individual patient that one is attempting to evaluate.

"Standard deviation" as used herein relates to a measure of the dispersion of a collection of numbers. For example, the standard deviation for the average normal level of the gene of interest is the dispersion of a collection of the gene of interest levels found in cancer samples. The more spread apart the data, the higher the deviation. Standard deviation can be obtained by extracting the square root of the mean of squared deviations of observed values from their mean in a frequency distribution.

Once the expression level of the gene of interest in a sample from a subject with cancer, has been measured and compared with the average level, if the expression level of said gene is above the average plus one standard deviation with respect to the average level, then it can be concluded that said subject has a greater tendency to develop early bone metastasis.

### Method for designing customized therapy in cancer patients with bone metastasis

In another aspect, the invention relates to an *in vitro* method for designing a customized therapy for a subject with cancer (hereinafter fourth method of the invention) which comprises
i) quantifying the copy number or amplification of a gene of interest in a sample of said subject, wherein the gene of interest is selected from the group consisting of: VAT1L, CLEC3A or the gene the sequence of which is provided in the Gene Database with ID645957 or quantifying the expression level, copy number or amplification of the gene of interest WWOX in a bone metastatic sample of said subject and
ii) comparing the expression level, copy number or amplification obtained in step (i) with a reference value,
wherein if the expression level of the gene of interest is increased with respect to said reference value, then said subject is susceptible to receive a therapy aiming to prevent the bone degradation.

In a preferred embodiment, the bone metastasis is osteolytic metastasis.

The fourth method of the invention comprises in a first step, quantifying the expression level of a gene of interest (or translocation or amplification of the gene of interest) in a sample in a subject suffering cancer. In the case of the fourth method of the invention, the sample can be a tissue sample from bone metastasis.

In a preferred embodiment, the fourth method of the invention comprises quantifying only the copy number, amplification or expression level of the gene of interest as a single marker, *i.e.,* the method does not involve determining the expression level of any additional marker.

In a second step the copy number, amplification or expression level of the gene of interest (or translocation or amplification of the gene of interest) obtained in the tumor sample of the subject is compared with the reference value. In a preferred embodiment, the reference value is the expression level of the gene of interest in a control sample. Depending on the type of tumor to be analyzed, the exact nature of the control sample may vary. Thus, in the case involving the fifth method of the invention, then the reference sample is a sample of a subject with cancer who has not suffered metastasis or that corresponds to the median value of the expression level of the gene of interest measured in a tumor tissue collection in biopsy samples of subjects with cancer who have not suffered metastasis.

Once the copy number, amplification or expression level of the gene of interest in the sample is measured and compared with the reference value (*e.g.* the expression level of a gene of interest of a control sample), if the copy number, amplification or expression level of said gene is increased with respect to the reference value, then this is indicative that said subject is susceptible to receive a therapy aiming to avoid or prevent bone degradation.

Illustrative examples of agents used for avoiding and/or preventing bone degradation include, although not limited to:
- Parathyroid hormone (PTH) and Parathyroid like hormone (PTHLH) inhibitors (including blocking antibodies) or recombinant forms thereof (teriparatide corresponding to the amino acids 7-34 of PTH). This hormone acts by stimulating the osteoclasts and increasing their activity.
- Strontium ranelate: is an alternative oral treatment, and forms part of the group of drugs called "dual action bone agents" (DABAs) because they stimulate the osteoblast proliferation and inhibit the osteoclast proliferation.
- "Estrogen receptor modulators" (SERM) refers to compounds which interfere or inhibit the binding of estrogens to the receptor, regardless of the mechanism. Examples of estrogen receptor modulators include, among others, estrogens progestagen, estradiol, droloxifene, raloxifene, lasofoxifene, TSE-424, tamoxifen, idoxifene, L Y353381, LY117081, toremifene, fluvestrant, 4-[7-(2,2-dimethyl-1-oxopropoxy-4-methyl-2-[4-[2-(1-piperidinyl)ethoxy]phenyl]-2H-1-benzopyran-3-yl]-phenyl-2,2-dimethylpropanoate 4,4'dihydroxybenzophenone-2,4-dinitrophenyl-hydrazone and SH646.
- Calcitonin: directly inhibits the osteoclast activity through the calcitonin receptor. The calcitonin receptors have been identified on the surface of the osteoclasts.
- Bisphosphonates: are a group of medicinal products used for the prevention and the treatment of diseases with bone resorption and reabsorption such as osteoporosis and cancer with bone metastasis, the latter being with or without hypercalcaemia, associated to breast cancer and prostate cancer. Examples of bisphosphonates which can be used in the therapy designed by means of the fifth method of the invention include, although not limited to, nitrogenous bisphosphonates (such as pamidronate, neridronate, olpadronate, alendronate, ibandronate, risedronate, incadronate, zoledronate or zoledronic acid, etc.) and non-nitrogenous bisphosphonates (such as etidronate, clodronate, tiludronate, etc.).
- "Cathepsin K inhibitors" refers to compounds which interfere in the cathepsin K cysteine protease activity. Non-limiting examples of cathepsin K inhibitors include 4-amino-pyrimidine-2-carbonitrile derivatives (described in the International patent application WO 03/020278 under the name of Novartis Pharma GMBH), pyrrolo-pyrimidines described in the publication WO 03/020721 (Novartis Pharma GMBH) and the publication WO 04/000843 (ASTRAZENECA AB) as well as the inhibitors described in the publications PCT WO 00/55126 of Axys Pharmaceuticals, WO 01/49288 of Merck Frosst Canada & Co. and Axys Pharmaceuticals.
- "DKK-1(Dickkopf-1) inhibitor" as used herein refers to any compound which is capable of reducing DKK-1 activity. DKK-1 is a soluble Wnt pathway antagonist expressed predominantly in adult bone and upregulated in myeloma patients with osteolytic lesions. Agents targeting DKK-1 may play a role in preventing osteolytic bone disease in multiple myeloma patients. BHQ880 from Novartis is a first-in-class, fully human, anti-DKK-1 neutralizing antibody. Preclinical studies support the hypothesis that BHQ880 promotes bone formation and thereby inhibits tumor-induced osteolytic disease (Ettenberg S. et al., American Association for Cancer Research Annual Meeting. April 12-16, 2008; San Diego, Calif. Abstract).
- "Dual MET and VEGFR2 inhibitor" as used herein refers to any compound which is a potent dual inhibitor of the MET and VEGF pathways designed to block MET driven tumor escape. MET is expressed not only in tumor cells and endothelial cells, but also in osteoblasts (bone-forming cells) and osteoclasts (bone-removing cells). HGF binds to MET on all of these cell types, giving the MET pathway an important role in multiple autocrine and paracrine loops. Activation of MET in tumor cells appears to be important in the establishment of metastatic bone lesions. At the same time, activation of the MET pathway in osteoblasts and osteoclasts may lead to pathological features of bone metastases, including abnormal bone growth (*i.e.,* blastic lesions) or destruction (*i.e.,* lytic lesion). Thus, targeting the MET pathway may be a viable strategy in preventing the establishment and progression of metastatic bone lesions. Cabozantinib (Exelixis, Inc), formerly known as XL184 (CAS 849217-68-1), is a potent dual inhibitor of the MET and VEGF pathways designed to block MET driven tumor escape. In multiple preclinical studies cabozantinib has been shown to kill tumor cells, reduce metastases, and inhibit angiogenesis (the formation of new blood vessels necessary to support tumor growth). Another suitable dual inhibitors are E7050 (N-[2-Fluoro-4-({2-[4-(4-methylpiperazin-1-yl)piperidin-1-yl] carbonylaminopyridin-4-yl} oxy) phenyl]-N'-(4-fluorophenyl) cyclopropane-1,1-dicarboxamide (2R,3R)-tartrate) (CAS 928037-13-2) or Foretinib (also known as GSK1363089, XL880, CAS 849217-64-7).
- "RANKL inhibitors" as used herein refer to any compound which is capable of reducing the RANK activity. RANKL is found on the surface of the osteoblast membrane of the stroma and T-lymphocyte cells, and these T-lymphocyte cells are the only ones which have demonstrated the capacity for secreting it. Its main function is the activation of the osteoclasts, cells involved in the bone resorption. The RANKL inhibitors can act by blocking the binding of RANKL to its receptor (RANK), blocking the RANK-mediated signaling or reducing the expression of RANKL by blocking the transcription or the translation of RANKL. RANKL antagonists or inhibitors suitable for use in the present invention include, without limitation:
   o a suitable RANK protein which is capable of binding RANKL and which comprises the entire or a fragment of the extracellular domain of a RANK protein. The soluble RANK may comprise the signal peptide and the extracellular domain of the murine or human RANK polypeptides, or alternatively, the mature form of the protein with the signal peptide removed can be used.
   ∘ Osteoprotegerin or a variant thereof with RANKL-binding capacity.
   ∘ RANKL-specific antisense molecules
   ∘ Ribozymes capable of processing the transcribed products of RANKL
   ∘ Specific anti-RANKL antibodies. "Anti-RANKL antibody or antibody directed against RANKL" is understood herein as all that antibody which is capable of binding specifically to the ligand of the activating receptor for the nuclear factor κB (RANKL) inhibiting one or more RANKL functions. The antibodies can be prepared using any of the methods which are known by the person skilled in the art. Thus, the polyclonal antibodies are prepared by means of immunizing an animal with the protein to be inhibited. The monoclonal antibodies are prepared using the method described by Kohler, Milstein et al. (Nature, 1975, 256: 495). Antibodies suitable in the context of the present invention include intact antibodies which comprise a variable antigen binding region and a constant region, fragments "Fab", "F(ab')2" and "Fab'", Fv, scFv, diabodies and bispecific antibodies.
   ∘ Specific anti-RANKL nanobodies. Nanobodies are antibody-derived therapeutic proteins that contain the unique structural and functional properties of naturally-occurring heavy-chain antibodies. The Nanobody technology was originally developed following the discovery that camelidae (camels and llamas) possess fully functional antibodies that lack light chains. The general structure of nanobodies is

      FR1-CDR1-FR2-CDR2-FR3-CDR3-FR4
   wherein FR1 to FR4 are the framework regions 1 to 4 CDR1 to CDR3 are the complementarity determining regions 1 to 3. These heavy-chain antibodies contain a single variable domain (VHH) and two constant domains (CH2 and CH3). Importantly, the cloned and isolated VHH domain is a perfectly stable polypeptide harbouring the full antigen-binding capacity of the original heavy-chain antibody. These newly discovered VHH domains with their unique structural and functional properties form the basis of a new generation of therapeutic antibodies which Ablynx has named Nanobodies.

In one embodiment, the RANKL inhibitor is selected from the group consisting of a RANKL specific antibody, a RANKL specific nanobody and osteoprotegerin. In a specific embodiment, the anti-RANKL antibody is a monoclonal antibody. In a yet more specific embodiment, the anti-RANKL antibody is Denosumab (Pageau, Steven C. (2009). mAbs 1 (3): 210-215, CAS number 615258-40-7). Denosumab is a fully human monoclonal antibody which binds to RANKL and prevents its activation (it does not bind to the RANK receptor). Various aspects of Denosumab are covered by U.S. Pat. Nos. 6,740,522; 7,411,050; 7,097,834; 7,364,736. In another embodiment, the RANKL inhibitor an antibody, antibody fragment, or fusion construct that binds the same epitope as Denosumab.

In a preferred embodiment, the anti-RANKL nanobody is any of the nanobodies as described in WO2008142164. In a still more preferred embodiment, the anti-RANKL antibody is the ALX-0141 (Ablynx). ALX-0141 has been designed to inhibit bone loss associated with post-menopausal osteoporosis, rheumatoid arthritis, cancer and certain medications, and to restore the balance of healthy bone metabolism.

In a preferred embodiment, the agent preventing the bone degradation is selected from the group consisting of a bisphosphonate, a RANKL inhibitor, PTH and PTHLH inhibitor or a PRG analog, strontium ranelate, a DKK-1 inhibitor, a dual MET and VEGFR2 inhibitor, an estrogen receptor modulator, calcitonin, and a cathepsin K inhibitor. In a more preferred embodiment the agent preventing the bone degradation is a bisphosphonate. In a yet more preferred embodiment, the bisphosphonate is the zoledronic acid.

In one embodiment, a CCR5 antagonist is administered to prevent or inhibit metastasis of the primary cancer tumor to bone. In one embodiment, the CCR5 antagonist is a large molecule. In another embodiment, the CCR5 antagonist is a small molecule. In some embodiments, the CCR5 antagonist is Maraviroc (Velasco-Veláquez, M. et al. 2012. CCR5 Antagonist Blocks Metastasis of Basal Breast Cancer Cells. Cancer Research. 72:3839-3850.). In some embodiments, the CCR5 antagonist is Vicriviroc. Velasco-Veláquez, M. et al. 2012. CCR5 Antagonist Blocks Metastasis of Basal Breast Cancer Cells. Cancer Research. 72:3839-3850.). In some aspects, the CCR5 antagonist is Aplaviroc (Demarest J.F. et al. 2005. Update on Aplaviroc: An HIV Entry Inhibitor Targeting CCR5. Retrovirology 2(Suppl. 1): S13). In some aspects, the CCR5 antagonist is a spiropiperidine CCR5 antagonist. (Rotstein D.M. et al. 2009. Spiropiperidine CCR5 antagonists. Bioorganic & Medicinal Chemistry Letters. 19 (18): 5401-5406. In some embodiments, the CCR5 antagonist is INCB009471 (Kuritzkes, D.R. 2009. HIV-1 entry inhibitors: an overview. Curr. Opin. HIV AIDS. 4(2): 82-7).

In a preferred embodiment the dual MET and VEGFR2 inhibitor is selected from the group consisting of Cabozantinib, Foretinib and E7050.

In a preferred embodiment the Radium-223 salt is selected from the group consisting of Radium-223 dichloride.

Alternatively a combined treatment can be carried out in which more than one agent from those mentioned above are combined to treat and/or prevent the metastasis or said agents can be combined with other supplements, such as calcium or vitamin D or with a hormone treatment.

### Method of prognosis of metastasis in cancer, based on detecting the amplification of the gene of interest

In another aspect, the invention relates to an *in vitro* method (hereinafter fifth method of the invention) for predicting bone metastasis of a cancer, in a subject suffering said cancer which comprises determining if a gene of interest selected from the group consisting of: VAT1L, CLEC3A, WWOX or the gene the sequence of which is provided in the Gene Database with ID 645957 is amplified in a sample of said subject relative to a reference gene copy number wherein an amplification of the gene of interest with respect to said reference gene copy number is indicative of increased risk of developing bone metastasis.

In some embodiments, the amplification is in region at the 16q23 locus. In some embodiments, the amplification is in any part of the chromosomal region between about Chr. 16 - 79,392,959 bp to about 79,663,806 bp (from centromere to telomere). In some embodiments, the amplification is in the genomic region between about Chr. 16 - 79,392,959 bp to about 79,663,806 bp, but excluding DNA repeating elements. In some embodiments, amplification is measured using a probe specific for that region.

In a particular embodiment, the gene of interest that is amplified is VAT1L, CLEC3A, WWOX, or the gene the sequence of which is provided in the Gene Database with ID 645957. In a particular embodiment, the degree of amplification of the gene of interest can be determined by means of determining the amplification of a chromosome region containing said gene. Preferably, the chromosome region the amplification of which is indicative of the existence of amplification of the gene of interest is the locus 16q22-q24 which includes the VAT1L, CLEC3A, WWOX, and the gene the sequence of which is provided in the Gene Database with ID 645957 genes. The locus 16q22-q24 is located in chromosome 16, in the long arm of said chromosome and in a range between band 22 and band 24. This region corresponds in the NCBI database with the contigs NT_010498.15 and NT_010542.15. In another preferred embodiment, the degree of amplification of the gene can be determined by means of using a probe specific for said gene. In another preferred embodiment, the amplification of the gene of interest is determined by means of using the Vysis LSI IGH/MAF Dual Color dual fusion probe that comprises a probe against 14q32 and 16q23.

The fifth method of the invention comprises, in a first step, determining if a gene of interest is amplified in a sample of a subject. In a preferred embodiment, the sample is a tumor tissue sample. To that end, the amplification of a gene of interest in the tumor sample is compared with respect to a control sample. In a particular embodiment, the gene of interest that is amplified is VAT1L, CLEC3A, WWOX, or the gene the sequence of which is provided in the Gene Database with ID 645957.

In a particular embodiment, the fifth method of the invention for the prognosis of the tendency to develop bone metastasis in a subject with cancer, comprises determining the gene copy number in a sample of said subject and comparing said copy number with the copy number of a control or reference sample, wherein if the copy number is greater with respect to the gene of interest copy number of a control sample, then the subject has a greater tendency to develop bone metastasis.

The control sample refers to a sample of a subject with cancer, who has not suffered metastasis or that correspond to the median value of the gene copy number measured in a tumor tissue collection in biopsy samples of subjects with cancer, respectively, who have not suffered metastasis. Said reference sample is typically obtained by combining equal amounts of samples from a subject population. If the gene copy number is increased with respect to the copy number of said gene in the control sample, then the subject has a greater tendency to develop metastasis.

In a preferred embodiment, the gene of interest is amplified with respect to a reference gene copy number when the gene copy number is higher than the copy number that a reference sample or control sample has. In one example, the gene of interest is said to be "amplified" if the genomic copy number of the gene is increased by at least 2- (*i.e.,* 6 copies), 3- (*i.e.,* 8 copies), 4-, 5-, 6-, 7-, 8-, 9-, 10-, 15-, 20-, 25-, 30-, 35-, 40-, 45-, or 50-fold in a test sample relative to a control sample. In another example, a c-MAF gene is said to be "amplified" if the genomic copy number of the c-MAF gene per cell is at least 3, 4, 5, 6, 7, 8, 9, 10, 11, 12, 13, 14, 15, 16, 17, 18, 19, 20, 21, 22, 23, 24, 25, 26, 27, 28, 29, 30, and the like.

In a particular embodiment, the amplification or the copy number is determined by means of *in situ* hybridization or PCR.

Methods for determining whether the gene of interest or the chromosome region 16q22-q24 is amplified are widely known in the state of the art. Said methods include, without limitation, *in situ* hybridization (ISH) (such as fluorescence *in situ* hybridization (FISH), chromogenic *in situ* hybridization (CISH) or silver *in situ* hybridization (SISH)), genomic comparative hybridization or polymerase chain reaction (such as real time quantitative PCR). For any ISH method, the amplification or the copy number can be determined by counting the number of fluorescent points, colored points or points with silver in the chromosomes or in the nucleus.

The fluorescence *in situ* hybridization (FISH) is a cytogenetic technique which is used for detecting and locating the presence or absence of specific DNA sequences in chromosomes. FISH uses fluorescence probes which only bind to some parts of the chromosome with which they show a high degree of sequence similarity. In a typical FISH method, the DNA probe is labeled with a fluorescent molecule or a hapten, typically in the form of fluor-dUTP, digoxigenin-dUTP, biotin-dUTP or hapten-dUTP which is incorporated in the DNA using enzymatic reactions, such as nick translation or PCR. The sample containing the genetic material (the chromosomes) is placed on glass slides and is denatured by a formamide treatment. The labeled probe is then hybridized with the sample containing the genetic material under suitable conditions which will be determined by the person skilled in the art. After the hybridization, the sample is viewed either directly (in the case of a probe labeled with fluorine) or indirectly (using fluorescently labeled antibodies to detect the hapten).

In the case of CISH, the probe is labeled with digoxigenin, biotin or fluorescein and is hybridized with the sample containing the genetic material in suitable conditions.

Any marking or labeling molecule which can bind to a DNA can be used to label the probes used in the fourth method of the invention, thus allowing the detection of nucleic acid molecules. Examples of labels for the labeling include, although not limited to, radioactive isotopes, enzyme substrates, cofactors, ligands, chemiluminescence agents, fluorophores, haptens, enzymes and combinations thereof. Methods for labeling and guidelines for selecting suitable labels for different purposes can be found, for example, in Sambrook et al. (Molecular Cloning: A Laboratory Manual, Cold Spring Harbor, New York, 1989) and Ausubel et al. (In Current Protocols in Molecular Biology, John Wiley and Sons, New York, 1998).

Once the existence of amplification is determined, either by directly determining the amplification of the gene of interest or by determining the amplification of the locus 16q22-q24, and after being compared with the amplification of said gene in the control sample, if amplification in the gene of interest is detected, it is indicative of the fact that the subject has a greater tendency to develop bone metastasis. In a particular embodiment, the gene of interest that is amplified and indicative of the fact that the subject has a greater tendency to develop bone metastasis is c-MAF, VAT1L, CLEC3A, WWOX, or the gene the sequence of which is provided in the Gene Database with ID 645957.

The determination of the amplification of the gene of interest needs to be correlated with values of a control sample or reference sample that correspond to the level of amplification of the gene of interest measured in a sample of a subject with cancer who has not suffered metastasis or that correspond to the median value of the amplification of the gene of interest measured in a tumor tissue collection in biopsy samples of subjects with cancer who have not suffered metastasis. Said reference sample is typically obtained by combining equal amounts of samples from a subject population. In general, the typical reference samples will be obtained from subjects who are clinically well documented and in whom the absence of metastasis is well characterized. The sample collection from which the reference level is derived will preferably be made up of subjects suffering the same type of cancer as the patient object of the study. Once this median value has been established, the level of amplification of the gene of interest in tumor tissues of patients can be compared with this median value, and thus, if there is amplification, the subject has a greater tendency to develop metastasis.

In a preferred embodiment, the bone metastasis is osteolytic bone metastasis. As used herein, the expression "osteolytic bone metastasis" refers to a type of metastasis in which bone resorption (progressive loss of bone density) is produced in the proximity of the metastasis resulting from the stimulation of the osteoclast activity by the tumor cells and is characterized by severe pain, pathological fractures, hypercalcaemia, spinal cord compression and other syndromes resulting from nerve compression.

### Method of prognosis of metastasis in cancer based on detecting the translocation of the gene of interest

In another aspect, the disclosure relates to an *in vitro* method for predicting the clinical outcome of a patient suffering from cancer, which comprises determining if a gene of interest is translocated in a sample of said subject wherein a translocation of the gene of interest is indicative of a poor clinical outcome. In some embodiments, the gene of interest that is translocated is c-MAF, VAT1L, CLEC3A, WWOX, or the gene the sequence of which is provided in the Gene Database with ID 645957.

In another aspect, the disclosure relates to an *in vitro* method for predicting the clinical outcome of a patient suffering cancer, which comprises determining if the gene of interest is translocated in a sample of said subject wherein a translocation of the gene of interest is indicative of a poor clinical outcome.

In some embodiments, the translocated gene is from the region at the 16q23 locus. In some embodiments, the translocated gene is from any part of the chromosomal region between about Chr. 16 - 79,392,959 bp to about 79,663,806 bp (from centromere to telomere). In some embodiments, the translocated gene is from the genomic region between about Chr. 16 - 79,392,959 bp to about 79,663,806 bp, but excluding DNA repeating elements. In some embodiments, the translocation is measured using a probe specific for that region.

In a particular embodiment, the translocation of the gene of interest can be determined by means of determining the translocation of a chromosome region containing said gene. In one embodiment, the translocation is the t(14,16) translocation. In another embodiment, the chromosome region that is translocated is from locus 16q22-q24. The locus 16q22-q24 is located in chromosome 16, in the long arm of said chromosome and in a range between band 22 and band 24. This region corresponds in the NCBI database with the contigs NT_010498.15 and NT_010542.15. In a preferred embodiment, the c-MAF gene translocates to chromosome 14 at the locus 14q32, resulting in the translocation t(14,16)(q32,q23). This translocation places the gene of interest next to the strong enhancers in the IgH locus, which, in some cases, leads to overexpression of the gene of interest. (Eychène, A., Rocques, N., and Puoponnot, C., A new MAFia in cancer. 2008. Nature Reviews: Cancer. 8: 683-693.) In some embodiments, the gene of interest that is translocated and overexpressed c-MAF.

In a preferred embodiment, the translocation of the gene of interest can be determined by means of using a probe specific for said translocation. In some embodiments, the translocation is measured using a dual color probe. In some embodiments, the translocation is measured using a dual fusion probe. In some embodiments, the translocation is measured using a dual color, dual fusion probe. In some embodiments, the translocation is measured using two separate probes.

In another preferred embodiment, the translocation of the gene of interest is determined using the Vysis LSI IGH/MAF Dual Color dual fusion probe (http://www.abbottmolecular.com/us/products/analyte-specific-reagent/fish/vysis-lsi-igh-maf-dual-color-dual-fusion-probe.html; last accessed 11/5/2012), which comprises a probe against 14q32 and 16q23. In another preferred embodiment, the translocation of the gene of interest is determined using a Kreatech diagnostics MAF/IGH gt(14;16) Fusion probe (http://www.kreatech.com/products/repeat-freetm-poseidontm-fish-probes/hematology/maf-igh-gt1416-fusion-probe.html; last accessed 11/5/2012), an Abnova MAF FISH probe (http://www.abnova.com/products/products_detail.asp?Catalog_id=FA0375; last accessed 11/5/2012), a Cancer Genetics Italia IGH/MAF Two Color, Two Fusion translocation probe (http://www.cancergeneticsitalia.com/dna-fish-probe/ighmaf/; last accessed 11/5/2012), a Creative Bioarray IGH/MAF-t(14;16)(q32;q23) FISH probe (http://www.creative-bioarray.com/products.asp?cid=35&page=10; last accessed 11/5/2012), a Arup Laboratories multiple myeloma panel by FISH (http://www.aruplab.com/files/technical-bulletins/Multiple%20Myeloma%20%28MM%29%20by%20FISH.pdf; last accessed 11/5/2012), an Agilent probe specific to 16q23 or 14q32 (http://www.genomics.agilent.com/ProductSearch.aspx?chr=16&start=79483 700&end=7 9754340; last accessed 11/5/2012; http://www.genomics.agilent.com/ProductSearch.aspx?Pageid=3000&ProductID=637; last accessed 11/5/2012), a Dako probe specific to 16q23 or 14q32 (http://www.dako.com/us/ar42/psg42806000/baseproducts_surefish.htm?setCountry=true &purl=ar42/psg42806000/baseproducts_surefish.htm?undefined&submit=Accept%20cou ntry; last accessed 11/5/2012), a Cytocell IGH/MAF Translocation, Dual Fusion Probe (http://www.zentech.be/uploads/docs/products_info/prenatalogy/cytocell%202012-2013%20catalogue%5B3%5D.pdf; last accessed 11/5/2012), a Metasystems XL IGH / MAF Translocation - Dual Fusion Probe (http://www.metasystems-international.com/index.php?option=com_joodb&view=article&joobase=5&id=12%3Ad-5029-100-og&Itemid=272; last accessed 11/5/2012), a Zeiss FISH Probes XL, 100µl, IGH / MAFB (https://www.micro-shop.zeiss.com/?s=440675675dedc6&1=en&p=uk&f=r&i=5000&o=&h=25&n=1&sd=00 0000-0528-231-uk; last accessed 11/5/2012) or a Genycell Biotech IGH/MAF Dual Fusion Probe (http://www.google.com/url?sa=t&rct=j&q=&esrc=s&source=web&cd=1&ved=0CCQQ FjAA&url=http%3A%2F%2Fwww.genycell.es%2Fimages%2Fproductos%2Fbrochures %2Flphmie6_86.ppt&ei=MhGYUOi3GKWH0QGlt4DoDw&usg=AFQjCNEqQMbT8v QGjJbi9riEf31VgoFTFQ&sig2=V5IS8juEMVHB18Mv2Xx_Ww; last accessed 11/5/2012)

In some embodiments, the label on the probe is a fluorophore. In some embodiments, the fluorophore on the probe is orange. In some embodiments, the fluorophore on the probe is green. In some embodiments, the fluorophore on the probe is red. In some cases, the fluorophore on the probe is yellow. In some embodiments, one probe is labeled with a red fluorophore, and one with a green fluorophore. In some embodiments, one probe is labeled with a green fluorophore and one with an orange fluorophore. In some cases, the fluorophore on the probe is yellow. For instance, if the MAF-specific probe is labeled with a red fluorophore, and the IGH-specific probe is labeled with a green fluorophore, if white is seen it indicates that the signals overlap and translocation has occurred.

In some embodiments, the fluorophore is SpectrumOrange. In some embodiments, the fluorophore is SpectrumGreen. In some embodiments, the fluorophore is DAPI. In some embodiments, the fluorophore is PlatinuniBright405. In some embodiments, the fluorophore is Platinuni*Bright*415. In some embodiments, the fluorophore is Platinum*Bright*495. In some embodiments, the fluorophore is Platinuni*Bright*505. In some embodiments, the fluorophore is Platinum*Bright*550*.* In some embodiments, the fluorophore is Platinum*Bright*547. In some embodiments, the fluorophore is Platinum*Bright*570. In some embodiments, the fluorophore is Platinum*Bright*590. In some embodiments, the fluorophore is Platinum*Bright*647. In some embodiments, the fluorophore is Platinum*Bright*495/550. In some embodiments, the fluorophore is Platinum*Bright*415/495/550. In some embodiments, the fluorophore is DAPI/Platinum*Bright*495/550. In some embodiments, the fluorophore is FITC. In some embodiments, the fluorophore is Texas Red. In some embodiments, the fluorophore is DEAC. In some embodiments, the fluorophore is R6G. In some embodiments, the fluorophore is Cy5. In some embodiments, the fluorophore is FITC, Texas Red and DAPI. In some embodiments, a DAPI counterstain is used to visualize the translocation, amplification or copy number alteration.

One embodiment of the invention comprises a method in which in a first step it is determined if the gene of interest is translocated in a sample of a subject. In a preferred embodiment, the sample is a tumor tissue sample.

In a particular embodiment, a method of the invention for the prognosis of the tendency to develop bone metastasis in a subject with cancer comprises determining the gene of interest copy number in a sample of said subject wherein the gene of interest is translocated and comparing said copy number with the copy number of a control or reference sample, wherein if the gene of interest copy number is greater with respect to the gene of interest copy number of a control sample, then the subject has a greater tendency to develop bone metastasis.

Methods for determining whether the gene of interest or the chromosome region 16q22-q24 is translocated are widely known in the state of the art and include those described previously for the amplification and translocation of c-MAF. Said methods include, without limitation, *in situ* hybridization (ISH) (such as fluorescence *in situ* hybridization (FISH), chromogenic *in situ* hybridization (CISH) or silver *in situ* hybridization (SISH)), genomic comparative hybridization or polymerase chain reaction (such as real time quantitative PCR). For any ISH method, the amplification, the copy number, or the translocation can be determined by counting the number of fluorescent points, colored points or points with silver in the chromosomes or in the nucleus. In other embodiments, the detection of copy number alterations and translocations can be detected through the use of whole genome sequencing, exome sequencing or by the use of any PCR derived technology. For instance, PCR can be performed on samples of genomic DNA to detect translocation. In one embodiment, quantitative PCR is used. In one embodiment, PCR is performed with a primer specific to the c-MAF gene and a primer specific to the IGH promoter region; if a product is produced, translocation has occurred.

In some embodiments, the amplification and copy number of the gene of interest are determined after translocation of the gene of interest is determined. In some embodiments, the probe is used to determine if the cell is polyploid for the gene of interest. In some embodiments, a determination of polyploidy is made by determining if there are more than 2 signals from the gene of interest. In some embodiments, polyploidy is determined by measuring the signal from the probe specific for the gene of interest and comparing it with a centromeric probe or other probe.

### Method of prognosis of clinical outcome in cancer, based on detecting the amplification of a gene of interest

In another aspect, the invention relates to an *in vitro* method (hereinafter seventh method of the invention) for predicting the clinical outcome of a patient suffering cancer, which comprises determining if a gene of interest is amplified in a sample of said subject relative to a reference gene copy number wherein an amplification of the gene of interest with respect to said reference gene copy number is indicative of a poor clinical outcome. In some embodiments, the gene of interest is c-MAF, VAT1L, CLEC3A, WWOX, or the gene the sequence of which is provided in the Gene Database with ID 645957.

The sixth method of the invention comprises, in a first step, determining if the gene of interest is amplified in a sample of a subject. The determination of the amplification of gene of interest is carried out essentially as described in the fifth method of the invention. In a preferred embodiment the sample is a tumor tissue sample. In a preferred embodiment, the amplification of the gene of interest is determined by means of determining the amplification of the locus 16q22-q24. In another preferred embodiment, the amplification of the gene of interest is determined by means of using a gene of interest-specific probe.

In some embodiments, the amplification is in region at the 16q23 locus. In some embodiments, the amplification is in any part of the chromosomal region between about Chr. 16 - 79,392,959 bp to about 79,663,806 bp (from centromere to telomere). In some embodiments, the amplification is in the genomic region between about Chr. 16 - 79,392,959 bp to about 79,663,806 bp, but excluding DNA repeating elements. In some embodiments, amplification is measured using a probe specific for that region.

In a second step, the sixth method of the invention comprises comparing said copy number with the copy number of a control or reference sample, wherein if the gene of interest copy number is greater with respect to the gene of interest copy number of a control sample, then this is indicative of a poor clinical outcome.

In a preferred embodiment, the gene of interest gene is amplified with respect to a reference gene copy number when the gene of interest gene copy number is higher than the copy number that a reference sample or control sample has. In one example, the gene of interest gene is said to be "amplified" if the genomic copy number of the gene of interest gene is increased by at least 2- (*i.e.,* 6 copies), 3- (*i.e.,* 8 copies), 4-, 5-, 6-, 7-, 8-, 9-, 10-, 15-, 20-, 25-, 30-, 35-, 40-, 45-, or 50-fold in a test sample relative to a control sample. In another example, a c-MAF gene is said to be "amplified" if the genomic copy number of the c-MAF gene per cell is at least 3, 4, 5, 6, 7, 8, 9, 10, 11, 12, 13, 14, 15, 16, 17, 18, 19, 20, 21, 22, 23, 24, 25, 26, 27, 28, 29, 30, and the like.

In another embodiment, the reference gene copy number is the gene copy number in a sample of cancer, from a subject who has not suffered bone metastasis.

In another embodiment, the amplification is determined by means of *in situ* hybridization or PCR.

### Methods for treating bone metastasis from cancer, using c-MAF, VAT1L, CLEC3A, WWOX, or the gene the sequence of which is provided in the Gene Database with ID 645957 inhibitory agents

It is also disclosed the use of a c-MAF, VAT1L, CLEC3A, WWOX, or the gene the sequence of which is provided in the Gene Database with ID 645957 inhibitory agent (hereinafter, inhibitory agent of the invention) for use in the treatment or prevention of bone metastasis cancer after the use of a probe specific for c-MAF, VAT1L, CLEC3A, WWOX, or the gene the sequence of which is provided in the Gene Database with ID 645957 as a diagnostic agent.

It is also disclosed the use of a c-MAF, VAT1L, CLEC3A, WWOX, or the gene the sequence of which is provided in the Gene Database with ID 645957 inhibitory agent for the manufacture of a medicament for the treatment or prevention of bone metastasis from cancer.

Additionally it is described a method for the treatment or prevention of the bone metastasis from cancer, in a subject in need thereof comprising the administration to said subject of a c-MAF, VAT1L, CLEC3A, WWOX, or the gene the sequence of which is provided in the Gene Database with ID 645957 inhibitory agent.

It is also described a method for preventing or reducing the risk of bone metastasis in a subject suffering from cancer, said method comprising administering to said subject an agent that prevents or reduces bone metastasis, wherein said agent is administered in accordance with a treatment regimen determined from quantifying the expression level of c-MAF, VAT1L, CLEC3A, WWOX, or the gene the sequence of which is provided in the Gene Database with ID 645957 in said subject.

By way of non-limiting illustration, c-MAF inhibitory agents suitable for use in the present invention include antisense oligonucleotides, interference RNAs (siRNAs), catalytic RNAs, specific ribozymes, inhibitory antibodies or nanobodies, a dominant negative c-MAF variant or a compound from Table 1 or 2.

### Antisense oligonucleotides

Also disclosed is the use of isolated "antisense" nucleic acids to inhibit expression, for example, for inhibiting transcription and/or translation of a nucleic acid which encodes c-MAF, VAT1L, CLEC3A, WWOX, or the gene the sequence of which is provided in the Gene Database with ID 645957 the activity of which is to be inhibited. The antisense nucleic acids can be bound to the potential target of the drug by means of conventional base complementarity or, for example, in the case of binding to double stranded DNA through specific interaction in the large groove of the double helix. Generally, these methods refer to a range of techniques generally used in the art and they include any method which is based on the specific binding to oligonucleotide sequences.

An antisense construct of the present disclosure can be distributed, for example, as an expression plasmid which, when it is transcribed in a cell, produces RNA complementary to at least one unique part of the cellular mRNA encoding c-MAF, VAT1L, CLEC3A, WWOX, or the gene the sequence of which is provided in the Gene Database with ID 645957. Alternatively, the antisense construct is a oligonucleotide probe generated *ex vivo* which, when introduced into the cell, produces inhibition of gene expression hybridizing with the mRNA and/or gene sequences of a target nucleic acid. Such oligonucleotide probes are preferably modified oligonucleotides which are resistant to endogenous nucleases, for example, exonucleases and/or endonucleases and are therefore stable *in vivo.* Examples of nucleic acids molecules for use thereof as antisense oligonucleotides are DNA analogs of phosphoramidate, phosphothionate and methylphosphonate (see also US patent Nos. 5,176,996; 5,264,564; and 5,256,775). Additionally, the general approximations for constructing oligomers useful in the antisense therapy have been reviewed, for example, in Van der Krol et al., BioTechniques 6: 958-976, 1988; and Stein et al., Cancer Res 48: 2659-2668, 1988.

With respect to the antisense oligonucleotide, the oligodeoxyribonucleotide regions derived from the starting site of the translation, for example, between -10 and +10 of the target gene are preferred. The antisense approximations involve the oligonucleotide design (either DNA or RNA) that are complementary to the mRNA encoding the target polypeptide. The antisense oligonucleotide will be bound to the transcribed mRNA and translation will be prevented.

The oligonucleotides which are complementary to the 5' end of the mRNA, for example the non-translated 5' sequence up to and including the start codon AUG must function in the most efficient manner to inhibit translation. Nevertheless, it has been shown recently that the sequences complementary to the non-translated 3' sequences of the mRNA are also efficient for inhibiting mRNA translation (Wagner, Nature 372: 333, 1994). Therefore, complementary oligonucleotides could be used at the non-translated 5' or 3' regions, non-coding regions of a gene in an antisense approximation to inhibit the translation of that mRNA. The oligonucleotides complementary to the non-translated 5' region of the mRNA must include the complement of the start codon AUG. The oligonucleotides complementary to the coding region of the mRNA are less efficient translation inhibitors but they could also be used according to the disclosure. If they are designed to hybridize with the 5' region, 3' region or the coding region of the mRNA, the antisense nucleic acids must have at least six nucleotides long and preferably have less than approximately 100 and more preferably less than approximately 50, 25, 17 or 10 nucleotides long.

Preferably, *in vitro* studies are performed first to quantify the capacity of the antisense oligonucleotides for inhibiting gene expression. Preferably these studies use controls which distinguish between antisense gene inhibition and nonspecific biological effects of the oligonucleotides. Also preferably these studies compared the levels of target RNA or protein with that of an internal control of RNA or protein. The results obtained using the antisense oligonucleotides can be compared with those obtained using a control oligonucleotide. Preferably the control oligonucleotide is approximately of the same length as the oligonucleotide to be assayed and the oligonucleotide sequence does not differ from the antisense sequence more than it is deemed necessary to prevent the specific hybridization to the target sequence.

The antisense oligonucleotide can be a single or double stranded DNA or RNA or chimeric mixtures or derivatives or modified versions thereof. The oligonucleotide can be modified in the base group, the sugar group or the phosphate backbone, for example, to improve the stability of the molecule, its hybridization capacity etc. The oligonucleotide may include other bound groups, such as peptides (for example, for directing them to the receptors of the host cells) or agents for facilitating transport through the cell membrane (see, for example, Letsinger et al., Proc. Natl. Acad. Sci. U.S.A. 86: 6553-6556, 1989; Lemaitre et al., Proc. Natl. Acad. Sci. 84: 648-652, 1987; PCT Publication No. WO 88/09810) or the blood-brain barrier (see, for example, PCT Publication No. WO 89/10134), intercalating agents (see, for example, Zon, Pharm. Res. 5: 539-549, 1988). For this purpose, the oligonucleotide can be conjugated to another molecule, for example, a peptide, a transporting agent, hybridization triggered cleaving agent, etc.

The antisense oligonucleotides may comprise at least one group of modified base. The antisense oligonucleotide may also comprise at least a modified sugar group selected from the group including but not limited to arabinose, 2-fluoroarabinose, xylulose, and hexose. The antisense oligonucleotide may also contain a backbone similar to a neutral peptide. Such molecules are known as peptide nucleic acid (PNA) oligomers and are described, for example, in Perry-O'Keefe et al., Proc. Natl. Acad. Sci. U.S.A. 93: 14670, 1996, and in Eglom et al., Nature 365: 566, 1993.

In yet another embodiment, the antisense oligonucleotide comprises at least one modified phosphate backbone. In yet another embodiment, the antisense oligonucleotide is an alpha-anomeric oligonucleotide.

While antisense oligonucleotides complementary to the coding region of the target mRNA sequence can be used, those complementary to the transcribed non translated region can also be used.

In some cases, it may be difficult to reach the sufficient intracellular concentrations of the antisense to suppress the endogenous mRNA translation. Therefore, a preferred approximation uses a recombinant DNA construct in which the antisense oligonucleotide is placed under the control of a strong pol III or pol II promoter.

Alternatively, the target gene expression can be reduced by directing deoxyribonucleotide sequences complementary to the gene regulating region (*i.e.,* the promoter and/or enhancers) to form triple helix structures preventing gene transcription in the target cells in the body (see in general, Helene, Anticancer Drug Des. 6(6): 569-84, 1991). In certain embodiments, the antisense oligonucleotides are antisense morpholines.

### siRNA

Small interfering RNA or siRNA are agents which are capable of inhibiting the expression of a target gene by means of RNA interference. A siRNA can be chemically synthesized, can be obtained by means of *in vitro* transcription or can be synthesized *in vivo* in the target cell. Typically, the siRNA consist of a double stranded RNA between 15 and 40 nucleotide long and may contain a 3' and/or 5' protruding region of 1 to 6 nucleotides. The length of the protruding region is independent of the total length of the siRNA molecule. The siRNA acts by means of degrading or silencing the target messenger after transcription.

The siRNA of the disclosure are substantially homologous to the mRNA of the c-MAF encoding gene or to the gene sequence which encodes said protein. "Substantially homologous" is understood as having a sequence which is sufficiently complementary or similar to the target mRNA such that the siRNA is capable of degrading the latter through RNA interference. The siRNA suitable for causing said interference include siRNA formed by RNA, as well as siRNA containing different chemical modifications such as:
- siRNA in which the bonds between the nucleotides are different than those that appear in nature, such as phosphorothionate bonds.
- Conjugates of the RNA strand with a functional reagent, such as a fluorophore.
- Modifications of the ends of the RNA strands, particularly of the 3' end by means of the modification with different hydroxyl functional groups in 2' position.
- Nucleotides with modified sugars such as O-alkylated residues on 2' position like 2'-O-methylribose or 2'-O-fluororibose.
- Nucleotides with modified bases such as halogenated bases (for example 5-bromouracil and 5-iodouracil), alkylated bases (for example 7-methylguanosine).

The siRNA can be used as is, *i.e.,* in the form of a double stranded RNA with the aforementioned characteristics. Alternatively, the use of vectors containing the sense and antisense strand sequence of the siRNA is possible under the control of suitable promoters for the expression thereof in the cell of interest.

Vectors suitable for expressing siRNA are those in which the two DNA regions encoding the two strands of siRNA are arranged in tandem in one and the same DNA strand separated by a spacer region which, upon transcription, forms a loop and wherein a single promoter directs the transcription of the DNA molecule giving rise to shRNA.

Alternatively, the use of vectors in which each of the strands forming the siRNA is formed from the transcription of a different transcriptional unit is possible. These vectors are in turn divided into divergent and convergent transcription vectors. In divergent transcription vectors, the transcriptional units encoding each of the DNA strands forming the siRNA are located in tandem in a vector such that the transcription of each DNA strand depends on its own promoter which may be the same or different (Wang, J. et al., 2003, Proc. Natl. Acad. Sci. USA., 100:5103-5106 and Lee, N.S., et al., 2002, Nat. Biotechnol., 20:500-505). In convergent transcription vectors, the DNA regions giving rise to the siRNA form the sense and antisense strands of a DNA region which are flanked by two reverse promoters. After the transcription of the sense and antisense RNA strands, the latter will form the hybrid for forming a functional siRNA. Vectors with reverse promoter systems in which 2 U6 promoters (Tran, N. et al., 2003, BMC Biotechnol., 3:21), a mouse U6 promoter and a human HI promoter (Zheng, L., et al., 2004, Proc. Natl. Acad. Sci. USA., 135-140 and WO 2005026322) and a human U6 promoter and a mouse HI promoter (Kaykas, A. and Moon, R., 2004, BMC Cell Biol., 5:16) are used have been described.

Promoters suitable for use thereof in the expression of siRNA from convergent or divergent expression vectors include any promoter or pair of promoters compatible with the cells in which the siRNA is to be expressed. Thus, promoters suitable for the present disclosure include but are not necessarily limited to constitutive promoters such as those derived from the genomes of eukaryotic viruses such as the polyoma virus, adenovirus, SV40, CMV, avian sarcoma virus, hepatitis B virus, the metallothionein gene promoter, the thymidine kinase gene promoter of the herpes simplex virus, retrovirus LTR regions, the immunoglobulin gene promoter, the actin gene promoter, the EF-1alpha gene promoter as well as inducible promoters in which the protein expression depends on the addition of a molecule or an exogenous signal such as the tetracycline system, the NFkappaB/UV light system, the Cre/Lox system and the heat shock gene promoter, the regulatable RNA polymerase II promoters described in WO/2006/135436 as well as specific tissue promoters (for example, the PSA promoter described in WO2006012221). In a preferred embodiment, the promoters are RNA polymerase III promoters which act constitutively. The RNA polymerase III promoters are found in a limited number of genes such as 5S RNA, tRNA, 7SL RNA and U6 snRNA. Unlike other RNA polymerase III promoters, type III promoters do not require any intragenic sequence but rather need sequences in 5' direction comprising a TATA box in positions -34 and -24, a proximal sequence element or PSE between -66 and -47 and, in some cases, a distal sequence element or DSE between positions -265 and -149. In a preferred embodiment, the type III RNA polymerase III promoters are the human or murine H1 and U6 gene promoters. In a yet more preferred embodiment, the promoters are 2 human or murine U6 promoters, a mouse U6 promoter and a human HI promoter or a human U6 promoter and a mouse HI promoter. In the context of the present disclosure, the ER alpha gene promoters or cyclin D1 gene promoters are especially suitable and therefore they are especially preferred to specifically express the genes of interest in cancer tumors.

The siRNA can be generated intracellularly from the so called shRNA (short hairpin RNA) characterized in that the antiparallel strands forming the siRNA are connected by a loop or hairpin region. The shRNAs can be encoded by plasmids or viruses, particularly retroviruses, and are under the control of a promoter. Promoters suitable for expressing shRNA are those indicated in the paragraph above for expressing siRNA.

Vectors suitable for expressing siRNA and shRNA include prokaryotic expression vectors such as pUC18, pUC19, Bluescript and the derivatives thereof, mp18, mp19, pBR322, pMB9, CoIEl, pCR1, RP4, phages and shuttle vectors such as pSA3 and pAT28, yeast expression vectors such as 2-micron plasmid type vectors, integration plasmids, YEP vectors, centromeric plasmids and the like, insect cell expression vectors such as pAC series vectors and pVL series vectors, plant expression vectors such as pIBI, pEarleyGate, pAVA, pCAMBIA, pGSA, pGWB, pMDC, pMY, pORE series vectors and the like and viral vector-based (adenovirus, viruses associated with adenoviruses as well as retroviruses and particularly lentiviruses) higher eukaryotic cell expression vectors or non-viral vectors such as pcDNA3, pHCMV/Zeo, pCR3.1, pEF1/His, pIND/GS, pRc/HCMV2, pSV40/Zeo2, pTRACER-HCMV, pUB6/V5-His, pVAX1, pZeoSV2, pCI, pSVL and pKSV-10, pBPV-1, pML2d and pTDT1. In a preferred embodiment, the vectors are lentiviral vectors.

The siRNA and shRNA of the disclosure can be obtained using a series of techniques known by the person skilled in the art. The region of the nucleotide sequence taken as a basis for designing the siRNA is not limiting and it may contain a region of the coding sequence (between the start codon and the end codon) or it may alternatively contain sequences of the non-translated 5' or 3' region preferably between 25 and 50 nucleotides long and in any position in 3' direction position with respect to the start codon. One way of designing an siRNA involves the identification of the AA(N19)TT motifs wherein N can be any nucleotide in the c-MAF gene sequence, and the selection of those having a high G/C content. If said motif is not found, it is possible to identify the NA(N21) motif wherein N can be any nucleotide.

c-MAF specific siRNAs include the siRNA described in WO2005046731, one of the strands of which is ACGGCUCGAGCAGCGACAA (SEQ ID NO: 6). Other c-MAF specific siRNA sequences include, but are not limited to, CUUACCAGUGUGUUCACAA (SEQ ID NO: 7), UGGAAGACUACUACUGGAUG (SEQ ID NO: 8), AUUUGCAGUCAUGGAGAACC (SEQ ID NO: 9), CAAGGAGAAAUACGAGAAGU (SEQ ID NO: 10), ACAAGGAGAAAUACGAGAAG (SEQ ID NO: 11) and ACCUGGAAGACUACUACUGG (SEQ ID NO: 12).

### DNA Enzymes

On the other hand, the disclosure also contemplates the use of DNA enzymes to inhibit the expression of the c-MAF gene of the disclosure. DNA enzymes incorporate some of the mechanistic features of both antisense and ribozyme technologies. DNA enzymes are designed such that they recognize a particular target nucleic acid sequence similar to the antisense oligonucleotide, nevertheless like the ribozyme they are catalytic and specifically cleave the target nucleic acid.

### Ribozymes

Ribozyme molecules designed for catalytically cleaving transcription products of a target mRNA to prevent the translation of the mRNA which encodes c-MAF the activity of which is to be inhibited, can also be used. Ribozymes are enzymatic RNA molecules capable of catalyzing specific RNA cleaving (For a review, see, Rossi, Current Biology 4: 469-471, 1994). The mechanism of ribozyme action involves a specific hybridization of a ribozyme molecule sequence to a complementary target RNA followed by an endonucleolytic cleavage event. The composition of the ribozyme molecules preferably includes one or more sequences complementary to the target mRNA and the well-known sequence responsible for cleaving the mRNA or a functionally equivalent sequence (see, for example, US patent No. 5093246).

The ribozymes used in the present disclosure include hammer-head ribozymes, endoribonuclease RNA (hereinafter "Cech type ribozymes") (Zaug et al., Science 224:574-578, 1984.

The ribozymes can be formed by modified oligonucleotides (for example to improve the stability, targeting, etc.) and they should be distributed to cells expressing the target gene *in vivo.* A preferred distribution method involves using a DNA construct which "encodes" the ribozyme under the control of a strong constitutive pol III or pol II promoter such that the transfected cells will produce sufficient amounts of the ribozyme to destroy the endogenous target messengers and to inhibit translation. Since the ribozymes are catalytic, unlike other antisense molecules, a low intracellular concentration is required for its efficiency.

### Inhibitory antibodies

In the context of the present disclosure, "inhibitory antibody" is understood as any antibody capable of binding specifically to the protein expressed by the gene of interest (such as MAF, VAT1L, CLEC3A, WWOX, the gene the sequence of which is provided in the Gene Database with ID 645957) and inhibiting one or more of the functions of said protein, preferably those related to transcription. The antibodies can be prepared using any of the methods which are known by the person skilled in the art, some of which have been mentioned above. Thus, the polyclonal antibodies are prepared by means of immunizing an animal with the protein to be inhibited. The monoclonal antibodies are prepared using the method described by Kohler, Milstein et al. (Nature, 1975, 256: 495). In the context of the present disclosure, suitable antibodies include intact antibodies comprising a variable antigen binding region and a constant region, "Fab", "F(ab')2" and "Fab'", Fv, scFv fragments, diabodies, bispecific antibodies, alphabodies, cyclopeptides and stapled peptides. Once antibodies with gene of interest protein binding capacity are identified, those capable of inhibiting the activity of this protein will be selected using an inhibitory agent identification assay.

### Inhibitory peptides

As used herein, the term "inhibitory peptide" refers to those peptides capable of binding to the protein expressed by the gene of interest and inhibiting its activity as has been explained above.

### Negative c-MAF dominants

Since the proteins from the MAF family are capable of homodimerizing and heterodimerizing with other members of the AP-1 family such as Fos and Jun, one way of inhibiting c-MAF activity is by means of using negative dominants capable of dimerizing with c-MAF but lacking the capacity for activating transcription. Thus, the negative c-MAF dominants can be any of the small maf proteins existing in the cell and lacking two-thirds of the amino terminal end containing the transactivation domain (for example, mafK, mafF, mafg and pi 8) (Fujiwara et al (1993) Oncogene 8, 2371-2380; Igarashi et al. (1995) J. Biol.Chem. 270, 7615-7624; Andrews et al. (1993) Proc. Natl. Acad. Sci. USA 90, 11488-11492; Kataoka et al. (1995) Mol. Cell. Biol. 15, 2180-2190) (Kataoka et al. (1996) Oncogene 12, 53-62).

Alternatively, the negative c-MAF dominants include c-MAF variants which maintain the capacity for dimerizing with other proteins but lack the capacity for activating transcription. These variants are, for example, those lacking the c-MAF transactivation domain located at the N-terminal end of the protein. Thus, negative c-MAF dominant variants include in an illustrative manner the variants in which at least amino acids 1 to 122, at least amino acids 1-187 or at least amino acids 1 to 257 (by considering the numbering of human c-MAF as described in US6274338) have been removed.

The disclosure contemplates the use of both the negative c-MAF dominant variants and of polynucleotides encoding c-MAF under the operative control of a promoter suitable for expression in target cell. The promoters that can be used for regulating the polynucleotide transcription of the disclosure can be constitutive promoters, *i.e.,* promoters directing the transcription at a basal level, or inducible promoters in which the transcriptional activity requires an external signal. Constitutive promoters suitable for regulating transcription are, among others, the CMV promoter, the SV40 promoter, the DHFR promoter, the mouse mammary tumor virus (MMTV) promoter, the 1a elongation factor (EFla) promoter, the albumin promoter, the ApoA1 promoter, the keratin promoter, the CD3 promoter, the immunoglobulin heavy or light chain promoter, the neurofilament promoter, the neuron specific enolase promoter, the L7 promoter, the CD2 promoter, the myosin light chain kinase promoter, the HOX gene promoter, the thymidine kinase promoter, the RNA polymerase II promoter, the MyoD gene promoter, the phosphoglyceratekinase (PGK) gene promoter, the low density lipoprotein (LDL) promoter, the actin gene promoter. In a preferred embodiment, the promoter regulating the expression of the transactivator is the PGK gene promoter. In a preferred embodiment, the promoter regulating the polynucleotide transcription of the disclosure is the RNA polymerase promoter of the T7 phage.

Preferably, the inducible promoters that can be used in the context of the present disclosure are those responding to an inducer agent showing zero or negligible basal expression in the absence of an inducer agent and are capable of promoting the activation of gene located in the 3' position. Depending on the type of inducer agent, the inducible promoters are classified as Tet on/off promoters (Gossen, M. and H. Bujard (1992) Proc. Natl. Acad. Sci. USA, 89:5547-5551; Gossen, M. et al., 1995, Science 268:1766-1769; Rossi, F.M.V. and H.M. Blau, 1998, Curr. Opin. Biotechnol. 9:451-456); Pip on/off promoters (US 6287813); antiprogestin-dependent promoters (US 2004132086), ecdysone-dependent promoters (Christopherson et al., 1992, Proc. Natl. Acad. Sci. USA, 89:6314-6318; No et al., 1996, Proc. Natl. Acad. Sci. USA, 93:3346-3351, Suhr et al., 1998, Proc. Natl. Acad. Sci. USA, 95:7999-8004 and WO9738117), a metallothionein-dependent promoter (WO8604920) and rapamycin-dependent promoters (Rivera et al., 1996, Nat. Med. 2:1028-32).

Vectors suitable for expressing the polynucleotide encoding the negative c-MAF dominant variant include vectors derived from prokaryotic expression vectors such as pUC18, pUC19, Bluescript and derivatives thereof, mp18, mp19, pBR322, pMB9, ColEl, pCR1, RP4, phages and shuttle vectors such as pSA3 and pAT28, yeast expression vectors such as 2-micron type plasmid vectors, integration plasmids, YEP vectors, centromeric plasmids and the like, insect cell expression vectors such as pAC series vectors and pVL series vectors, plant expression vectors such as pIBI, pEarleyGate, pAVA, pCAMBIA, pGSA, pGWB, pMDC, pMY, pORE series vectors and the like and viral vector-based (adenoviruses, viruses associated with adenoviruses as well as retroviruses and particularly lentiviruses) higher eukaryotic cell expression vectors OR non-viral vectors such as pSilencer 4.1-CMV (Ambion), pcDNA3, pcDNA3.1/hyg pHCMV/Zeo, pCR3.1, pEF1/His, pIND/GS, pRc/HCMV2, pSV40/Zeo2, pTRACER-HCMV, pUB6/V5-His, pVAX1, pZeoSV2, pCI, pSVL and pKSV-10, pBPV-1, pML2d and pTDT1.

### Small molecules

Other c-MAF inhibitory compounds suitable for use in the present disclosure include:

**Table 1: Small molecules with c-MAF inhibiting capacity**

| | |
|---|---|
| I | Endiandric acid H derivatives such as those described in WO2004014888 corresponding to the general formula |
| | 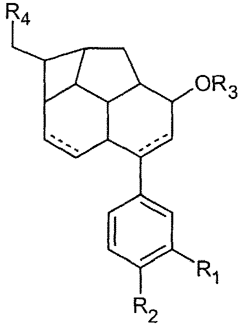 |
| | wherein |
| | R₁ and R₂ are, independently of one another, |
| | 1.0 H or |
| | 2.0 a O-C₁-C₆-alkyl, -O-C₂-C₆-alkenyl, -O-C₂-C₆-alkynyl or -O-C₆-C₁₀-aryl group, in which alkyl, alkenyl and alkynyl are straight-chain or branched, and in which the alkyl, alkenyl and alkynyl groups are mono- or disubstituted with: |
| | 2.1 -OH, |
| | 2.2 =O, |
| | 2.3 -O- C₁-C6-alkyl, in which alkyl is straight-chain or branched, |
| | 2.4-O-C₂-C₆-alkenyl, in which alkenyl is straight-chain or branched, |
| | 2.5 C₆-C₁₀-aryl, |
| | 2.6-NH-C₁-C₆-alkyl, in which alkyl is straight-chain or branched, |
| | 2.7 -NH-C₂-C₆-alkenyl, in which alkenyl is straight-chain or branched, |
| | 2.8 -NH₂ or |
| | 2.9 halogen, |
| | and in which the aryl group, is optionally mono- or disubstituted with the substituent 2.1 or 2.3 to 2.9, |
| | in which the substituents 2.3, 2.4, 2.6 and 2.7 may be further substituted with - CN, -amide or -oxime functions, and 2.5 may be further substituted with -CN or amide functions, or R₁ and R₂ together form a ring, wherein R₁ and R₂ mean a - O-[(C₁-C₆)-alkylene]-O- group, |
| | R₃ is |
| | 1.0 H or |
| | 2.0 a -O-C₁-C₆-alkyl, -O-C₂-C₆-alkenyl, -O-C₂-C₆-alkynyl or -O-C₆-C₁₀-aryl |
| | group, in which alkyl, alkenyl and alkynyl are straight-chain or branched, and in which the alkyl, alkenyl and alkynyl groups are mono- or disubstituted with: |
| | 2.1 -OH, |
| | 2.2 =O, |
| | 2.3 -O-C₁-C₆-alkyl, in which alkyl is straight-chain or branched, |
| | 2.4 -O-C₂-C₆-alkenyl, in which alkenyl is straight-chain or branched, |
| | 2.5 -C₆-C₁₀-aryl, |
| | 2.6 -NH-C₁-C₆-alkyl, in which alkyl is straight-chain or branched, |
| | 2.7 -NH-C₂-C₆-alkenyl, in which alkenyl is straight-chain or branched, |
| | 2.8 -NH₂ or |
| | 2.9 halogen, |
| | and in which the aryl group, is optionally mono- or disubstituted with the substituent 2.1 or 2.3 to 2.9, |
| | in which the substituents 2.3, 2.4, 2.6 and 2.7 may be further substituted with - CN, -amide or -oxime functions, and 2.5 may be further substituted with -CN or amide functions |
| | R₄ is CO₂R₃, CO₂NHR₃, CHO, CH₂OR₃, CH₂OSi(R₃)₃, CH₂Br, CH₂CN, in which R₃ is as defined above, |
| | and, in particular, the compounds |
| | 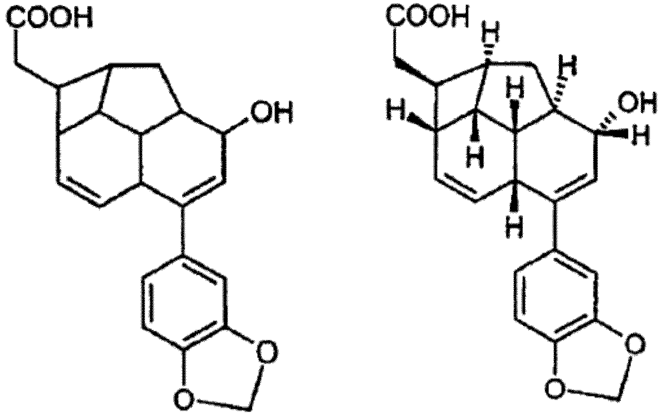 |
| II | 8-hydroxyquinoline derivatives such as those described in WO2009146546 of general formula |
| | 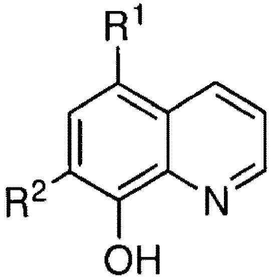 |
| | wherein |
| | R₁ is selected from the group consisting of NO₂, NH₂, NH(C₁-C₆-alkyl) and N(C₁-C₆-alkyl)(C₁-C₆-alkyl); |
| | R₂ is selected from H, halogen, C₁-C₆ alkyl, and fluoro-substituted C₁-C₆ alkyl, |
| | or |
| | R₁ is Cl and R₂ is Br or H, |
| | and, preferably, the compounds |
| | 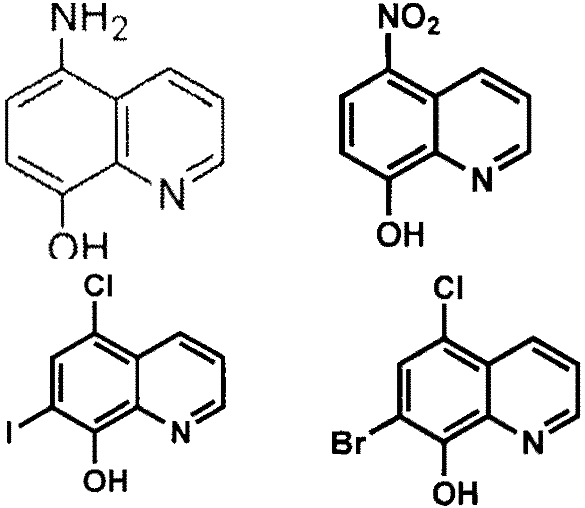 |
| | 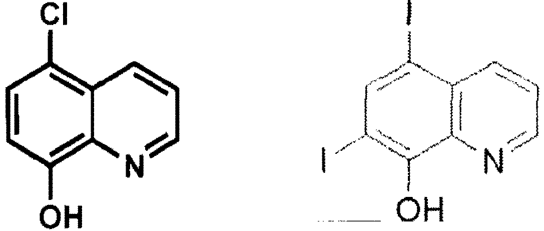 |
| III | Clioquinol (5-chloro-7-iodoquinolin-8-ol) as described in WO09049410 |
| IV | Compounds such as those described in WO08098351 of general formula |
| | 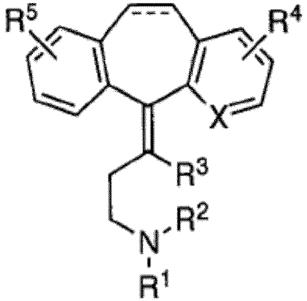 |
| | wherein |
| | ==-:-:-: is a single or double bond, |
| | R¹ is selected from the group consisting of H, C₁-C₄ alkyl, C(O)O C₁-C₄ alkyl, C(O)C₁-C₄ alkyl and C(O)NH C₁-C₄ alkyl; |
| | R² is selected from H and C₁-C₄ alkyl; |
| | R³ is selected from H and C₁-C₄ alkyl; |
| | or R² and R³ are bound together along with the carbon and nitrogen atoms to which they are bound to form a piperidine ring, |
| | R⁴ and R⁵ are independently selected from H, halogen, hydroxy, C₁-C₄ alkyl, fluoro-substituted C₁-C₄ alkyl and C₁-C₄ alkoxy; and |
| | X is selected from C and N, |
| | and preferred compounds such as |
| | Cyproheptadine (4-(5H-dibenzo-[a,d]cyclohepten-5-ylidene)-1-methylpiperidine hydrochloride), |
| | Amitriptyline (3-(10,11-dihydro-5H-dibenzo[[*a,d*]]cycloheptene-5-ylidene)-N,N-dimethyl- 1-propanamine), Loratadine (Ethyl 4-(8-chloro-5,6-dihydro-11H-benzo[5,6]cyclohepta[1,2-b]pyridin-11-ylidene)-1-piperidinecarboxylate, Cyclobenzrapine (3-(5H-dibenzo[a,d]cyclohepten-5-ylidene)-N,N-dimethyl-1-propanamine). |
| V | Nivalenol (12,13-epoxy-3,4,7,15-tetrahydroxytrichothec-9-en-8-one) as described in WO0359249 |

Other c-MAF inhibitors are described in the patent application WO2005063252, such as shown in the following table (Table 2).

**Table 2: c-MAF inhibitors**

| **Antagonist** | **Reference for cdk2 inhibitory activity** |
|---|---|
| **Purine Analogs** | |
| Purvalanols such as 2-(1R-Isopropyl-2-hydroxyethylamino)-6-(3-chloroanilino)-9-isopropylpurine having a molecular formula C₁₉H₂₅ClN₆O available from Sigma-Aldrich under the trade name Purvalanol A (#P4484, Sigma-Aldrich, St. Louis, MO), Purvalanol B, aminopurvalanol, compound 52 (where isopropyl of purvalanol A is replaced with H) | Gray, N.S. et al., Science, 281, 533-538 (1998); |
| | Chang, Y.T. et al., Chem. Biol., 6, 361-375 (1999). |
| 2-(Hydroxyethylamino)-6-benzylamino-9-methylpurine having a molecular formula C₁₅H₁₃N₆O available from Sigma-Aldrich under the trade name Olomoucine (#O0886), 2-(2'-Hydroxyethylamino)-6-benzylamino-9-isopropylpurine having a molecular formula C₁₇H₂₂N₆O available from Sigma-Aldrich under the trade name N⁹-isopropylolomoucine, (#I0763); CVT-313 | Vesely, J., et al., (1994) Eur. J. Biochem., 224, 771-86, 11; |
| | Brooks, E.E., et al., (1997) J. Biol. Chem., 272, 29207-1 1 |
| 6-(Benzylamino)-2(R)-[[1-(hydraxymethyl)propyl]amino]-9-isopropylpurine 2-(R)-[[9-(1-methyletbyl)-6-[(phenylmethyl)amino]-9H-purin-2-yl]amino]-1-butanol having a molecular formula of C₁₉H₂₆N₆O available from Sigma-Aldrich under the trade name Roscovitine (#R7772), methoxyroscovitine | Wang, D. et al., J. Virol., 75, 7266-7279 (2001); McClue, S.J. et al., Int. J. Cancer, 102, 463-468 (2002); |
| | Meijer, L., et al., (1997) Eur. J. Biochem., 243, 527-36 |
| Purine analog N2-(cis-2-Aminocyclohexyl)-N6-(3-chlorophenyl)-9-ethyl-9H-purine-2,6-diamine having a molecular formula of C₁₉H₂₄CIN₇ available from Sigma-Aldrich under the trade name CGP74514 (#C3353) | Imbach, P. et al., Bioorg. Med. Chem. Lett., 9, 91-96 (1999); |
| | Dreyer, M.K. et al., J. Med. Chem., 44, 524-530 (2001). |
| CGP79807, a purine analog of CGP74514 (supra) where Cl is replaced with CN, OH is removed, and the ortho position of cyclohexane ring is NH₂ | Imbach, P. et al., Bioorg. Med. Chem. Lett., 9, 91-96 (1999); |
| | Dreyer, M.K. et al., J. Med. Chem., 44, 524-530 (2001). |
| purine analog such as O6-cyclohexylmethyl guanine NU2058 | Arris, C.E. et al., J. Med. Chem., 43, 2797-2804 (2000); |
| | Davies et al, Nature Structural Biology, 9:10, 745-749, 2002 |
| purine analog such as NU6102 | Arris, C.E. et al., J. Med Chem., 43, 2797-2804 (2000); Davies, T.G. et al., Nat. Struct Biol., 9, 745-749 (2002). |
| isopentenyl-adenine | Vesely, J., et al., (1994) Eur. J. Biochem., 224, 771-86 |

| **Nonpurine based agents** | |
|---|---|
| Indirubins such as indirubin-3'-monoxime having a molecular formula of G₁₆H₁₁N₃O₂ available from Sigma-Aldrich under the trade name (#I0404), indirubin 5-sulfonate, 5-chloro indirubin | Davies, T.G, et al., Structure, 9, 389-397 (2001); |
| | Marko, D. et al., Br. J. Cancer, 84, 283-289 (2001); |
| | Hoessel, R., et al., (1999) Nat. Cell Biol., 1, 60-7; |
| | PCT/US02/30059 to Hellberg et al., published as WO 03/027275. |
| Oxindole 1 of Fischer as referenced in column 2 of this table, (#IN118, JMAR Chemical, | Porcs-Makkay, M., et al., Tetrahedron 2000, 56, 5893; Org. Process Res. Dev. 2000, 4, 10 |
| Indenopyrazoles | Nugiel, D.A. et al., J. Med. Chem., 44, 1334-1336 (2001); Nugiel, D.A. et. al., J. Med. Chem., 45, 5224-5232 (2002); Yue, E.W. et al., J. Med. Chem., 45, 5233-5248 (2002). |
| Pyrido(2,3-d)pyrimidine-7-ones, compound 3 of Fischer | Barvian, M. et al., J. Med. Chem., 43, 4606-4616 (2000); Toogood, P.L., Med. Res. Rev., 21,487-498 (2001). |
| Quinazolines such as anilinoquinazoline | Sielecki, T.M. et al., Bioorg. Med. Chem. Lett., 11, 1157-1160 (2001); |
| | Mettey et al., J. Med. Chem. 2003,46,222-236. |
| Thiazoles such as fused thiazole, 4-{[(7-Oxo-6,7-dihydro-8H-[1,3]thiazolo[5,4-e]indol-8-ylidene)methyl]amino}-N-(2-pyridyl)benzenesulfonamide having a molecular formula of C₂₁H₁₅N₅O₃S₂ available from Sigma-Aldrich under the trade name GW8510 (#G7791) | Davis, S.T. et al., Science, 291, 134-137 (2001); |
| | PCT/US02/30059 to Hellberg et al., published as WO 03/027275. |
| Flavopiridols such as flavopiridol (L86 8275; NCS 649890, National Cancer Institute, Bethesda, MD) and a dechloro derivative | Carlson, B.A., et al., (1996) Cancer Res., 56, 2973-8 |
| such as Staurosporine (#S1016, A.G. Scientific, San Diego, CA) or UCN-01 (7-hydroxystaurosporine) National Cancer Institute, Bethesda, MD | Rialet, V., et al., (1991) Anticancer Res., 11, 1581-90; |
| | Wang, Q., et al., (1995) Cell Growth Differ., 6, 927-36, Alciyama, T., et al, (1997) Cancer Res., 57, 1495-501, Kawakami, K., et al., (1996) Biochem. Biophys. Res. Commun.,219, 778-83 |
| Paullones such as 9-Bromo-7,12-dihydro-indolo[3,2-d][1]benzazepin-6(5H)-one having a molecular formula of C₁₆H₁₁BrN₂O available from Sigma-Aldrich under the trade name kenpaullone (#K3888), or 9-Nitro-7,12-dihydroindolo-[3,2-d][1]benzazepin-6(5)-one having a molecular formula of C₁₆H₁₁N₃O₃ available from Sigma-Aldrich under the trade name alsterpaullone (#A4847) | Zaharevitz, D.W. et al., Cancer Res., 59, 2566-2569 (1999); Schultz, C. et al., J. Med. Chem., 42, 2909-2919 (1999); |
| | Zaharevitz, D.W., et al., (1999) Cancer Res., 59, 2566-9; |
| | PCT/US02/30059 to Hellberg et al., published as WO 03/027275. |
| CGP 41251, an alkaloid | Begemann, M., et al., (1998) Anticancer Res., |
| | 18,2275-82; |
| | Fabbro et al., Pharmacol Ther. 1999 May-Jun;82(2-3):293-301 |
| Hymenialdisines such as 10z-hymenialdisine having a molecular formula of C₁₁H₁₀BrN₅O₂ available from Biochemicals.net, a division of A.G. Scientific, Inc. (San Diego, CA) (H-1150) | Meijer, L., et al., (1999) Chemistry & Biology, 7,51-63; |
| | PCT/US02/30059 to Hellberg et al.*,* published as WO 03/027275. |
| CGP60474, a phenylaminopyrimidine | 21; WO95/09853, Zimmermann et al., September 21, 1994 |
| Thiazolopyrimidine 2 | Attaby et al., Z. Naturforsch. 54b, 788-798 (1999) |
| Diarylurea | Honma, T. et al., J. Med. Chem., 44,4628-4640 (2001), Honma, T. et al., J. Med. Chem., 44, 4615-4627 (2001). |
| (2R)-2,5-Dihydro-4-hydroxy-2-[(4-hydroxy-3-(3-methyl-2-butenyl)phenyl)methyl]-3-(4-hydroxyphenyl)-5-oxo-2-furancarboxylic acid methyl ester having a molecular formula of C₂₄H₂₄O₇ available from Sigma-Aldrich under the trade name Butyrolactone-I (B7930) | Kitagawa, M. et al., Oncogene, 8,2425-2432 (1993). |
| Aloisine A, Cat. No, 128125 (Calbiochem, San Diego, CA) | Mettey et al., J. Med. Chem. 2003, 46, 222-236 |

In a preferred embodiment, the bone metastasis is osteolytic metastasis.

The c-MAF inhibitory agents are typically administered in combination with a pharmaceutically acceptable carrier.

The term "carrier" refers to a diluent or an excipient whereby the active ingredient is administered. Such pharmaceutical carriers can be sterile liquids such as water and oil, including those of a petroleum, animal, plant or synthetic origin such as peanut oil, soy oil, mineral oil, sesame oil and the like. Water or aqueous saline solutions and aqueous dextrose and glycerol solutions, particularly for injectable solutions, are preferably used as carriers. Suitable pharmaceutical carriers are described in "Remington's Pharmaceutical Sciences" by E.W. Martin, 1995. Preferably, the carriers of the disclosure are approved by the state or federal government regulatory agency or are listed in the United States Pharmacopeia or other pharmacopeia generally recognized for use thereof in animals and more particularly in human beings.

The carriers and auxiliary substances necessary for manufacturing the desired pharmaceutical dosage form of the pharmaceutical composition of the disclosure will depend, among other factors, on the pharmaceutical dosage form chosen. Said pharmaceutical dosage forms of the pharmaceutical composition will be manufactured according to the conventional methods known by the person skilled in the art. A review of the different methods for administering active ingredients, excipients to be used and processes for producing them can be found in "Tratado de Farmacia Galénica", C. Faulí i Trillo, Luzán 5, S.A. 1993 Edition. Examples of pharmaceutical compositions include any solid composition (tablets, pills, capsules, granules, etc.) or liquid composition (solutions, suspensions or emulsions) for oral, topical or parenteral administration. Furthermore, the pharmaceutical composition may contain, as deemed necessary, stabilizers, suspensions, preservatives, surfactants and the like.

For use in medicine, the c-MAF inhibitory agents can be found in the form of a prodrug, salt, solvate or clathrate, either isolated or in combination with additional active agents and can be formulated together with a pharmaceutically acceptable excipient. Excipients preferred for use thereof in the present disclosure include sugars, starches, celluloses, rubbers and proteins. In a particular embodiment, the pharmaceutical composition of the disclosure will be formulated in a solid pharmaceutical dosage form (for example tablets, capsules, pills, granules, suppositories, sterile crystal or amorphous solids that can be reconstituted to provide liquid forms, etc.), liquid pharmaceutical dosage form (for example solutions, suspensions, emulsions, elixirs, lotions, ointments, etc.) or semisolid pharmaceutical dosage form (gels, ointments, creams and the like). The pharmaceutical compositions of the disclosure can be administered by any route, including but not limited to the oral route, intravenous route, intramuscular route, intraarterial route, intramedularry route, intrathecal route, intraventricular router, transdermal route, subcutaneous route, intraperitoneal route, intranasal route, enteric route, topical route, sublingual route or rectal route. A review of the different ways for administering active ingredients, of the excipients to be used and of the manufacturing processes thereof can be found in Tratado de Farmacia Galénica, C. Faulí i Trillo, Luzán 5, S.A., 1993 Edition and in Remington's Pharmaceutical Sciences (A.R. Gennaro, Ed.), 20th edition, Williams & Wilkins PA, USA (2000). Examples of pharmaceutically acceptable carriers are known in the state of the art and include phosphate buffered saline solutions, water, emulsions such as oil/water emulsions, different types of wetting agents, sterile solutions, etc. The compositions comprising said carriers can be formulated by conventional processes known in the state of the art.

In the event that nucleic acids (siRNA, polynucleotides encoding siRNA or shRNA or polynucleotides encoding negative c-MAF dominants) are administered, the disclosure contemplates pharmaceutical compositions particularly prepared for administering said nucleic acids. The pharmaceutical compositions can comprise said naked nucleic acids, *i.e.,* in the absence of compounds protecting the nucleic acids from degradation by the nucleases of the body, which entails the advantage that the toxicity associated with the reagents used for transfection is eliminated. Administration routes suitable for naked compounds include the intravascular route, intratumor route, intracranial route, intraperitoneal route, intrasplenic route, intramuscular route, subretinal route, subcutaneous route, mucosal route, topical route and oral route (Templeton, 2002, DNA Cell Biol., 21:857-867). Alternatively, the nucleic acids can be administered forming part of liposomes conjugated to cholesterol or conjugated to compounds capable of promoting the translocation through cell membranes such as the Tat peptide derived from the HIV-1 TAT protein, the third helix of the homeodomain of the *D. melanogaster* antennapedia protein, the herpes simplex virus VP22 protein, arginine oligomers and peptides as described in WO07069090 (Lindgren, A. et al., 2000, Trends Pharmacol. Sci, 21:99-103, Schwarze, S.R. et al. , 2000, Trends Pharmacol. Sci., 21:45-48, Lundberg, M et al., 2003, Mol Therapy 8:143-150 and Snyder, E.L. and Dowdy, S.F., 2004, Pharm. Res. 21:389-393). Alternatively, the polynucleotide can be administered forming part of a plasmid vector or viral vector, preferably adenovirus-based vectors, in adeno-associated viruses or in retroviruses such as viruses based on murine leukemia virus (MLV) or on lentivirus (HIV, FIV, EIAV).

The c-MAF, VAT1L, CLEC3A, WWOX, or the gene the sequence of which is provided in the Gene Database with ID 645957 inhibitory agents or the pharmaceutical compositions containing them can be administered at a dose of less than 10 mg per kilogram of body weight, preferably less than 5, 2, 1, 0.5, 0.1, 0.05, 0.01, 0.005, 0.001, 0.0005, 0.0001, 0.00005 or 0.00001 mg per kg of body weight. The unit dose can be administered by injection, inhalation or topical administration.

The dose depends on the severity and the response of the condition to be treated and it may vary between several days and months or until the condition subsides. The optimal dosage can be determined by periodically measuring the concentrations of the agent in the body of the patient. The optimal dose can be determined from the EC50 values obtained by means of previous *in vitro* or *in vivo* assays in animal models. The unit dose can be administered once a day or less than once a day, preferably less than once every 2, 4, 8 or 30 days. Alternatively, it is possible to administer a starting dose followed by one or several maintenance doses, generally of a lesser amount than the starting dose. The maintenance regimen may involve treating the patient with a dose ranging between 0.01 µg and 1.4 mg/kg of body weight per day, for example 10, 1, 0.1, 0.01, 0.001, or 0.00001 mg per kg of body weight per day. The maintenance doses are preferably administered at the most once every 5, 10 or 30 days. The treatment must be continued for a time that will vary according to the type of disorder the patient suffers, the severity thereof and the condition of the patient. After treatment, the progress of the patient must be monitored to determine if the dose should be increased in the event that the disease does not respond to the treatment or the dose is reduced if an improvement of the disease is observed or if unwanted side effects are observed.

### Treatment or prevention of the bone degradation in cancer patients with bone metastasis having elevated c-MAF levels

Also disclosed is a c-MAF, VAT1L, CLEC3A, WWOX, or the gene the sequence of which is provided in the Gene Database with ID 645957 inhibitory agent or an agent capable of avoiding or preventing bone degradation for use in the treatment of bone metastasis in a subject suffering cancer, and having determined there are elevated c-MAF, VAT1L, CLEC3A, WWOX, or the gene the sequence of which is provided in the Gene Database with ID 645957 levels in a metastatic sample with respect to a control sample through use of a probe specific to c-MAF or the chromosomal region containing c-MAF, VAT1L, CLEC3A, WWOX, or the gene the sequence of which is provided in the Gene Database with ID 645957.

It is also disclosed the use of a c-MAF, VAT1L, CLEC3A, WWOX, or the gene the sequence of which is provided in the Gene Database with ID 645957 inhibitory agent or an agent capable of avoiding or preventing bone degradation for the manufacture of a medicament for the treatment of bone metastasis in a subject suffering cancer, and having elevated c-MAF, VAT1L, CLEC3A, WWOX, or the gene the sequence of which is provided in the Gene Database with ID 645957 levels in a metastatic sample with respect to a control sample through use of a probe specific to c-MAF, VAT1L, CLEC3A, WWOX, or the gene the sequence of which is provided in the Gene Database with ID 645957 or the chromosomal region containing c-MAF, VAT1L, CLEC3A, WWOX, or the gene the sequence of which is provided in the Gene Database with ID 645957.

It is also disclosed a method of prevention and/or treatment of the degradation in a subject suffering cancer and has elevated c-MAF, VAT1L, CLEC3A, WWOX, or the gene the sequence of which is provided in the Gene Database with ID 645957 levels in a metastatic sample with respect to a control sample, which comprises administering a c-MAF, VAT1L, CLEC3A, WWOX, or the gene the sequence of which is provided in the Gene Database with ID 645957 inhibitory agent or an agent for avoiding or preventing bone degradation to said subject.

In a particular embodiment the bone metastasis is osteolytic metastasis.

c-MAF inhibitory agents and agents capable of avoiding or preventing bone degradation suitable for the therapeutic method disclosed have been described in detail above in the context of the customized therapy method.

The reference or control sample is a sample of a subject cancer, who has not suffered metastasis or that correspond to the median value of the c-MAF, VAT1L, CLEC3A, WWOX, or the gene the sequence of which is provided in the Gene Database with ID 645957 gene expression level measured in a tumor tissue collection in biopsy samples of subjects with cancer who have not suffered metastasis.

Methods for determining or quantifying if the c-MAF, VAT1L, CLEC3A, WWOX, or the gene the sequence of which is provided in the Gene Database with ID 645957 levels are elevated with respect to a control sample have been described in detail in relation with the first method of the invention and are equally applicable to the agent for avoiding or preventing bone degradation.

Alternatively a combined treatment can be carried out, in which more than one agent for avoiding or preventing bone degradation from those mentioned above are combined to treat and/or prevent the metastasis or said agents can be combined with other supplements, such as calcium or vitamin D or with a hormone.

The agents for avoiding or preventing bone degradation are typically administered in combination with a pharmaceutically acceptable carrier. The term "carrier" and the types of carriers have been defined above for the c-MAF, VAT1L, CLEC3A, WWOX, or the gene the sequence of which is provided in the Gene Database with ID 645957 inhibitory agent, as well as the form and the dose in which they can be administered and are equally applicable to the agent for avoiding or preventing bone degradation.

The following examples illustrate the invention and do not limit the scope thereof.

### Kits

In another aspect, the disclosure relates to a kit for predicting bone metastasis of cancer, in a subject suffering from said cancer, the kit comprising: a) a probe for quantifying the expression level, amplification or translocation of a gene of interest in a sample of said subject; and b) means for comparing the quantified level of expression of gene of interest in said sample to a reference gene of interest expression level.

In another aspect, the disclosure relates to a kit for predicting the clinical outcome of a subject suffering from bone metastasis from cancer, the kit comprising: a) a probe for quantifying the expression level, amplification or translocation of gene of interest in a sample of said subject; and b) means for comparing the quantified expression level, amplification or translocation of the gene of interest in said sample to a reference gene of interest expression level.

In another aspect the disclosure relates to a kit for determining a therapy for a subject suffering from cancer, the kit comprising: a) a probe for quantifying the expression level of a gene of interest in a sample of said subject; b) means for comparing the quantified expression level of a gene of interest in said sample to a reference gene of interest expression level; and c) means for determining a therapy for preventing and/or reducing bone metastasis in said subject based on the comparison of the quantified expression level to the reference expression level.

In another aspect the disclosure relates to a kit comprising: i) a probe for quantifying the expression level of a gene of interest in a sample of a subject suffering from cancer, and ii) one or more gene of interest expression level indices that have been predetermined to correlate with the risk of bone metastasis.

A probe for quantifying the expression level, amplification or translocation of a gene of interest in a sample of said subject have been previously described in detail. In a preferred embodiment, the probe for quantifying expression comprises a set of probes and/or primers that specifically bind and/or amplify the gene of interest.

All the particular embodiments of the methods of the present invention are applicable to the kits of the invention and to their uses.

### Method for typing a sample of a subject suffering cancer.

In another aspect, the invention relates to an *in vitro* method for typing a sample of a subject suffering from cancer, the method comprising:
a) providing a sample from said subject;
b) using a probe to quantify the copy number or amplification of a gene of interest in said sample, wherein the gene of interest is selected from the group of: VAT1L, CLE3A, or the gene the sequence of which is provided in the Gene Database with ID 645957 or quantifying the expression level, copy number or amplification of the gene of interest WWOX in said sample;
c) typing said sample by comparing the quantified expression level, copy number or amplification of the gene of interest to a predetermined reference level of gene of interest expression, copy number or amplification;
wherein said typing provides prognostic information related to the risk of bone metastasis in said subject.

A probe for quantifying the expression level, amplification or translocation of a gene of interest (*e.g*. c-MAF) in a sample of said subject has been previously described in detail.

In a preferred embodiment the sample is a tumor tissue sample.

### Method for classifying a subject suffering from cancer.

In another aspect, the invention relates to a method for classifying a subject suffering from cancer into a cohort, comprising: a) using a probe to determine the expression level, copy number or amplification of a gene of interest in a sample of said subject ; b) comparing the expression level, amplification or copy number of a gene of interest in said sample to a predetermined reference level of gene of interest expression; and c) classifying said subject into a cohort based on said expression level, copy number or amplification of the gene of interest in the sample.

Probes for quantifying the expression level, amplification or copy number of a gene of interest in a sample of said subject have been previously described in detail.

In a preferred embodiment the sample is a tumor tissue sample.

In a preferred embodiment said cohort comprises at least one other individual who has been determined to have a comparable expression level of a gene of interest in comparison to said reference expression level.

In another preferred embodiment said expression level, amplification or translocation of a gene of interest in said sample is increased relative to said predetermined reference level, and wherein the members of the cohort are classified as having increased risk of bone metastasis.

In another preferred embodiment said cohort is for conducting a clinical trial. In a preferred embodiment, the sample is a tumor tissue sample.

### EXAMPLES

### EXAMPLE 1

### Analysis of breast cancer tumor samples using the Vysis probe

### Cohort I. Discovery breast cancer primary tumor cohort

The patients' information was downloaded from GEO (Barrett et al.(2007) (T. Barrett, D. B. Troup, S. E. Wilhite, P. Ledoux, D. Rudnev, C. Evangelista, I. F. Kim, A. Soboleva, M. Tomashevsky, and R. Edgar. NCBI GEO: mining tens of millions of expression profiles-database and tools update. Nucleic Acids Research, 35, January 2007. ISSN 1362-4962.)). The following set of data was used: union of GSE12276. This cohort has 204 breast cancer patients primary tumor biopsy gene expression profile and its clinical annotation for time and site of distant metastasis as described in the GEO. In order to remove systematic biases, prior to merging the expression measurements were converted to z-scores for all genes. All statistical analyses were performed using Bioconductor (Gentleman et al. (2004) (R.C. Gentleman, V.J. Carey, D.M. Bates, B. Bolstad, M. Dettling, oS. Du- doit, B. Ellis, L. Gautier, Y. Ge, J. Gentry, K. Hornik, T. Hothorn, W. Huber, S. Iacus, R. Irizarry, F. Leisch, C. Li, M. Maechler, A.J. Rossini, G. Sawitzki, C. Smith, G. Smyth, L. Tierney, J.Y.H. Yang, and J. Zhang. Bioconductor: Open software development for computational biology and bioinformatics. Genome Biology, 5:R80, 2004. URL http://genomebiology.com/2004/5/10/R80.)).

### Cohort II. Validation breast cancer primary tumor sample cohort:

A second human breast tumor cohort was used to validate the hypothesis discovered with the above patient tumor sample cohort I. The independent validation set is composed of more than 380 primary breast cancer specimens from patients with stage I, II or III BC and annotated follow up (Rojo F., Ann Oncol 23(5): 1156-1164 (2012)). Tissue microarrays were processed as per standard procedures. Tumors could be classified in 3 subtypes according to ER+, Triple Negative and HER2+. The appropriate statistical analysis was performed to see if the 16q23 amplification or the expression of some of the genes included within in these tumors correlates with bone metastasis events in the overall population or in some of the given subtypes.

Statistical analyses in this second cohort were based on the following premises:
i) Comparison of baseline characteristics.
   Equality of variances of age is tested with the Folded F test. Differences in the mean of age are tested with the pooled or Satterhwaite t-test (ANOVA or Kruskal-Wallis for multiple categories comparison) depending on equality of variances. Categorical variables are compared with a chi-square or Fisher test when applicable.
ii) Diagnostic performance FISH and IHC
   - Multivariate analysis is done via stepwise selection with a p-value criterion for entering the variable of p< 0:2 and a criterion for retaining the variable in the model after adjusting of p < 0.10. Diagnostic performance will be evaluated by comparing the AUC of the ROC curves. Goodness of fit of the model will be assessed with the Hosmer-Lemeshow test (if significant, no further analysis will be done).
   - Sensitivity (Se), specificity (Sp), positive predictive value (PPV) and negative predictive value (NPV) will be computed for each of the classification categories based on the most predictive variables (16q23 FISH).
iii) Prognostic role
   - Cox regression modeling of the outcome time to bone metastasis was done, with an "efron" management of ties. The number of events will drive the number of variables that are entered in the models (about one variable for each 5-10 events).

### EXAMPLE 2

### Amplification of the chromosomal region located in 16q23 including VAT1L, CLEC3A, WWOX, the gene the sequence of which is provided in the Gene Database with ID 645957 and MAF genes is associated with bone metastasis

Copy number alterations (CNA) were identified in primary breast cancer specimens associated to risk of metastasis by means of an adaptation of the ACE algorithm (analysis of CNAs by expression data) (Fig. 1a). Among them, an amplified region located in chromosome 16q23 was significantly (p<0.05) associated with risk of metastasis and included *VAT1L, CLEC3A, WWOX, the gene the sequence of which is provided in the Gene Database with ID 645957 and MAF,* gene whose increased expression is expected to be individually and independently associated with risk of bone metastasis in ER+ human Breast Cancer (*i.e. WWOX* expression, HR=1.33 p=0.044, breast cancer primary tumor data set based on the GSE12276 cohort). Similarly, when comparing Parental MCF7 (ER+) to Bone metastatic MCF7 derivatives (BoM2) cells by FISH(16q23) and Comparative Genomic Hybridization (CGH), a gain was confirmed in the 16q23 chromosomal region (Fig. 1a,b). A subset of parental cells (32.7%) carried this genomic amplification, yet the *in vivo* bone metastasis selection led this residual population to take over the rest (88.6%). Thus, we show that the 16q23 is amplified in breast cancers with risk of metastasis, particularly bone metastasis and corroborated in in vivo selected cells for their ability to metastasis to the bone.

### EXAMPLE 3

### Validation in cohort II of the prognostic capacity to predict bone metastasis of the 16q23 DNA genomic amplification by FISH determination.

To further validate the ability of 16q23 genomic amplification to specifically predict bone metastasis risk, 16q23 chromosome region genomic gain was analyzed by means of FISH (a commercially available diagnostic probe that determines the 16q23 genomic region, the IGH/MAF Abbott Vysis probe, was used) in an independent validation set composed of 334 primary breast cancer specimens from patients with stage I, II or III BC and annotated follow up (Rojo F., Ann Oncol 23 (5): 1156-1164 (2012)).

The commercially available diagnostic probe from Abbott Diagnostics was used. This SpectrumOrange probe flanks the MAF gene region and is composed of two segments that are each approximately 350 kb with an approximately 2.2 Mb gap. The centromeric segment is located at chr16:75729985-76079705 (March 2006 assembly, UCSC Genome Browser) and the telomeric segment is located at chr16:78290003-78635873 (March 2006 assembly, UCSC Genome Browser). This probe flanks five genes VAT1L, CLEC3A, WWOX, the gene the sequence of which is provided in the Gene Database with ID 645957 and MAF (ordered from centromere to telomere).

We focused our attention five genes VAT1L, CLEC3A, WWOX, the gene the sequence of which is provided in the Gene Database with ID 645957 and MAF (ordered from centromere to telomere) flanked by the FISH probe.

Tissue microarrays were processed as per standard procedures. The slides were incubated with 16q23 and IGH 14q32 probe mixture (Abbott vysis probe). DAPI counterstain was applied and images were acquired with adequate microscope.

Kaplan-Meier curve of bone (Figure 2a) metastasis-free or overall (Figure 2b) survival in stage I, II, and III BC human primary tumor set (n=334) was determined. Patients were stratified according to 16q23 FISH negative and 16q23 FISH positive group based on cut-off of 2.5 16q23 copies per cell as an average, using 3 cores per tumor. Hazard ratio (Bone Metastasis), specificity and sensitivity of the marker to predict bone metastasis was calculated. Baseline characteristics of the data set and Cox multivariate analysis for overall breast cancers were performed as described above (Table 3 and 4).

### Table 3: Comparison of baseline characteristics by 16q23 FISH>2.5 copies per cell.

**Measured in 3 cores per tumor. (*Percentages computed over the patients without missing values on this variable)**

| | **16q23** | **16q23** | |
|---|---|---|---|
| | **FISH** | **FISH** | |
| | **≤ 2.5 (n=262)** | **> 2.5 (n=75)** | **p-value** |
| Median age (IQR), years | 58 (17) | 58 (21) | 0.32 |
| Postmenopausal (%) | 187 (71.4) | 46 (61.3) | 0.10 |
| ER+ (%) | 200 (76.4) | 53 (70.7) | 0.32 |
| PR+ (%) | 172 (65.7) | 45 (60.0) | 0.37 |
| High grade (%) | 83 (31.7) | 35 (36.7) | 0.016 |
| Ki67* (%) | 55 (22.3) | 29 (41.4) | 0.0014 |
| Subtype* (%) | | | 0.58 |
| Luminal | 151 (66.5) | 39 (66.1) | |
| Her2 | 44 (19.4) | 9 (15.3) | |
| TN | 32 (14.1) | 11 (18.6) | |
| HER2+ (%) | 51 (19.5) | 13 (17.3) | 0.68 |
| pT (%) | | | 0.21 |
| 1 | 164 (62.6) | 41 (54.7) | |
| 2-4 | 98 (37.4) | 34 (45.3) | |
| pN (%) | | | 0.27 |
| 0 | 163 (62.2) | 42 (56.0) | |
| 1-2 | 89 (34.0) | 27 (36.0) | |
| 3 | 10 (3.8) | 6 (8.0) | |

### Table 4: Stage I, II, III Breast Cancer

**Cox regression of time to bone metastasis as first site of relapse. 16q23 FISH >2.5 copies per cell. Measured 3 cores per tumor**

| **Variable** | **Unvariate** | | **Multivariate** | |
|---|---|---|---|---|
| | **HR (95%) (CI)** | **p-value** | **HR (95% CI)** | **p-value** |
| 16q23Fish>2.5 | 27.2 (8.1-91.0) | < 0.0001 | 26.1 (7.8-87.4) | < 0.0001 |
| Ki67 | 2.8 (1.2-6.4) | 0.014 | | |
| pT | | | | |
| 1 | Ref | | Ref | |
| 2-4 | 2.4 (1.1-5.3) | 0.035 | 2.1 (0.9-4.6) | 0.077 |
| pN | | | | |
| 0 | Ref | | | |
| 1-2 | 1.4 (0.6-3.3) | 0.44 | | |
| 3 | 4.8 (1.5-15.1) | 0.0076 | | |

### Table 5: Stage I, II, III ER+ Breast Cancer

**Cox regression of time to bone metastasis as first site of relapse. 16q23 FISH >2.5 copies per cell. Measured 3 cores per tumor**

| **Variable** | **Unvariate** | | **Multivariate** | |
|---|---|---|---|---|
| | **HR (95%) (CI)** | **p-value** | **HR (95% CI)** | **p-value** |
| 16q23Fish | 53.5 (7.0-406.7) | 0.0001 | 49.5 (6.5-376.3) | 0.0002 |
| pT | | | | |
| 1 | Ref | | Ref | |
| 2-4 | 3.4 (1.2-9.4) | 0.018 | 2.8 (1.0-7.9) | 0.047 |
| pN | | | | |
| 0 | Ref | | | |
| 1-2 | 2.6 (0.8-8.0) | 0.094 | | |
| 3 | 6.8 (1.6-28.8) | 0.0089 | | |

Kaplan-Meier curve of bone metastasis free survival for ER-positive (left) or triple negative (right) patients in I, II, and III BC human primary tumor set (n=250 and n=43 respectively) (Figure 2c) were also determined. Patients were divided to 16q23 FISH negative and 16q23 FISH positive group based on cut-off of 2.5 for 16q23 copies per cell as an average, using 3 cores per tumor. Cox multivariate analysis for ER+ breast cancers were performed as described above (Table 5)

Receiver Operating Characteristic (ROC) curves for diagnostic performance of 16q23 amplification in overall (Figure 2d) and ER+ breast cancer (Figure 2e) were also calculated to estimate the diagnostic performance. In a ROC curve the true positive rate (Sensitivity) is plotted in function of the false positive rate (100-Specificity) for different cut-off points. Each point on the ROC curve represents a sensitivity/specificity pair corresponding to a particular decision threshold.

In summary, the 16q23 amplification measured herein using a 16q23 FISH probe flanking five genes VAT1L, CLEC3A, WWOX, the gene the sequence of which is provided in the Gene Database with ID 645957 and MAF (ordered from centromer to telomer) significantly predicts risk of bone metastasis in breast cancer primary tumors, particularly in TN and ER+ breast cancer subtypes. Thus, in principle the mRNA or Protein expression levels of any of these five genes could be used to predict bone metastasis.

### EXAMPLE 4

### Analysis of WWOX gene expression capacity to predict bone metastasis in a cohort breast cancer

When analyzing bone metastasis in cohort I (GSE12276), the Cox Proportional Hazards Models (using the R function coxph from Packaged survival) were adjusted to see if bone metastasis could be explained through WWOX expression. WWOX had a statistically significant capacity to predict bone metastasis (HR(bone metastasis)=1.23 p=0.02) in breast cancer primary tumors. WWOX expression in the primary tumor can be used to determine the prognosis of the tendency to develop metastasis in a subject with breast cancer.

Similarly, WWOX had a statistically significant capacity to predict bone metastasis (HR(bone metastasis)=1.33 p=0.044) in ER+ breast cancer primary tumors. WWOX expression in the primary tumor can be used to determine the prognosis of the tendency to develop metastasis in a subject with breast cancer.

It is understood that the examples described herein are for illustrative purposes only and that various modifications or changes in light thereof will be suggested to persons skilled in the art and are to be included with this application.

## Claims

1. An in vitro method for the prognosis of the tendency to develop metastasis and/or recurrence in a subject with cancer, said method comprising:
(i) quantifying the copy number or amplification of a gene of interest in a sample of 5 said subject, wherein the gene of interest is selected from the group consisting of: VAT1L, CLEC3A, or the gene with Gene ID 645957 or quantifying the expression level, copy number or amplification of the gene of interest WWOX and
(ii) comparing the expression level, copy number or amplification obtained in (i) with the expression level, copy number or degree of amplification of the gene of interest 10 in a control sample,
wherein if the expression level, copy number or amplification of a gene of interest in said sample is increased with respect to the expression level, copy number or degree of amplification of the gene of interest in the control sample, then said subject has a greater tendency to develop metastasis and/or recurrence.

2. An in vitro method for designing a customized therapy for a subject with cancer which comprises
(i) quantifying the copy number or amplification of a gene of interest in a sample of said subject, wherein the gene of interest is selected from the group consisting of: VAT1L, CLEC3A, or the gene with Gene ID 645957 or quantifying the expression 20 level, copy number or amplification of the gene of interest WWOX and
(ii) comparing the expression level, copy number or amplification obtained in (i) with the expression level, copy number or degree of amplification of the gene of interest in a control sample,
wherein if the expression level, copy number or degree of amplification of a 25 gene of interest in the sample is increased with respect to the expression level of the gene of interest in the control sample, then said subject is susceptible to receive a therapy intended to prevent, inhibit and/or treat metastasis of the cancer and/or a therapy intended to prevent or inhibit bone degradation.

3. The method according to claim 1 or 2, wherein the quantification of the expression level, copy number or amplification of the gene of interest comprises quantifying the messenger RNA (mRNA) of said gene, or a fragment of said mRNA, the 5 complementary DNA (cDNA) of said gene, or a fragment of said cDNA or comprises quantifying the protein level of such gene of interest.

4. An in vitro method for the prognosis of the tendency to develop metastasis and/or recurrence in a subject with cancer which comprises determining if a gene of interest is amplified, gained or shows polyploidy in a tumor sample of said subject 10 relative to a reference gene copy number, wherein said amplification, gain or polyploidy of the gene of interest with respect to said reference gene copy number is indicative of an increased risk of developing metastasis and/or recurrence, wherein the gene of interest is selected from the group consisting of: VAT1L, CLEC3A, WWOX, or the gene with Gene ID 645957.

5. The method according to claim 4, wherein the amplification of the gene of interest is determined by means of determining the amplification of the locus 16q22-q24.

6. The method according to claim 4 or 5, wherein the amplification of the gene of interest is determined by means of using a gene-specific probe.

7. The method of claim 2, wherein the agent capable of avoiding or preventing bone 20 degradation is selected from the group consisting of: a bisphosphonate, a RANKL inhibitor, a PTH inhibitor, a PTHLH inhibitor, a PRG analog, strontium ranelate, a DKK-1 inhibitor, a dual MET and VEGFR2 inhibitor, an estrogen receptor modulator, an EGFR inhibitor, calcitonin, Radium-223 and a cathepsin K inhibitor.

8. An in vitro method for typing a sample of a subject suffering from cancer, the method 25 comprising:
a) providing a sample from said subject;
b) quantifying the copy number or amplification of a gene of interest in said sample, wherein the gene of interest is selected from the group consisting of: VAT1L, CLEC3A, or the gene with Gene ID 645957 or quantifying the expression level, copy number or amplification of the gene of interest WWOX;
c) typing said sample by comparing the quantified expression level, copy number or amplification of the gene of interest to a predetermined reference level of gene of 5 interest expression, copy number or degree of amplification;
wherein said typing provides prognostic information related to the risk of bone metastasis and/or recurrence in said subject.

9. A method of classifying a subject suffering from cancer into a cohort for conducting a clinical trial comprising: a) determining the copy number or amplification of a gene of 10 interest in a sample of said subject, wherein the gene of interest is selected from the group consisting of: VAT1L, CLEC3A, or the gene with Gene ID 645957 or quantifying the expression level, copy number or amplification of the gene of interest WWOX; b) comparing the expression level, copy number or amplification of the gene of interest in said sample to a predetermined reference level of expression, copy number 15 or amplification of the gene of interest; and c) classifying said subject into a cohort based on said expression level, copy number or amplification of the gene of interest in said sample, and wherein said expression level, copy number or degree of amplification of the gene of interest in said sample is increased relative to said predetermined reference level, and wherein members of the cohort are classified as having increased 20 risk of bone metastasis.

10. A method according to claim 9, wherein said cohort comprises at least one other individual who has been determined to have a comparable expression level, copy number or amplification of a gene of interest in comparison to said reference expression level, copy number or amplification.

11. The method of any one of claims 1-6 or 7-10, wherein the cancer is breast cancer, prostate cancer, lung cancer or renal cell carcinoma.

12. The method of any one of claims 1-6 or 9, wherein the metastasis is bone metastasis.

13. The method of any one of claims 1-6 or 7-12, wherein the bone metastasis is osteolytic bone metastasis.

14. The method of claim 2, wherein the therapy aiming to prevent, inhibit and/or treat metastasis is selected from the group consisting of: a c-MAF inhibitory agent; systemic 5 treatments including but not limited to chemotherapy, hormone therapy, and immunotherapy; radiotherapy; surgery; an mTor inhibitor; a Src kinase inhibitor; a CCR5 antagonist; a COX-2 inhibitor; Alpharadin and/or wherein the agent preventing the bone degradation is selected from the group consisting of a bisphosphonate, a RANKL inhibitor, a PTH inhibitor, or a PTHLH inhibitor or a PRG analog, strontium 10 ranelate, a DKK-1 inhibitor, a dual MET and VEGFR2 inhibitor, an estrogen receptor modulator, calcitonin, Radium-223, a cathepsin K inhibitor and combinations thereof.

15. The *in vitro* method of any one of claims 1-6 or 7-14, wherein the sample is a tumor sample.

## Patentansprüche

1. Ein *In-vitro-*Verfahren zur Prognose der Tendenz zur Entwicklung von Metastasen und/oder eines Rückfalls bei einem krebskranken Subjekt, wobei das Verfahren umfasst:
(i) Quantifizieren der Kopienzahl oder der Amplifikation eines interessierenden Gens in einer Probe des Subjekts, wobei das interessierende Gen ausgewählt ist aus der Gruppe bestehend aus VAT1L, CLEC3A, oder dem Gen mit der Gen-ID 645957 oder Quantifizieren des Expressionsniveaus, der Kopienzahl oder der Amplifikation des interessierenden Gens WWOX, und
(ii) Vergleichen des in (i) erhaltenen Expressionsniveaus, der Kopienzahl oder der Amplifikation mit dem Expressionsniveau, der Kopienzahl oder dem Grad der Amplifikation des interessierenden Gens in einer Kontrollprobe, wobei, wenn das Expressionsniveau, die Kopienzahl oder die Amplifikation eines interessierenden Gens in der Probe in Bezug auf das Expressionsniveau, die Kopienzahl oder den Grad der Amplifikation des interessierenden Gens in der Kontrollprobe erhöht ist, dann das Subjekt eine größere Tendenz zur Entwicklung von Metastasen und/oder eines Rückfalls aufweist.

2. Ein *In-vitro*-Verfahren zum Entwerfen einer maßgeschneiderten Therapie für ein krebskrankes Subjekt umfassend:
(i) Quantifizieren der Kopienzahl oder der Amplifikation eines interessierenden Gens in einer Probe des Subjekts, wobei das interessierende Gen ausgewählt ist aus der Gruppe bestehend aus VAT1L, CLEC3A, oder dem Gen mit der Gen-ID 645957 oder Quantifizieren des Expressionsniveaus, der Kopienzahl oder der Amplifikation des interessierenden Gens WWOX, und
(ii) Vergleichen des in (i) erhaltenen Expressionsniveaus, der Kopienzahl oder der Amplifikation mit dem Expressionsniveau, der Kopienzahl oder dem Grad der Amplifikation des interessierenden Gens in einer Kontrollprobe, wobei, wenn das Expressionsniveau, die Kopienzahl oder der Grad der Amplifikation eines interessierenden Gens in der Probe in Bezug auf das Expressionsniveau des interessierenden Gens in der Kontrollprobe erhöht ist, dann das Subjekt empfänglich ist, eine Therapie zur Vorbeugung, Hemmung und/oder Behandlung von einer Metastasierung des Krebses und/oder eine Therapie zur Vorbeugung oder Hemmung von Knochenabbau zu erhalten.

3. Das Verfahren gemäß Anspruch 1 oder 2, wobei die Quantifizierung des Expressionsniveaus, der Kopienzahl oder der Amplifikation des interessierenden Gens das Quantifizieren der Messenger-RNA (mRNA) des Gens, oder eines Fragments der mRNA, der komplementären DNA (cDNA) des Gens, oder eines Fragments der cDNA umfasst oder das Quantifizieren des Proteinniveaus eines solchen interessierenden Gens umfasst.

4. Ein *In-vitro*-Verfahren zur Prognose der Tendenz zur Entwicklung von Metastasen und/oder eines Rückfalls bei einem krebskranken Subjekt, umfassend die Bestimmung, ob ein interessierendes Gen in einer Tumorprobe des Subjekts amplifiziert ist, Polyploidie erworben hat oder zeigt, in Bezug auf eine Referenz-Kopienzahl, wobei die Amplifikation, der Erwerb oder das Vorhandensein einer Polyploidie des interessierenden Gens in Bezug auf die Referenz-Kopienzahl ein erhöhtes Risiko anzeigt, Metastasen zu entwickeln und/oder für einen Rückfall anzeigt, wobei das interessierende Gen ausgewählt ist aus der Gruppe bestehend aus VAT1L, CLEC3A, WWOX oder dem Gen mit der Gen-ID 645957.

5. Das Verfahren gemäß Anspruch 4, wobei die Amplifikation des interessierenden Gens mittels Bestimmung der Amplifikation des Locus 16q22-q24 bestimmt wird.

6. Das Verfahren gemäß Anspruch 4 oder 5, wobei die Amplifikation des interessierenden Gens mittels einer genspezifischen Sonde bestimmt wird.

7. Das Verfahren gemäß Anspruch 2, wobei das Mittel zur Vermeidung oder Vorbeugung von Knochenabbau ausgewählt ist aus der Gruppe bestehend aus einem Bisphosphonat, einem RANKL-Hemmer, einem PTH-Hemmer, einem PTHLH-Hemmer, einem PRG-Analogon, Strontium-Ranelat, einem DKK-1-Hemmer, einem dualen MET- und VEGFR2-Hemmer, einem Östrogenrezeptormodulator, einem EGFR-Hemmer, Calcitonin, Radium-223, und einem Cathepsin-K-Hemmer.

8. Ein *In-vitro*-Verfahren zum Typisieren einer Probe eines an Krebs leidenden Subjekts, wobei das Verfahren umfasst:
a) Bereitstellen einer Probe von dem Subjekt;
b) Quantifizieren der Kopienzahl oder der Amplifikation eines interessierenden Gens in der Probe, wobei das interessierende Gen ausgewählt ist aus der Gruppe bestehend aus VAT1L, CLEC3A, oder dem Gen mit der Gen-ID 645957 oder Quantifizieren des Expressionsniveaus, der Kopienzahl oder der Amplifikation des interessierenden Gens WWOX;
c) Typisieren der Probe durch Vergleichen des quantifizierten Expressionsniveaus, der Kopienzahl oder der Amplifikation des interessierenden Gens mit einem vorbestimmten Referenzniveau der Expression des interessierenden Gens, der Kopienzahl oder des Grads der Amplifikation;
wobei die Typisierung prognostische Informationen bezüglich des Risikos einer Knochenmetastasierung und/oder eines Rückfalls bei dem Subjekt liefert.

9. Ein Verfahren zum Klassifizieren eines an Krebs leidenden Subjekts in eine Kohorte zur Durchführung einer klinischen Studie, umfassend: a) Quantifizieren der Kopienzahl oder der Amplifikation eines interessierenden Gens in einer Probe des Subjekts, wobei das interessierende Gen ausgewählt ist aus der Gruppe bestehend aus VAT1L, CLEC3A, oder dem Gen mit der Gen-ID 645957 oder Quantifizieren des Expressionsniveaus, der Kopienzahl oder der Amplifikation des interessierenden Gens WWOX; b) Vergleichen des Expressionsniveaus, der Kopienzahl oder der Amplifikation des interessierenden Gens in der Probe mit einem vorbestimmten Referenz-Expressionsniveau, Kopienzahl oder Amplifikation des interessierenden Gens; und c) Klassifizieren des Subjekts in eine Kohorte basierend auf dem Expressionsniveau, der Kopienzahl oder der Amplifikation des interessierenden Gens in der Probe, und wobei das Expressionsniveau, die Kopienzahl oder der Grad der Amplifikation des interessierenden Gens in der Probe in Bezug auf das vorbestimmte Referenzniveau erhöht ist, und wobei Mitglieder der Kohorte als solche mit einem erhöhten Risiko einer Knochenmetastasierung klassifiziert werden.

10. Das Verfahren gemäß Anspruch 9, wobei die Kohorte mindestens ein anderes Individuum umfasst, von dem festgestellt wurde, dass es im Vergleich zu dem Referenz-Expressionsniveau, Kopienzahl oder Amplifikation ein vergleichbares Expressionsniveau, Kopienzahl oder Amplifikation eines interessierenden Gens aufweist.

11. Das Verfahren gemäß irgendeinem der Ansprüche 1 bis 6 oder 7 bis 10, wobei der Krebs Brustkrebs, Prostatakrebs, Lungenkrebs oder Nierenzellkarzinom ist.

12. Das Verfahren gemäß irgendeinem der Ansprüche 1 bis 6 oder 9, wobei die Metastasierung Knochenmetastasierung ist.

13. Das Verfahren gemäß irgendeinem der Ansprüche 1 bis 6 oder 7 bis 12, wobei die Knochenmetastasierung eine osteolytische Knochenmetastasierung ist.

14. Das Verfahren gemäß Anspruch 2, wobei die Therapie zur Vorbeugung, Hemmung und/oder Behandlung von Metastasierung ausgewählt ist aus der Gruppe bestehend aus einem c-MAF hemmenden Mittel; systemischen Behandlungen einschließlich aber nicht beschränkt auf Chemotherapie, Hormontherapie und Immuntherapie; Radiotherapie; Chirurgie; einem mTor-Hemmer; einem Src-Kinase-Hemmer; einem CCR5-Antagonisten; einem COX-2-Hemmer; Alpharadin und/oder wobei das Mittel zur Vermeidung von Knochenabbau ausgewählt ist aus der Gruppe bestehend aus einem Bisphosphonat, einem RANKL-Hemmer, einem PTH-Hemmer, oder einem PTHLH-Hemmer, oder einem PRG-Analogon, Strontium-Ranelat, einem DKK-1-Hemmer, einem dualen MET- und VEGFR2-Hemmer, einem Östrogenrezeptormodulator, Calcitonin, Radium-223, einem Cathepsin-K-Hemmer und Kombinationen davon.

15. Das *In-vitro*-Verfahren gemäß irgendeinem der Ansprüche 1 bis 6 oder 7 bis 14, wobei die Probe eine Tumorprobe ist.

## Revendications

1. Procédé in vitro pour le pronostic d'une tendance à développer des métastases et/ou d'une rechute chez un sujet cancéreux, ledit procédé comprenant :
(i) la quantification du nombre de copies ou de l'amplification d'un gène présentant un intérêt dans un échantillon dudit sujet, lequel gène présentant un intérêt est choisi dans le groupe constitué par : VAT1L, CLEC3A, ou le gène ayant l'identification de gène 645957 ou la quantification du niveau d'expression, du nombre de copies ou de l'amplification du gène présentant un intérêt WWOX et
(ii) la comparaison du niveau d'expression, du nombre de copies ou de l'amplification, obtenu en (i), avec le niveau d'expression, le nombre de copies ou le degré d'amplification du gène présentant un intérêt dans un échantillon témoin,
dans lequel, si le niveau d'expression, le nombre de copies ou l'amplification d'un gène présentant un intérêt dans ledit échantillon est accru par rapport au niveau d'expression, au nombre de copies ou au degré d'amplification du gène présentant un intérêt dans l'échantillon témoin, alors ledit sujet a une plus forte tendance à développer des métastases et/ou une rechute.

2. Procédé in vitro pour concevoir un traitement personnalisé d'un sujet cancéreux, qui comprend
(i) la quantification du nombre de copies ou de l'amplification d'un gène présentant un intérêt dans un échantillon dudit sujet, lequel gène présentant un intérêt est choisi dans le groupe constitué par : VAT1L, CLEC3A, ou le gène ayant l'identification de gène 645957 ou la quantification du niveau d'expression, du nombre de copies ou de l'amplification du gène présentant un intérêt WWOX et
(ii) la comparaison du niveau d'expression, du nombre de copies ou de l'amplification, obtenu en (i), avec le niveau d'expression, le nombre de copies ou le degré d'amplification du gène présentant un intérêt dans un échantillon témoin,
dans lequel, si le niveau d'expression, le nombre de copies ou l'amplification d'un gène présentant un intérêt dans ledit échantillon est accru par rapport au niveau d'expression, au nombre de copies ou au degré d'amplification du gène présentant un intérêt dans l'échantillon témoin, alors ledit sujet est susceptible de recevoir un traitement destiné à prévenir, inhiber et/ou traiter des métastases du cancer et/ou un traitement destiné à prévenir ou inhiber la dégradation des os.

3. Procédé selon la revendication 1 ou 2, dans lequel la quantification du niveau d'expression, du nombre de copies ou de l'amplification du gène présentant un intérêt comprend la quantification de l'ARN messager (ARNm) dudit gène, ou d'un fragment dudit ARNm, de l'ADN complémentaire (ADNc) dudit gène, ou d'un fragment dudit ADNc, ou comprend la quantification du niveau de protéine de ce gène présentant un intérêt.

4. Procédé in vitro pour le pronostic de la tendance à développer des métastases et/ou une rechute chez un sujet cancéreux, qui comprend le fait de déterminer si un gène présentant un intérêt est amplifié, accumulé ou présente une polyploïdie dans un échantillon de tumeur dudit sujet par rapport à un nombre de copies de gène de référence, dans lequel ladite amplification, accumulation ou polyploïdie du gène présentant un intérêt par rapport audit nombre de copies de gène de référence est indicative d'un plus grand risque de développer des métastases et/ou une rechute, dans lequel le gène présentant un intérêt est choisi dans le groupe constitué par : VAT1L, CLEC3A, WWOX, ou le gène ayant l'identification de gène 645957.

5. Procédé selon la revendication 4, dans lequel l'amplification du gène présentant un intérêt est déterminée au moyen de la détermination de l'amplification du locus 16q22-q24.

6. Procédé selon la revendication 4 ou 5, dans lequel l'amplification du gène présentant un intérêt est déterminée au moyen de l'utilisation d'une sonde à spécificité de gène.

7. Procédé selon la revendication 2, dans lequel l'agent capable d'éviter ou de prévenir la dégradation des os est choisi dans le groupe constitué par : un bisphosphonate, un inhibiteur de RANKL, un inhibiteur de PTH, un inhibiteur de PTHLH, un analogue de PRG, le ranélate de strontium, un inhibiteur de DKK-1, un inhibiteur double de MET et de VEGFR2, un modulateur des récepteurs d'oestrogènes, un inhibiteur d'EGFR, la calcitonine, le radium-223 et un inhibiteur de cathepsine K.

8. Procédé in vitro pour typer un échantillon d'un sujet cancéreux, le procédé comprenant :
a) la fourniture d'un échantillon auprès dudit sujet ;
b) la quantification du nombre de copies ou de l'amplification d'un gène présentant un intérêt dans ledit échantillon, lequel gène présentant un intérêt est choisi dans le groupe constitué par : VAT1L, CLEC3A, ou le gène ayant l'identification de gène 645957 ou la quantification du niveau d'expression, du nombre de copies ou de l'amplification du gène présentant un intérêt WWOX ;
c) le typage dudit échantillon par comparaison du niveau d'expression, du nombre de copies ou de l'amplification, ainsi quantifié, du gène présentant un intérêt, avec un niveau de référence prédéterminé de l'expression, du nombre de copies ou du degré d'amplification du gène présentant un intérêt ;
dans lequel ledit typage donne une information de pronostique concernant le risque de métastases osseuses et/ou de rechute chez ledit sujet.

9. Procédé pour classer un sujet cancéreux dans une cohorte pour effectuer un essai clinique, comprenant : a) la détermination du nombre de copies ou de l'amplification d'un gène présentant un intérêt dans un échantillon dudit sujet, lequel gène présentant un intérêt est choisi dans le groupe constitué par : VAT1L, CLEC3A, ou le gène ayant l'identification de gène 645957 ou la quantification du niveau d'expression, du nombre de copies ou de l'amplification du gène présentant un intérêt WWOX ; b) la comparaison du niveau d'expression, du nombre de copies ou de l'amplification du gène présentant un intérêt dans ledit échantillon avec un niveau de référence d'expression, un nombre de copies ou une amplification, prédéterminé, dudit gène présentant un intérêt ; et c) la classification dudit sujet dans une cohorte sur la base dudit niveau d'expression, nombre de copies ou amplification du gène présentant un intérêt dans ledit échantillon, et dans lequel ledit niveau d'expression, nombre de copies ou degré d'amplification du gène présentant un intérêt dans ledit échantillon est accru par rapport audit niveau de référence prédéterminé, et dans lequel les membres de la cohorte sont classés comme ayant un plus grand risque de métastases osseuses.

10. Procédé selon la revendication 9, dans lequel ladite cohorte comprend au moins un autre individu qui a été déterminé comme ayant un niveau d'expression, un nombre de copies ou une amplification comparable d'un gène présentant un intérêt en comparaison avec ledit niveau d'expression, nombre de copies ou amplification de référence.

11. Procédé selon l'une quelconque des revendications 1 à 6 et 7 à 10, dans lequel le cancer est un cancer du sein, un cancer de la prostate, un cancer du poumon ou un carcinome à cellules rénales.

12. Procédé selon l'une quelconque des revendications 1 à 6 et 9, dans lequel les métastases sont des métastases osseuses.

13. Procédé selon l'une quelconque des revendications 1à 6 et 7 à 12, dans lequel les métastases osseuses sont des métastases osseuses ostéolytiques.

14. Procédé selon la revendication 2, dans lequel le traitement visant à prévenir, inhiber et/ou traiter des métastases est choisi dans le groupe constitué par : un agent inhibiteur de c-MAF ; des traitements systémiques comprenant, mais sans s'y limiter, une chimiothérapie, une hormonothérapie, et une immunothérapie ; une radiothérapie ; une chirurgie ; un inhibiteur de mTor ; un inhibiteur de Src kinase ; un antagoniste de CCR5 ; un inhibiteur de COX-2 ; l'alpharadine, et/ou dans lequel l'agent prévenant une dégradation des os est choisi dans le groupe constitué par un bisphosphonate, un inhibiteur de RANKL, un inhibiteur de PTH, ou un inhibiteur de PTHLH ou un analogue de PRG, le ranélate de strontium, un inhibiteur de DKK-1, un inhibiteur double de MET et de VEGFR2, un modulateur des récepteurs d'oestrogènes, la calcitonine, le radium-223, un inhibiteur de cathepsine K et leurs combinaisons.

15. Procédé *in vitro* selon l'une quelconque des revendications 1 à 6 et 7 à 14, dans lequel l'échantillon est un échantillon de tumeur.
